(19) Europäisches Patentamt
European Patent Office
Office européen des brevets

(11) EP 1 121 364 B1

(12) EUROPÄISCHE PATENTSCHRIFT

(45) Veröffentlichungstag und Bekanntmachung des Hinweises auf die Patenterteilung: **08.01.2003 Patentblatt 2003/02**

(21) Anmeldenummer: **99952569.4**

(22) Anmeldetag: **14.10.1999**

(51) Int Cl.[7]: **C07D 493/04**, C07D 277/24, C07F 7/18 // (C07D493/04, 313:00), C07D303:00

(86) Internationale Anmeldenummer: **PCT/EP99/07746**

(87) Internationale Veröffentlichungsnummer: **WO 00/023452 (27.04.2000 Gazette 2000/17)**

(54) **VERFAHREN ZUR HERSTELLUNG VON EPOTHILON B UND DERIVATEN DAVON**

METHOD FOR PRODUCING EPOTHILONE B AND DERIVATIVES THEREOF

PROCEDE DE PREPARATION D'EPOTHILONE B ET DE SES DERIVES

(84) Benannte Vertragsstaaten: **AT BE CH CY DE DK ES FI FR GB GR IE IT LI LU MC NL PT SE**

(30) Priorität: **14.10.1998 DE 19848306**

(43) Veröffentlichungstag der Anmeldung: **08.08.2001 Patentblatt 2001/32**

(73) Patentinhaber: **Schering Aktiengesellschaft 13353 Berlin (DE)**

(72) Erfinder:
- **MULZER, Johann D-13467 Berlin (DE)**
- **MANTOULIDIS, Andreas A-1190 Wien (AT)**
- **ÖHLER, Elisabeth A-3400 Klosterneuburg (AT)**

(56) Entgegenhaltungen:
**WO-A-93/10121** **WO-A-97/19086** **WO-A-98/25929**

- **CLAUS E ET AL: "Synthesis of the C1-C9 Segment of Epothilons" TETRAHEDRON LETTERS,NL,ELSEVIER SCIENCE PUBLISHERS, AMSTERDAM, Bd. 38, Nr. 8, 24. Februar 1997 (1997-02-24), Seiten 1359-1362, XP004053058 ISSN: 0040-4039**
- **K C NICOLAOU ET AL: "Total syntheses of Epothilones A and B via a macrolactonization-based strategy" JOURNAL OF THE AMERICAN CHEMICAL SOCIETY,US,AMERICAN CHEMICAL SOCIETY, WASHINGTON, DC, Bd. 119, Nr. 34, 1997, Seiten 7974-7991-7991, XP002110540 ISSN: 0002-7863**
- **MULZER J ET AL: "Easy Access to the Epothilone Family - Synthesis of Epothilone B" TETRAHEDRON LETTERS,NL,ELSEVIER SCIENCE PUBLISHERS, AMSTERDAM, Bd. 39, Nr. 47, 19. November 1998 (1998-11-19), Seiten 8633-8636, XP004140600 ISSN: 0040-4039**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 121 364 B1

## Beschreibung

Die vorliegende Erfindung betrifft ein Verfahren zur Herstellung von Epothilon B und Derivaten davon.

Es ist bekannt, daß die Naturstoffe Epothilon A (R = H) und Epothilon B (R = Methyl) (Verbindung I, DE 195 42 986 A1, DE 41 38 042 C2)

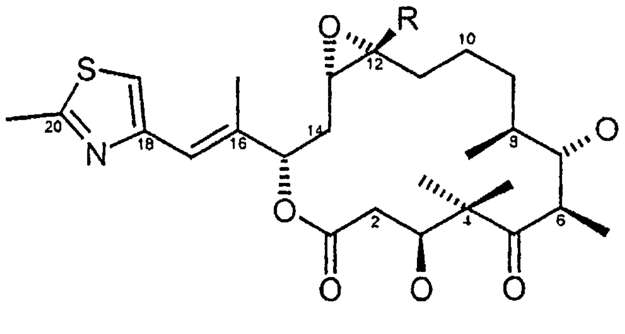

(I)

R = H, $CH_3$

fungizid und cytotoxisch wirken. Nach Hinweisen für eine in vitro Aktivität gegen Brust- und Darmtumorzelllinien erscheint diese Verbindungsklasse in besonderem Maße interessant für die Entwicklung eines Arzneimittels. Verschiedene Arbeitsgruppen beschäftigen sich daher mit der Synthese dieser makrocyclischen Verbindungen. Die Arbeitsgruppen gehen von unterschiedlichen Bruchstücken des Makrocyclus aus, um die gewünschten Naturstoffe zu synthestisieren.

In jedem Fall werden für eine erfolgreiche Epothilon-Synthese diastereomerenreine Bruchstücke als Ausgangs- und Zwischenprodukte benötigt. Diastereomerenreinheit ist oft entscheidend für die Wirkung und Sicherheit eines Arzneimittels und somit Voraussetzung für dessen Herstellung.

Die Totalsynthese von Epothilon A ist von Schinzer et al. in Chem. Eur. J. 1996, 2, No. 11, 1477-1482 und in Angew. Chem. 1997, 109, Nr. 5, S. 543-544) beschrieben.

Epothilon-Derivate wurden bereits von Höfle et al. in der WO 97/19086 beschrieben. Diese Derivate wurden ausgehend vom natürlichen Epothilon A oder B hergestellt.

Eine weitere Synthese von Epothilon und Epothilonderivaten wurde von Nicolaou et al. in Angew. Chem. 1997, 109, Nr. 1/2, S. 170 - 172 beschrieben. Die Synthese von Epothilon A und B und einiger Epothilon-Analoga wurde in Nature, Vol. 387, 1997, S. 268-272, die Synthese von Epothilon A und seinen Derivaten in J. Am. Chem. Soc., Vol. 119, No. 34, 1997, S. 7960- 7973 sowie die Synthese von Epothilon A und B und einiger Epothilon-Analoga in J. Am. Chem. Soc., Vol. 119, No. 34, 1997, S. 7974- 7991 ebenfalls von Nicolaou et al. beschrieben.

Ebenfalls Nicolaou et al. beschreiben in Angew. Chem. 1997, 109, Nr. 19, S. 2181-2187 die Herstellung von Epothilon A-Analoga mittels kombinatorischer Festphasensynthese. Auch einige Epothilon B-Analoga sind dort beschrieben.

Weitere Verfahren zur Herstellung von Epothilonderivaten werden in WO-A-9825929 beschrieben.

Aufgabe der vorliegenden Erfindung ist es, ein Verfahren zur Herstellung von Epothilon B und Derivaten bereitzustellen, bei welchem Epothilon aus preiswert und enantioselektiv erhältlichen Fragmenten als Ausgangsprodukte aufgebaut wird.

Eine weitere Aufgabe ist die Bereitstellung von Epothilon B bzw. dessen Derivaten in höheren Ausbeuten als nach den bisherbekannten Verfahren.

Die Herstellung von Epothilon B gemäß vorliegender Erfindung basiert auf der Verknüpfung der drei Teilfragmente **2**, **3** und **4** gemäß nachfolgendem Schema:

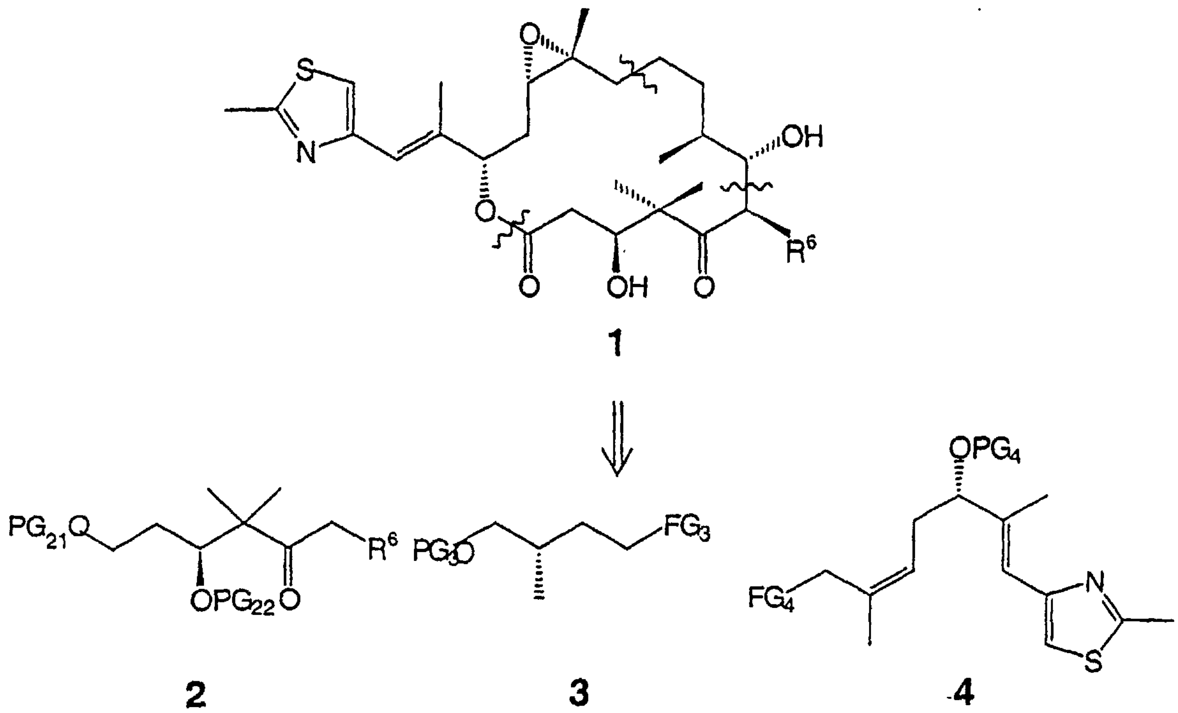

1

2 3 4

**2** bedeutet ein C1-C6-Fragment (Epothilon-Zählweise) der allgemeinen Formel:

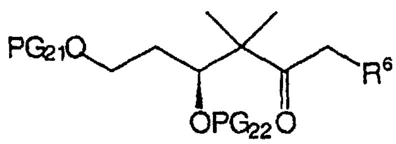

2

worin

$PG_{21}$ und $PG_{22}$ unabhängig voneinander jeweils eine Hydroxyschutzgruppe und

$R^6$ eine gerad- oder verzweigtkettige Alkylgruppe mit bis zu 6 Kohlenstoffatomen, eine Cycloatkylalkylgruppe mit bis zu 10 Kohlenstoffatomen, eine Phenyl-, 1- oder 2-Naphthyl-, Heteroaryl-, Benzyl- oder Methylheteroarylgruppe bedeuten.

**3** steht für ein C7-C10-Fragment (Epothilon-Zählweise) der allgemeinen Formel:

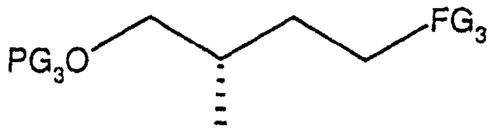

(3)

worin

$PG_3$ eine Hydroxyschutzgruppe und

$FG_3$ eine Phenylsulfonylgruppe
bedeuten.

**4** steht für ein C11-C20-Fragment (Epothilon-Zählweise) der allgemeinen Formel:

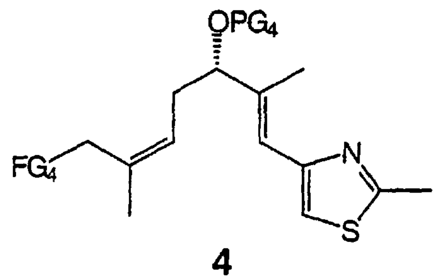

**4**

worin
$FG_4$ ein Iodatom oder eine andere Fluchtgruppe und
$PG_4$ eine Hydroxyschutzgruppe
bedeuten.

Synthese:

Darstellung von **2**:

Das Teilfragment **2** wird gemäß vorliegender Erfindung mittels der nachfolgenden Syntheseroute enantioselektiv aus sehr preiswerten Ausgangsverbindungen in effizienter Weise mit hohen Enantiomerenüberschüssen erhalten.
Die Substituenten haben -soweit nicht anders angegeben- die bereits vorstehend bei den einzelnen Fragmenten angegebenen Bedeutungen

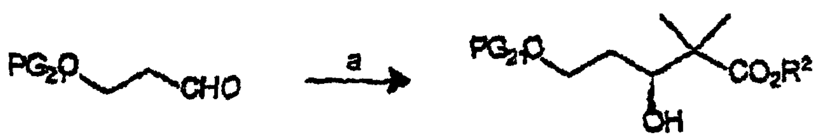


**2-I** **2-II**

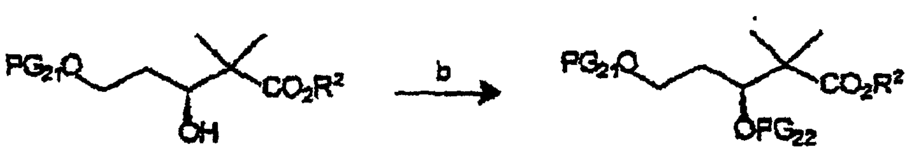


**2-II** **2-III**

Schritt a und b:

Das geschützte 3-Hydroxypropanal (2-I), welches analog zur Literatur (Kiyooka et al., J. Org. Chem., 1991, 58,2276-2278) aus 1,3-Propandiol durch Monoschutz und Oxydation hergestellt wurde, wird unter chiraler Katalyse mit einem Silylketenacetal der allgemeinen Formel

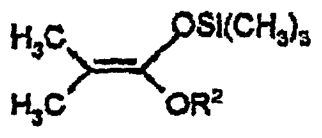

($R^2$ = Methyl, Ethyl)

unter Vermittlung durch N-Tosylvalin/Diboran zu (2-II). mit hohem enantiomeren Überschuss, umgesetzt In der Verbindung (2-II) wird anschließend die 3-Hydroxyfunktion nach, dem Fachmann bekannten Methoden, zur Darstellung der Verbindung der allgemeinen Formel (2-III) geschützt.

Als Alkyl-, Silyl- und Acylreste für die Schutzgruppen $PG_{21}$, $PG_{22}$, $PG_3$ und $PG_4$ kommen die dem Fachmann bekannten Reste in Betracht. Bevorzugt sind aus den entsprechenden Alkyl- und Silylethern leicht abspaltbare Alkyl- bzw. Silylreste, wie beispielsweise der Methoxymethyl-, Methoxyethyl, Ethoxyethyl-, Tetrahydropyranyl-, Tetrahydrofuranyl-, Trimethylsilyl-, Triethylsilyl-, tert.-Butyldimethylsilyl-, tert.-Butyldiphenylsilyl-, Tribenzylsilyl-, Triisopropylsilyl-, Benzyl-, para-Nitrobenzyl-, para-Methoxybenzyl-Rest sowie Alkylsulfonyl- und Arylsulfonylreste. Als Acylreste kommen z.B. Formyl, Acetyl, Propionyl, Pivalyl-, Butyryl oder Benzoyl, die mit Amino- und/oder Hydroxygruppen substituiert sein können, in Frage.
Eine Übersicht über Schutzgruppen findet sich z.B. in "Protective Groups in Organic Synthesis" Theodora W. Green, John Wiley and Sons.
Bevorzugt sind hierbei solche Schutzgruppen, die unter Einwirkung von Fluorid gespalten werden können, wie z.B. Trimethylsilyl-, tert.-Butyldimethylsilyl-, Triisopropyll-, Triethyl-silyl-, tert.-Butyldiphenylsilyl-Rest, und von diesen insbesondere der tert.-Butyldimethylsilyl-, der Triisopropylsilyl- und der tert.-Butyldiphenylsilyl-Rest.

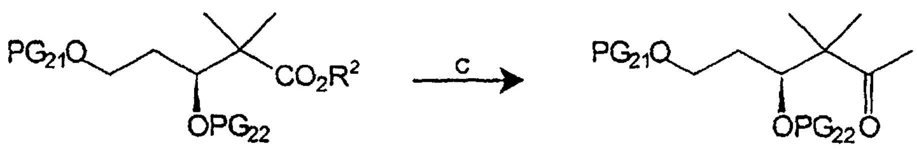


2-III 2-IV

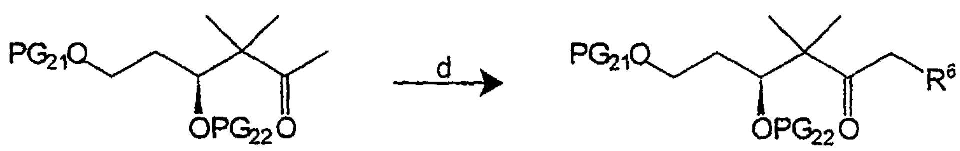


2-IV 2

Schritt c und d:

Verbindung (2-III) wird durch Umsetzung mit Trimethylsilylmethyllithium ins Methylketon (2-IV) verwandelt und wird anschließend nach, dem Fachmann bekannten Methoden, mit einem Alkyl-, Cycloalkylalkyl-, Aryl-, Heteroaryl-, Methylaryl- oder Methylheteroarylhalogenid der Formel (2-X), $R^6$-Hal, worin $R^6$ eine gerad- oder verzweigtkettige Alkylgruppe mit bis zu 6 Kohlenstoffatomen, eine Cycloalkylalkylgruppe mit bis zu 10 Kohlenstoffatomen, eine Phenyl-, 1-oder 2-Naphthyl-, Heteroaryl-, Benzyl- oder Methylheteroarylgruppe und Hal ein Halogenatom (Chlor, Brom oder Iod) bedeuten, zu der Verbindung der allgemeinen Formel **2** umgesetzt werden.
Alternativ kann Verbindung (2-III) auch nach, dem Fachmann bekannten Methoden, zum Alkohol reduziert, selektiv zum Aldehyd oxidiert und anschließend mit einem Metallorganyl Me-$CH_2$-$R^6$ (Me steht für ein Lithiumatom oder für einen Rest MgHal, Hal = Cl, Br; $R^6$ hat die oben angegebene Bedeutung) umgesetzt werden. Abschließende Oxidation liefert dann ebenfalls eine Verbindung der allgemeinen Formel **2**.
Als $C_1$ - $C_6$-Alkylgruppe für $R^6$ kommen beispielsweise eine Methyl, Ethyl, Propyl, Isopropyl, Butyl, Isobutyl, tert.-Butyl, Pentyl, Isopentyl, Neopentyl, Hexylgruppe in Frage.
Für eine Cycloalkylalkylgruppe $R^6$ mit bis zu 10 Kohlenstoffatomen ist beispielsweise die Cyclopropylmethyl-, Cyclobutylmethyl-, Cyclopentylmethyl- oder Cyclohexylmethylgruppe zu nennen. Der Heteroarylrest, entweder als $R^6$ oder in der Methylheteroarylgruppe $R^6$, kann zum Beispiel ein Fury-l, Thienyl-, Pyridyl-, Pyrazolyl-, Pyrimidinyl-, Oxazolyl-, Pyridazinyl-, Pyrazinyl-, Chinolyl-, Thiazolylrest sein.
Die vorstehend für $R^6$ möglichen Reste können einfach oder mehrfach substituiert sein durch Halogen, OH, O-Alkyl, $CO_2H$, $CO_2$-Alkyl, $-NH_2$, $-NO_2$, $-N_3$, -CN, $C_1$-$C_{20}$-Alkyl, $C_1$-$C_{20}$-Acyl, $C_1$-$C_{20}$-Acyloxy-Gruppen. Heteroatome in den Heteroarylresten können oxidiert sein

Darstellung von 3:

Die Substituenten haben -soweit nicht anders angegeben- die bereits vorstehend bei den einzelnen Fragmenten angegebenen Bedeutungen
Das Teilfragment **3** kann aus dem wohlfeit erhältlichen Hydroxyisobuttersäure-methylester in hoher optischer Reinheit hergestellt werden.

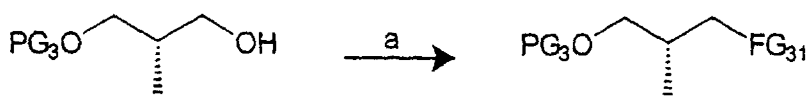


**3-I** **3-II**

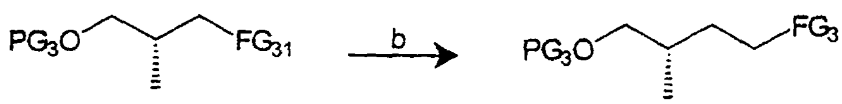


**3-II** **3**

Schritt a und b:

Verbindung (3-I) ist in bekannter Weise durch Monoschutz und Reduktion aus dem kommerziell erhältlichen Hydroxyisobuttersäure-methylester zugänglich. Die Verbindung (3-I) wird mit Tosylchlorid zur Verbindung (3-II) umgesetzt (dadurch Überführung der Hydroxygruppe in eine bessere Abgangsgruppe; anstelle des Tosylrestes sind auch andere, gängige Abgangsgruppen, wie Mesylat, Triflat etc. möglich); anschließende Addition eines Methylsulfonanions (im Rahmen der Beispiele vorliegender Erfindung ist dies Phenylsulfonylmethyl-Anion) liefert eine Verbindung der allgemeinen Formel **3**.

Darstellung von 4:

Die Substituenten haben -soweit nicht anders angegeben- die bereits vorstehend bei den einzelnen Fragmenten angegebenen Bedeutungen
Die Teilfragmente der Formel **4** können aus wohlfeiler, preiswert erhältlicher Äpfelsäure in effizienter Weise mit sehr hoher optischer Reinheit hergestellt werden.
Die Synthese wird im folgenden am Beispiel der L-(-)-Äpfelsäure beschrieben. Ausgehend von D-(+)-Äpfelsäure erhält man die entsprechenden enantiomeren Verbindungen, bzw ausgehend von racemischer Äpfelsäure die entsprechenden racemischen Verbindungen.

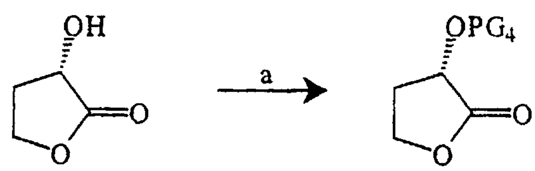


**4-I** **4-II**

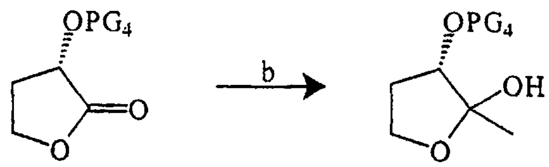


4-II 4-III

Schritte a und b:

Die Verbindung (4-I) ist kommerziell oder nach bekannten Verfahren aus der gängigen Literatur (Green et al., Tetrahedron, 1995, 51, 2865-2874) erhältlich. Die Verbindung (4-I) wird nach den, dem Fachmann bekannten Methoden geschützt. Als Schutzgruppen kommen die bereits vorstehend unter "Schritt a und b" näher aufgeführten Schutzgruppen in Frage. Im zweiten Schritt wird Verbindung (4-II) durch Addition von Methyllithium zur Hemiacetalverbindung umgesetzt, welche mit der offenkettigen Form (4-III) im Gleichgewicht steht.

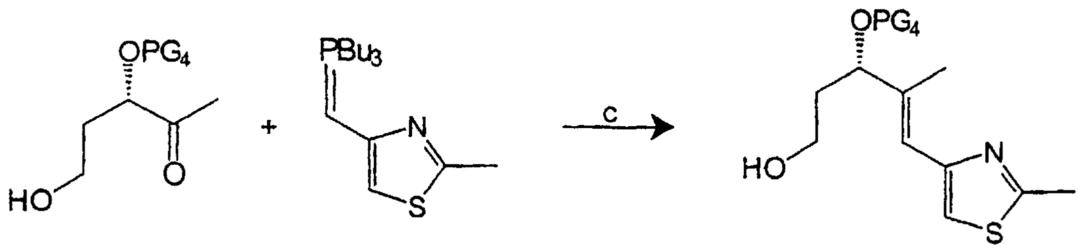


4-III 4-IV

Schritt c:

Verbindung (4-III) reagiert hoch (*E*)-stereoselektiv, aus der im Gleichgewicht vorliegenden offenen Form, mit dem (2-Methyl-thiazol-4-yl-methyl)-tributyl-phosphoniumylid, das durch Deprotonierung des entsprechenden Chlorids- oder Bromids erhalten wurde, zur Verbindung (4-IV).

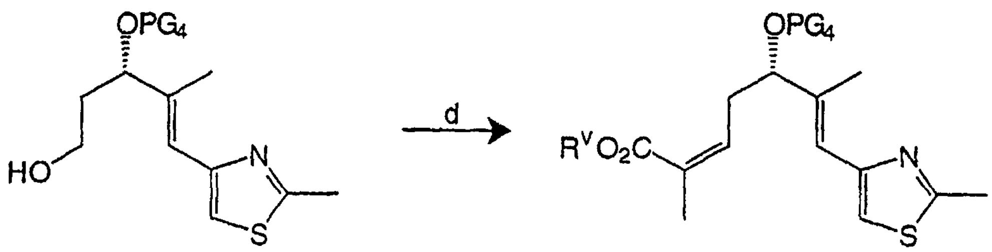


4-IV 4-V

Schritt d:

Nach selektiver Oxidation der Verbindung (4-IV) zum Aldehyd wird dieser nach einer, dem Fachmann bekannten Methode, über eine Still-Genari-Wittig Reaktion (*Z*)-stereospezifisch in die Verbindung (4-V) umgewandelt.

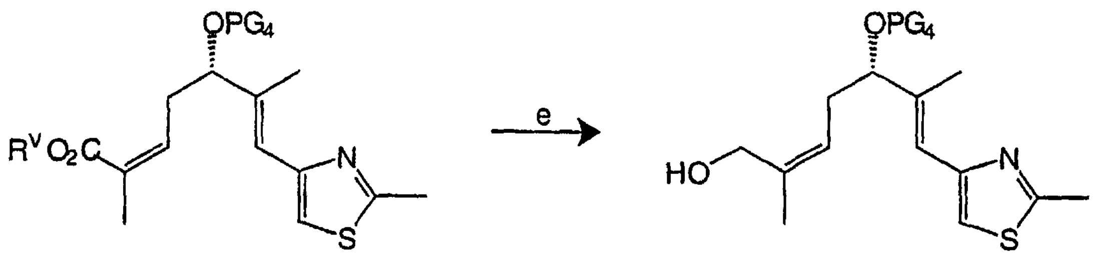


4-V 4-VI

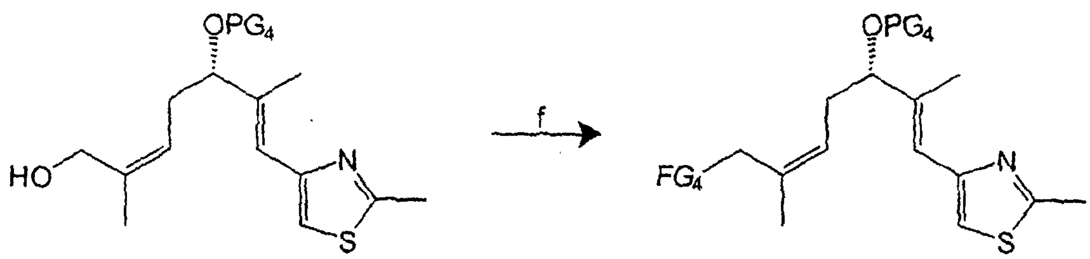


4-VI 4

Schritte e und f:

Nach Reduktion der Verbindung (4-V) zum Allylalkohol (4-VI) wird die entständige Hydroxylgruppe durch Überführung in eine bessere Abgangsgruppe, beispielsweise in das Iodid, für die Verknüpfung funktionalisiert und liefert die Verbindung der allgemeinen Formel **4**.

Als weitere Fluchtgruppen seien beispielsweise das Mesylat, Tosylat sowie Triflat genannt.

Die Herstellung der Verbindungen 4-V und 4-VI kann auch wie in der internationalen Patentanmeldung PCT/ EP 98/ 04462 beschrieben durchgeführt werden. Die einzelnen Fragmente 2, 3 und 4 werden wie in der internationalen Patentanmeldung PCT/ EP 98/ 04462 gezeigt und nachstehend genauer beschrieben zusammengefügt.

Zunächst ist hierzu die Synthese eines Kupplungsfragmentes 34-1 aus den einzelnen Fragmenten 3 und 4 durchzuführen. Dieses C7-C20-Kupplungsfragment 34-1 (Epothilon-Zählweise) wird durch anionische, sulfonstabilisierte Kupplung und anschließende reduktive Desulfonierung

hergestellt.

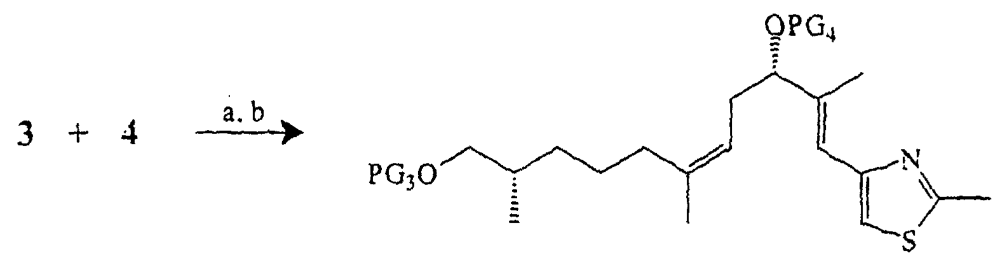


34-I

Schritte a und b:

Nach Deprotonierung der Verbindung **3** wird an Verbindung **4** anionisch gekuppelt. Anschließende reduktive Desulfonierung nach, dem Fachmann bekannten Methoden, liefert das C7-C20-Kupplungsfragment (34-I).

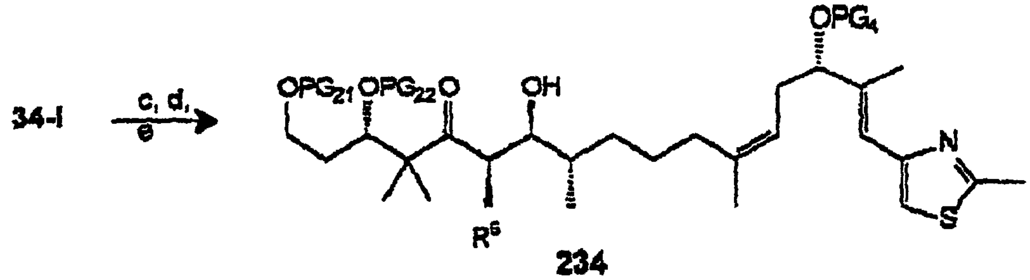


**234**

Schritte c, d und e:

Nach Entschützen der primären Hydroxylfunktion (Abspaltung von $PG_3$) In Verbindung 34-1 und selektiver Oxidation zum Aldehyd wird mit der Verbindung **2** in Form einer Aldolreaktion das C1-C20-Kuppiungsfragment (234) dargestellt.

Das Epothlion B 1 bzw. ein Derivat hiervon wird abschließend aus Verbindung (234) analog zur bestehenden Literatur (K.C. Nicolaou et al., Nature, Vol. 387, 1997, S. 268-272 und J. Am. Chem, Soc.1097,119, S. 7960 - 7973) in 8 weiteren synthetischen stufen dargestellt.

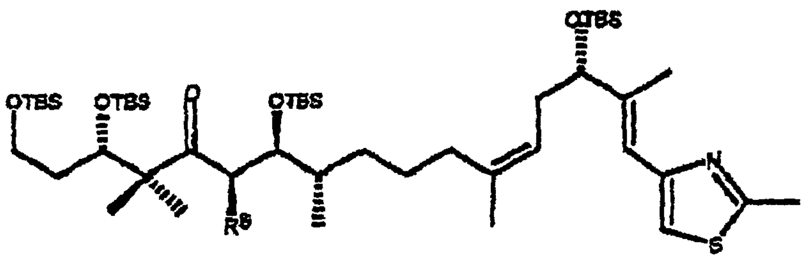

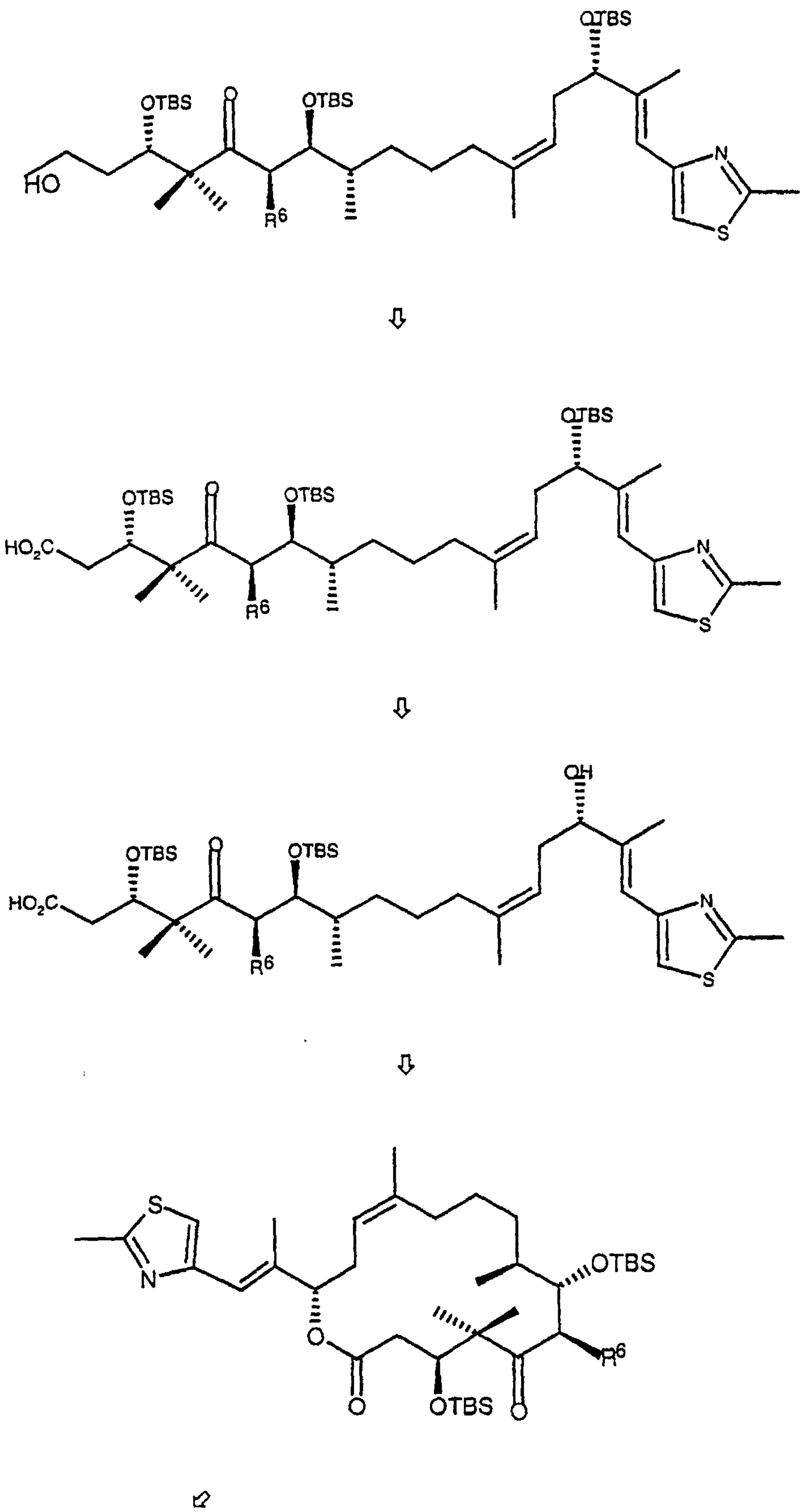

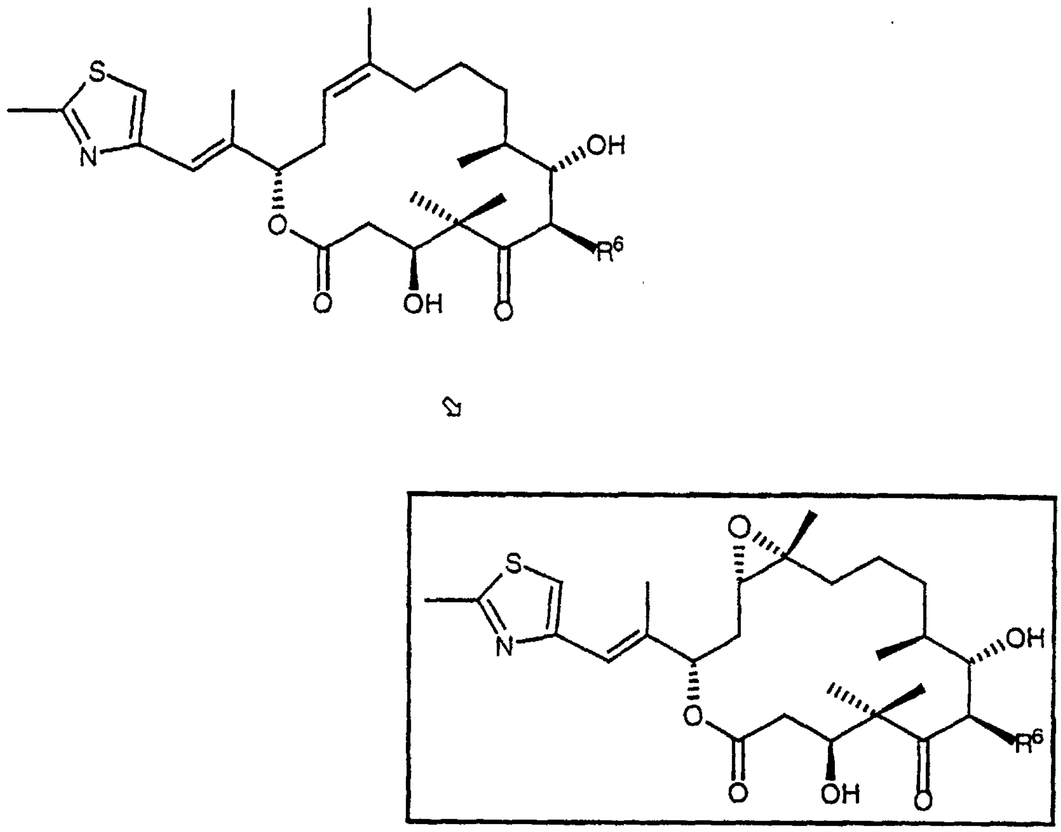

Die nachfolgenden Beispiele dienen der näheren Erläuterung der Erfindung, ohne sie darauf einschränken zu wollen:

**Beispiel A - C: Herstellung der Verbindung der Formel 3**

Beispiel A:

**(3S)-4-[(1,1 -Dimethylethyl)diphenylsilyloxy]-3-methyl-1-phenylsulfonylbutan**

In 20 mL abs. Pyridin werden bei 0 °C 6.57 g (20 mmol) TBDPS geschütztes (2S)-Methylpropan-1,3-diol mit 6,94 g (40 mmol) Tosylchlorid versetzt und 3.5 h gerührt (Pyr·HCl-Salze fallen aus). Zur Aufarbeitung wird Eis zugegeben und 1 h nachgerührt. Nun wird mit Ether mehrfach extrahiert, mit ges. $NaHCO_3$-Lsg. gewaschen, über $MgSO_4$ getrocknet, filtriert und im Vakuum am Rotationsverdampfer eingeengt.
Das noch nach Pyridin riechende Rohprodukt wird über eine 10:1-Hex/EE-Kieselgelsäule flashchromatographiert. Man erhielt 8.67 g (92.5%) tosyliertes Produkt, welches direkt weiterverarbeitet wurde.
In 180 mL abs. THF werden bei -20°C 4.34 g (27.9 mmol, 1.5 eq) Methylphenylsulfon mit 16.77 mL (26.832 mmol, 1.45 eq) einer 1.6 M-nBuLi-Lsg. versetzt und langsam (30 min) auf RT erwärmen gelassen (zunächst orange, klare Lsg. trüb sich ein). Nun wird das Tosylat, gelöst in etwa 20 mL abs. THF, bei RT zugegeben und ca. 12 h nachgerührt.
Die violette Lsg. wird mit ges. $NH_4Cl$-Lsg. gequenscht und dann NaK-Tartrat-Lsg. zugegeben, bis zwei klare Phasen entstehen. Die Phasen werden getrennt, die wässerige Phase noch zweimal mit Ether extrahiert, die vereinigten organischen Phasen über $MgSO_4$ getrocknet und über eine kurze Kieselgelfritte filtriert, mit Ether nachgewaschen und im Vakuum am Rotationsverdampfer eingeengt.
Nach Chromatographie über eine 5:1-Hex/EE-Kieselgelsäule wurden 7.689 g der Titelverbindung, als zähes, farbloses Öl und noch 630 mg tosylierten Produkts, farblos, erhalten. Man erhält somit in 89.1% bzw. unter Beachtung des rückgewonnenen Eduktes in 96.0% Ausbeute die Titelverbindung (über die zwei chemischen Umsetzungen somit 82.4% bzw. 88.8% Ausbeute).

**$R_f$-Wert** vom tosylierten Produkt (Hex/EE= 3:1) ≈ 0.6 F II (blau),
F III (schwach blau),
**$R_f$-Wert** der Titelverbindung (Hex/EE= 3:1) ≈ 0.39 F II (schwach blau);

**$^1$H-NMR** (400 MHz, $CDCl_3$): δ in ppm =
0.84 (d, $J$= 6.4 Hz, 3H, 3-$CH_3$); 0.96 (s, 9H, -SiC$(CH_3)_3$); 1.60 (ddt, $^2J$= 12.8 Hz, $J_{2a\text{-}H,\ 3\text{-}H}$= 8.9 Hz, $J_{2a\text{-}H,1\text{-}H}$= 7.4 Hz, 1H, 2a-H); 1.70 (mc, 1H, 3-H); 1.86 (dddd, $^2J$= 12.8 Hz, $J_{2b\text{-}H,3\text{-}H}$= 8.9 Hz, $J_{2b\text{-}H,1\text{-}H}$= 7.9 Hz, $J_{2b\text{-}H,1\text{-}H}$= 5.9 Hz, 1H, 2b-H); 3.09 (mc, 2H, 1-H); 3.36 (dd, $^2J$= 9.8 Hz, $J_{4a\text{-}H,3\text{-}H}$= 6.4 Hz, 1H, 4a-H); 3.44 (dd, $^2J$= 9.8 Hz, $J_{4b\text{-}H,3\text{-}H}$≅ 5.0 Hz, 1H, 4b-H); 7.37 (m, 6H, $CH_{arom}$); 7.55 (m, 6H, $CH_{arom}$); 7.63 (m, 1H, (Ts)$p$-$CH_{arom}$); 7.88 (mc, 2H, (Si) $p$-$CH_{arom}$).

**MS** (EI): m/e =
468 [M+1]; 467 [M]; 460; 425; 414; 383; 382; 326; 267; 252; 213; 182; 136; 57.

**Drehwert:**

$$[\alpha]_D^{20} = -5.8;\ (c = 2.01;\ CHCl_3)$$

| | | | | |
|---|---|---|---|---|
| **$C_{27}H_{34}O_3SSi$** (M= 466.71 g·mol$^{-1}$) | **EA** | ber.<br>gef. | C: 69.5 %<br>C: 69.37 % | H: 7.3 %<br>H: 7.50 % |

Beispiel B:

**(3S)-4-Hydroxy-3-methyl-1-phenylsulfonylbutan**

In 140 mL abs. THF werden 7.5 g (16.07 mmol) des nach Beispiel A hergestellten Sulfons mit 22 mL (24.105 mmol) einer 1.1 M TBAF-Lsg. versetzt und 10 h bei RT gerührt. Die Reaktion wird mit ges. $NH_4Cl$-Lsg. gequenscht, die Phasen werden getrennt, die wässerige Phase noch zweimal mit Ether extrahiert, die vereinigten organischen Phasen über $MgSO_4$ getrocknet, filtriert und im Vakuum am Rotationsverdampfer eingeengt.

Nach Chromatographie über eine 1:1-Hex/EE-Kieselgelsäule wurden 3.21 g (87.5%) der Titelverbindung, als farbloses, zähes Öl, erhalten.

**$R_f$-Wert** (Hex/EE= 1:1) ≈ 0.12 F III (schwach blau);

**$^1$H-NMR** (400 MHz, $CDCl_3$): δ in ppm =
0.87 (d, $J$= 6.9 Hz, 3H, 3-$CH_3$); 1.52 (t, $J_{4\text{-}OH,4\text{-}H}$= 4.9 Hz, 1H, 4-OH); 1.60 (mc, 1H, 2a-H); 1.71 (mc, 1H, 3-H); 1.85 (ddt, $^2J$= 12.8 Hz, $J_{2b\text{-}H,3\text{-}H}$= 9.4 Hz, $J_{2b\text{-}H,1\text{-}H}$= 5.9 Hz, 1H. 2b-H); 3.15 (mc, 2H, 1-H); 3.40 (ddt, 2H, 4-H); 7.55 (mc, 2H, $CH_{arom}$); 7.64 (mc, 1H, $CH_{arom}$); 7.89 (mc, 2H, $CH_{arom}$).

**$^{13}$C-NMR** (100 MHz, $CDCl_3$): δ in ppm =
16.3 (C-5); 26.1 (C-2); 34.6 (C-3); 54.3 (C-1); 67.4 (C-4); 128.0 + 129.3 (C-7 u. C-8); 133.7 (C-9); 139.1 (C-6).

**IR** (Si-Film): ν in cm$^{-1}$ =
3510br; 3063m; 2959s; 2934s; 2878s; 2668w; 1585w; 1447s; 1407m; 1302m; 1041m; 986m; 908m; 789s; 739s.

**MS** (EI, 70 eV, 100°C): m/e =
227 [M]; 210; 198 (58); 181 (8); 169 (13), 156 (17); 143 (100); 132 (26); 125 (37); 105 (30); 94 (15); 91 (27); 87 (83); 78 (85); 77 (97); 69 (93); 51 (30).

**$C_{23}H_{29}NO_4S$:**
(M= 228.30 g·mol$^{-1}$)

Beispiel C:

**(3S)-4-[(1,1-Dimethylethyl)dimethylsilyloxy]-3-methyl-1-phenylsulfonylbutan** (Verbindung der Formel 3)

In 30 mL abs. DMF werden 5.548 g (24.3 mmol) der nach Beispiel B hergestellten Verbindung mit 3.31 g (2 eq, 48.6 mmol) Imidazol vorgelegt. Nun werden 4.75 g (1.3 eq, 31.59 mmol) TBSCI langsam bei 0°C zugegeben und 3 h gerührt, wobei die Temperatur langsam auf RT ansteigen darf.

Zur Aufarbeitung werden 50 mL ges. $NH_4Cl$-Lsg. bei 0°C zugefügt und mit Ether verdünnt. Die Phasen werden getrennt, die wässrige Phase wird noch dreimal mit Ether extrahiert, die vereinigten organischen Phasen mit Wasser gewaschen, über $MgSO_4$ getrocknet, über 3 cm Kieselgel filtriert und im Vakuum eingeengt.

Chromatographische Reinigung über eine 5:1-Hex/EE-Kieselgelsäule erbrachte 8.216 g (98.7%) der Titelverbindung.

**$^1$H-NMR** (400 MHz, $CDCl_3$): δ in ppm =
-0.03 (s, 6H, -$Si(CH_3)_2$); 0.81 (s, 9H, -$SiC(CH_3)_3$); 0.83 (d, *J*= 6.5 Hz, 3H, 3-$CH_3$); 1.55 (m, 1H, 2a-H); 1.65 (mc, 1H, 3-H); 1.79 (m, 1H, 2b-H); 3.14 (dd, *J*= 9.0 Hz, *J*= 7.0 Hz, 2H, 1-H); 3.31 (dd, $^2J$= 10.0 Hz, $J_{4a\text{-}H,3\text{-}H}$ = 6.5 Hz, 1H, 4a-H); 3.42 (dd, $^2J$= 10.0 Hz, $J_{4b\text{-}H,3\text{-}H}$≅ 5.0 Hz, 1H, 4b-H); 7.55 (m, 2H, *p*-$CH_{arom}$); 7.64 (m, 1H, *m*-$CH_{arom}$); 7.90 (m, 2H, *o*-$CH_{arom}$).

**$^{13}$C-NMR** (100 MHz, $CDCl_3$): δ in ppm =
-5.5 ($Si(CH_3)_2$); 16.3 (3-$CH_3$); 18.2 (Si**C**$(CH_3)_3$); 25.8 (Si**C**$(CH_3)_3$); 26.4 (C-2); 34.6 (C-3); 54.6 (C-1); 67.5 (C-4); 128.1 (*o*-$CH_{arom}$); 129.2 (*m*-$CH_{arom}$); 133.5 (*p*-$CH_{arom}$); 139.1 (*i*-$CH_{arom}$).

**IR** (Si-Film): ν in $cm^{-1}$ =
2957s; 1586w; 1447s; 1389m; 1306s; 1106vs; 740s; 689s; 562s.

**MS** (FI, 7kV, 3mA, 40°C): m/e =
343 ([M]); 310; 288; 287; 286; 285; 252; 224; 202; 182; 166; 125; 110; 78; 58; 57.

**Drehwert:**

$$[\alpha]_D^{20} = -6.7;\ (c= 2.64;\ CHCl_3)$$

| | | | | |
|---|---|---|---|---|
| **$C_{17}H_{30}O_3SSi$** (M= 342.56 g-mol$^{-1}$) | **EA** | ber. | C: 59.6% | H: 8.8% |
| | | gef. | C: 59.38% | H: 8.72 % |

**Beispiel D - J: Herstellung der Verbindung 4**

Beispiel D:

**(3S)-3-[(1,1-Dimethylethyl)dimethylsilyloxy]-oxolan-2-on**

in 50 mL abs. DMF werden 3.92 g (38.4 mmol) (S)-3-Hydroxybutyrolacton mit 5.23 g (2 eq, 76.8 mmol) Imidazol bei 0°C vorgelegt. Nun werden 7.53 g (1.3 eq, 49.92 mmol) TBSCl langsam zugegeben und 2.5 h gerührt.

Zur Aufarbeitung wird mit 100 mL Ether verdünnt und durch Zugabe von 100 mL ges. $NH_4Cl$-Lsg. die Reaktion gequenscht. Die Phasen werden getrennt, die wässrige Phase wird noch dreimal mit Ether extrahiert, die vereinigten org. Phasen mit Wasser gewaschen, über $MgSO_4$ getrocknet, über 3 cm Kieselgel filtriert und im Vakuum eingeengt.

Chromatographische Reinigung über eine 15:1-Hex/EE-Kieselgelsäule erbrachte 8.272 g (99.6%) der Titelverbindung, als farbloses Öl (kristallisiert bei < -20°C).

**$R_f$-Wert** (Hex/EE= 1:1) ≈ 0.70
**$R_f$-Wert** (Hex/EE= 5:1) ≈ 0.51 F I (blau);

**$^1$H-NMR** (400 MHz, $CDCl_3$): δ in ppm =
0.14 (s, 3H, -$SiCH_3$); 0.17 (s, 3H, -$SiCH_3$); 0.91 (s, 9H, -$SiC(CH_3)_3$); 2.22 (dddd, *J*= 12.8 Hz, $J_{4\text{-}Ha,3\text{-}H}$= 9.0

Hz, $J_{4\text{-Ha},5\text{-Ha}}$= 9.0 Hz, $J_{4\text{-Ha},5\text{-Hb}}$= 8.6 Hz, 1H, 4-Ha); 2.45 (m, 1H, 4-Hb); 4.19 (td, $J_{5\text{-Ha},4\text{-Ha u. 5-Hb}}$= 9.0 Hz, $J_{5\text{-Ha},4\text{-Hb}}$= 6.6 Hz, 1H, 5-Ha); 4.34-4.43 (m, 2H, 5-Hb u. 3-H).

**$^{13}$C-NMR** (100 MHz, $CDCl_3$): δ in ppm =
- 5.3 ($SiCH_3$); -4.2 ($SiCH_3$); 18.2 (Si**C**$(CH_3)_3$); 25.6 (Si**C**$(CH_3)_3$); 32.3 (C-4); 64.7 (C-5); 68.2 (C-3); 175.8 (C-2).

**IR** (Si-Film): ν in $cm^{-1}$ =
2956s; 2931s; 2887m; 2858s; 1788vs; 1473w; 1464w; 1362w; 1254m; 1220m; 1154vs; 1022s; 999s; 947m; 888m; 840s.

**MS** (FI, 7kV, 3mA, 20°C): m/e =
217 ([M+1], <1); 161 (4); 160 (11); 159 ([M-tBu], 100); 132 (<1).

**Drehwert:**

$$[\alpha]_D^{20} = -30.5; \qquad (c= 5.82;\ CHCl_3)$$

**$C_{10}H_{20}O_3Si$:**
(M= 216.35 g·mol$^{-1}$)

Beispiel E:

**(3S)-3-[(1,1-Dimethylethyl)dimethylsilyloxy]-2-methyl-oxolan-2-ol** bzw. **(3S)-3-[(1,1-Dimethylethyl)dimethylsilyloxy]-5-hydroxy-pentan-2-on**

In 100 mL abs. THF werden 5.27 g (ex 24.34 mmol (S)-3-Hydroxybutyrolacton) roher Verbindung von Beispiel D bei -78°C tropfenweise mit 18.26 mL (≥1.2 eq, 29.216 mmol) einer 1.6M MeLi-Lsg. (in Ether, *Fluka*) versetzt und 90 min nachgerührt. Das Kühlbad wird entfernt und die Reaktion durch zügige, aber kontrollierte Zugabe von ges. $NH_4Cl$-Lsg. gequenscht. Man läßt die Reaktionslösung wird auf RT erwärmen. Nun wird mit ges. NaK-Tartrat-Lsg. versetzt, bis sich zwei klare Phasen bilden. Die Lösung wird mit Ether verdünnt, die Phasen werden getrennt, die wässrige Phase wird noch zweimal mit Ether extrahiert, die vereinigten org. Phasen, über $MgSO_4$ getrocknet, filtriert und im Vakuum eingeengt.

Chromatographische Reinigung über eine 10:1-5:1-Hex/EE-Gradientenkieselgelsäule erbrachte 4.958 g (87.7% über die zwei Stufen) der Titelverbindung(en), als farblos, kristalline Verbindung.

Aufgrund des vorliegenden Gleichgewichtes und der Diastereomerenmischung erfolgt keine vollständige Charakterisierung des Produktes.

**$R_f$-Wert** (Hex/EE= 5:1) ≈ 0.22 F I (blau);
F III (grün);

**MS** (FI, 7kV, 3mA, 20°C): m/e =
214 ([M-$H_2O$]); 203; 189; 176; 175 ([M-tBu]); 157; 132.

| | | | | |
|---|---|---|---|---|
| **$C_{11}H_{24}O_3Si$** | **EA** | ber. | C: 56.9% | H: 10.4% |
| (M= 232.39 g·mol$^{-1}$) | | gef. | C: 57.15 % | H: 10.34 % |

Beispiel F:

**4-Chlormethyl-2-methyl thiazol**

Eine Lösung von 1,3-Dichlorpropan-2-on (4.82 g, 38 mmol) und Thioacetamid (7.5 g, 0.1 mol) in wasserfreiem Ethanol (70 mL) wurde unter Argon 5.5 h zum Sieden erhitzt und dann im Vakuum eingeengt. Der dunkel gefärbte kristalline Rückstand wurde in Wasser (etwa 250 mL) gelöst, die wässrige Lsg. mehrfach mit Diethylether gewaschen und durch Zugabe von $NaHCO_3$ auf pH≈8 gebracht und danach mit Diethylether (viermal 100 mL) ausgeschüttelt. Die

getrocknete, dunkle org. Phase wurde über eine mit Kieselgel beschickte Fritte filtriert, mit Hex/EE (3:1) nachgewaschen und eingeengt.

Der Rückstand wurde durch Kugelrohrdestillation (Badtemp.: 90-95°C, 10 Torr) gereinigt. Man erhielt 11.91 g (81%) der Titelverbindung, als ein leicht gelbliches, hautreizenden Öls.

Beispiel G:

**(2-Methyl thiazol-4-yl)-methyl-tri-n-butyl-phosphonium-chlorid**

Einer Lösung von 4-Chlormethyl-2-methylthiazol (23 g, 0.156 mol) in wasserfreiem Benzen (200 mL) wurde unter Argon Tri-*n*-butylphosphan (38 mL, 0.156 mol) zugefügt und die Lsg. 8 h zum Sieden erhitzt. Anschließend wurde im Vakuum eingeengt, der Rückstand noch 30 min bei 0.01 Torr getrocknet und das Phosphoniumsalz durch Verreiben mit trockenem Diethytether zur Kristallisation gebracht. Man erhielt 53 g (97%) der Titelverbindung, als farblose, hygroskopische Kristalle.

Eine Analysenprobe wurde mit wässriger KBr-Lsg. ins entsprechende Bromid überführt und durch Flashchromatographie an Kieselgel (EE/MeOH, 4:1) gereinigt (Schmp.: 102-105°C).

**$^1$H-NMR** (400 MHz, $CDCl_3$): δin ppm =
0.86 (t, $J_{HH}$= 7.0 Hz, 9H, $CH_2C\mathbf{H}_3$); 1.35-1.50 (m, 12H, $CH_2$); 2.35 (mc, 6H, P-C$\mathbf{H}_2$-R); 2.59 (s, 3H, 2-$CH_3$); 4.28 (d, $^2J_{HP}$= 14.6 Hz, 2H, P-C$\mathbf{H}_2$-TAr); 7.65 (d, $^4J_{HP}$= 3.5 Hz, 1H, 5-H).

**$^{13}$C-NMR** (100 MHz, $CDCl_3$): δ in ppm =
13.2 (-$CH_2\mathbf{C}H_3$); 19.0 (2-$CH_3$); 19.0 ($^1J_{PC}$ = 46.8 Hz, P-$CH_2$-R); 22.6 ($^1J_{PC}$= 47.4 Hz, P-$CH_2$-TAr); 23.4 ($J_{PC}$ = 5.4 Hz, -$CH_2$-); 23.8 ($J_{PC}$= 15.3 Hz, -$CH_2$-); 119.8 ($^3J_{PC}$= 9.2 Hz, C-5); 142.9 ($^2J_{PC}$= 9.9 Hz, C-4); 166.7 ($^4J_{PC}$= 1.5 Hz, C-2).

Beispiel H:

**(3S,4*E*)-3-[(1,1-Dimethylethyl)dimethylsilyloxy]-4-methyl-5-(2-methylthiazol-4-yl)pent-4-enol**

16.8 g (48.0 mmol) der nach Beispiel G hergestellten Verbindung werden in 200 mL abs. THF bei -78°C mit einer Lösung von 1.1 eq, NaHMDS/KHMDS (ca. 1:1), gelöst in 70 mL abs. THF, deprotoniert. Nach 40 min werden 4.914 g (21.145 mmol) der nach Beispiel E hergestellten Verbindung, gelöst in 20 mL abs. THF, langsam zugegeben.

Nach 1 h bei -78°C (praktisch kein Umsatz), wird das Kühlbad entfernt, auf ca. 40°C erwärmt und 20 min nachgerührt.

Zur Aufarbeitung wird mit 100 mL Ether verdünnt und durch Zugabe von 250 mL ges. $NH_4Cl$-Lsg. gequenscht. Anschließend werden die Phasen getrennt, die wässrige Phase wird noch dreimal mit Ether extrahiert, die vereinigten org. Phasen werden über $MgSO_4$ getrocknet, filtriert und eingeengt.

Zweimalige chromatographische Reinigung über eine 3:1-Hex/EE-Kieselgelsäule erbrachte insgesamt 559 mg (noch leicht verunreinigtes) Produkt und 4.9 g der Titelverbindung. (Ausbeute 78.8% im Verhältnis 1:9), als zähe, farblose Öle, die im Tiefkühler (<-20°C) kristallisieren. (Bei der unpolareren Verbindung handelt es sich nicht um das vermutete DB-Isomer, sondern um die C1-silyfierte Verbindung aufgrund eines Silylshifts unter den Reaktionsbedingungen.)

**$R_f$-Wert** (Hex/EE= 3:1) unpol. Produkt ≈ 0.32 beide F I (blau);
**$R_f$-Wert** (Hex/EE= 3:1) Titeiverbindung ≈ 0.21

**$^1$H-NMR** (400 MHz, $CDCl_3$) unpol. Produkt: δ in ppm =
0.068 (s, 3H, -$SiCH_3$); 0.072 (s, 3H, -$SiCH_3$); 0.90 (s, 9H, -$SiC(CH_3)_3$); 1.82 (m, 2H, 2-H); 2.02 (s, 3H, 4-$CH_3$); 2.69 (s, 3H, TAr-$CH_3$); 3.46 (d, *J*= 2.5 Hz, 1H, 3-OH); 3.79-3.90 (m, 2H, 1-H); 4.36 (t, *J*= 6.0 Hz, 1H, 3-H); 6.59 (s, 1H, 5-H); 6.91 (S, 1H, TAr-$CH_{arom}$).

**$^{13}$C-NMR** (100 MHz, $CDCl_3$) unpol. Produkt: δ in ppm =
-5.53 ($SiCH_3$); -5.51 ($SiCH_3$); 14.8 (4-$CH_3$); 18.1 (Si$\mathbf{C}(CH_3)_3$); 19.2 (TAr-$CH_3$); 25.9 (Si$\mathbf{C}(CH_3)_3$); 37.1 (C-2); 62.2 (C-1); 76.9 (C-3); 115.3 (C-7); 118.2 (C-5); 141.8 (C-4); 153.2 (C-6); 164.4 (C-8).

**Drehwert:**

$$[\alpha]_D^{20} = -7.5;\ (c = 2.3;\ CHCl_3)$$

| | | | | | |
|---|---|---|---|---|---|
| **$C_{16}H_{29}NO_2SSi$** (M= 327.55 g·mol$^{-1}$) | **EA** | ber. | C: 58.7 % | H: 8.9 % | N: 4.3 % |
| | | gef. | C: 58.48 % | H: 8.85 % | N: 4.41 % |

**$^1$H-NMR** (400 MHz, $CDCl_3$) Titelverbindung: δ in ppm =
0.01 (s, 3H, $-SiCH_3$); 0.07 (s, 3H, $-SiCH_3$); 0.88 (s, 9H, $-SiC(CH_3)_3$); 1.73-1.90 (m, 2H, 2-H); 1.98 (s, 3H, 4-$CH_3$); 2.28 (t, *J*= 5.0 Hz, 1H, 1-OH); 2.67 (s, 3H, TAr-$CH_3$); 3.71 (m, 2H, 1-H); 4.35 (dd, $J_{3\text{-H}, 2'\text{-H}}$= 7.5 Hz, $J_{3\text{-H}, 2\text{-H}}$= 4.5 Hz, 1H, 3-H); 6.49 (s, 1H, 5-H); 6.89 (s, 1H, TAr-$CH_{arom}$).

**$^{13}$C-NMR** (100 MHz, $CDCl_3$) Titelverbindung: δ in ppm =
-4.8 ($SiCH_3$); -4.2 ($SiCH_3$); 14.7 (4-$CH_3$); 18.5 (Si**C**$(CH_3)_3$); 19.6 (TAr-$CH_3$); 26.2 (Si**C**$(CH_3)_3$); 38.7 (C-2); 60.6 (C-1); 77.7 (C-3); 115.7 (C-7); 119.2 (C-5); 142.0 (C-4); 153.4 (C-6); 164.9 (C-8).

**IR** (Si-Film): ν in cm$^{-1}$ =
3385br; 2954vs; 2928vs; 2885m; 2856s; 1655w; 1508w; 1472m; 1462m; 1440w; 1388w; 1360w; 1255m; 1184w; 1098vs; 1027s; 1005s; 978m; 939w; 884m; 837vs; 777vs; 738m.

**MS** (FI, 7kV, 3mA, 20°C): m/e =
327([M]); 270; 269; 223; 222; 215; 197; 195; 175; 165; 133; 132; 113; 112.

**Drehwert:**

$$[\alpha]_D^{20} = -31.5;\ (c = 2.81;\ CHCl_3)$$

**$C_{16}H_{29}NO_2SSi$:**
(M= 327.55 g·mol$^{-1}$)

Beispiel I:

**(5S, 2*Z*, 6*E*)-2,6-Dimethyl-5-[(1,1-dimethylethyl)-dimethylsilyloxy]-7-(2-methylthiazol-4-yl)hepta-2,6-diensäureethylester**

In 150 mL abs. $CH_2Cl_2$ werden 1.57 mL (1.2 eq, 17.95 mmol) Oxalylchlorid bei -78°C langsam mit 2.66 mL (2.5 eq, 37.4 mmol) DMSO versetzt und 10 min nachgerührt. Nun werden 4.9 g (14.96 mmol) der nach Beispiel H hergestellten Verbindung, gelöst in 20 mL abs. $CH_2Cl_2$, langsam zugetropft. Nach 45 min werden 12.8 mL (5 eq, 74.8 mmol) Hünigbase zugegeben, das Kühlbad wird entfernt und man läßt die Temperatur der Reaktionslösung über einen Zeitraum von 1 h auf RT ansteigen. Nun wird mit Ether verdünnt, mit ges. $NH_4Cl$-Lsg. gequenscht, die Phasen getrennt, die org. Phase noch sukzessive mit Wasser, ges. $NaHCO_3$-Lsg., Wasser und Brine gewaschen, über $MgSO_4$ getrocknet, filtriert und im Vakuum eingeengt.

Nach ausgiebigem Trocknen im Hochvakuum (ca. 0.1 mbar), wird der Rohaldehyd so direkt weiterverwendet.

In 200 ml abs. THF werden 6.73 g (≥1.3 eq, 19.455 mmol) 2-Phosphonopropionsäure-(trifluor)-triethylester und 11.87 g (M= 264.32 g·mol$^{-1}$; ≥3 eq, 44.91 mmol) 18-Krone-6 bei -78°C vorgelegt. Nun wird durch langsame Zugabe von 3.614 g (M= 199.49 g·mol$^{-1}$; 1.15 eq, 17.21 mmol, 95%iges KHMDS) KHMDS, gelöst in etwas abs. THF, deprotoniert und 15 min nachgerührt, wobei kurzzeitig das Kühlbad entfernt wird. Anschließend wird der Rohaldehyd, gelöst in ca. 45 ml abs. THF, langsam über einen Zeitraum von 70 min zugegeben und 30 min nachgerührt. Das Kühlbad wird entfernt und die Reaktion durch Zugabe von ges. $NH_4Cl$-Lsg. gequenscht.

Nach Phasentrennung wird mit ges. $NaHCO_3$-Lsg. gewaschen, die wässrigen Phasen noch zweimal mit Ether extrahiert und die vereinigten organischen Phasen über Magnesiumsulfat getrocknet. Nach Filtration der organischen Phasen über eine kurze Kieselgelfritte wird in Vakuum eingeengt. Chromatographische Reinigung über eine 15:1-10: 1 -Hex/EE-Gradientenkieselgelsäule erbrachte 5.453 g (89%) der Titelverbindung als einziges Doppelbindungsisomer.

**$R_f$-Wert** (Hex/EE= 3:1) ≈ 0.63 F III (sehr schwach blau);
**$R_f$-Wert** (Hex/EE= 5:1) ≈ 0.46 F II (blau);

**$^1$H-NMR** (400 MHz, $CDCl_3$): δin ppm =
0.01 (s, 3H, $-SiCH_3$); 0.04 (s, 3H, $-SiCH_3$); 0.88 (s, 9H, $-SiC(CH_3)_3$); 1.28 (t, *J*= 7.0 Hz, 3H, $-CO_2CH_2C\mathbf{H}_3$); 1.88 (s, 3H, 2-$CH_3$); 2.00 (s, 3H, 6-$CH_3$); 2.70 (s, 3H, TAr-$CH_3$); 2.75 (m, 2H, 4-H); 4.18 (q, *J*= 7.0 Hz, 2H, $-CO_2C\mathbf{H}_2CH_3$); 4.21 (t, *J*= 5.5 Hz, 1H, 5-H); 5.98 (td, $J_{3\text{-H, 4-H}}$= 7.3 Hz, $^4J_{3\text{-H}\cdot\ 2\text{-CH3}}$= 1.5 Hz, 1H, 3-H); 6.49 (s, 1H, 7-H); 6.91 (s, 1H, TAr-$CH_{arom}$).

**$^{13}$C-NMR** (100 MHz, $CDCl_3$): δ in ppm =
-5.1 ($SiCH_3$); -4.7 ($SiCH_3$); 14.1 (12-$CH_3$); 14.3 ($CO_2CH_2\mathbf{C}H_3$); 18.2 ($Si\mathbf{C}(CH_3)_3$); 19.2 (TAr-$\mathbf{C}H_3$); 20.7 (2-$CH_3$); 25.8 ($SiC(C\mathbf{H}_3)_3$); 36.6 (C-4); 60.1 ($CO_2\mathbf{C}H_2CH_3$); 77.9 (C-5); 115.2 (C-9); 118.8 (C-7); 128.3 (C-2); 139.0 (C-3); 141.9 (C-6); 153.2 (C-8); 164.4 (C-10); 168.0 ($\mathbf{C}O_2Et$).

**IR** (Si-Film): ν in $cm^{-1}$ =
2956vs; 2929vs; 2896m; 2856s; 1714vs; 1648w; 1506w; 1472m; 1462m; 1372m; 1252s; 1210s; 1185m; 1132s; 1096vs; 1032m; 951w; 837vs; 808m; 777s; 737w.

**MS** (FI, 7kV, 3mA, 40°C): m/e =
410([M]); 352; 336; 297; 282; 253; 224; 167; 132; 127; 58; 57.

| | | | | | |
|---|---|---|---|---|---|
| **$C_{21}H_{35}NO_3SSi$** | **EA** | ber. | C: 61.6% | H: 8.6% | N: 3.4% |
| (M= 409.65 g·$mol^{-1}$) | | gef. | C: 61.55 % | H: 8.53 % | N: 3.39 % |

Beispiel J:

**(5S, 2*Z*, 6*E*)-2,6-Dimethyl-5-[(1,1-dimethylethyl)-dimethylsilyloxy]-7-(2-methylthiazol-4-yl)hepta-2,6-dienol** (Verbindung der Formel 4)

In 250 ml abs. THF werden 5.43 g (13.255 mmol) der nach Beispiel I hergestellten Verbindung bei 0°C tropfenweise mit 40 ml einer 1 M-DIBAH-Lsg. (in Heptan) versetzt. Nach 2.5 h wird zum Reaktionsabbruch mit 3 ml MeOH bei 0°C gequenscht und nach Verdünnung mit Diethylether werden 200 ml halbkonzentrierte NaK-Tartrat-Lsg. zugegeben. Nach ca. 45 min. kräftigen Rührens bei RT werden die zwei klaren Phasen getrennt, die wässrige Phase noch zweimal mit Ether extrahiert und die vereinigten organischen Phasen über Magnesiumsulfat getrocknet, filtriert und eingeengt. Chromatographische Reinigung über eine 5:1-Hex/EE-Kieselgelsäule erbrachte 4.752 g (97.5%) der Titelverbindung, als zähes, farbloses Öl, welches im Tiefkühler kristallisiert.

**$R_f$-Wert** (Hex/EE= 3:1) ≈ 0.24 F III (intensiv violett);

**$^1$H-NMR** (400 MHz, $CDCl_3$): δ in ppm =
0.02 (s, 3H, $-SiCH_3$); 0.04 (s, 3H, $-SiCH_3$); 0.88 (s, 9H, $-SiC(CH_3)_3$); 1.79 (s, 3H, 2-$CH_3$); 2.00 (d, $^4J$= 1.0 Hz, 3H, 6-$CH_3$); 2.20 (t, *J*= 6.0 Hz, 1H, 1-OH); 2.22 (m, 1H, 4-H); 2.46 (dt, *J*= 14.1 Hz, *J*= 8.0 Hz, 1H, 4-H); 2.69 (s, 3H, TAr-$CH_3$); 4.00 (dd, *J*= 12.0 Hz, *J*= 6.5 Hz, 1H, 1-H); 4.12 (dd, *J*= 12.0 Hz, *J*= 5.0 Hz, 1H, 1-H); 4.13 (dd, *J*= 11.0 Hz, *J*= 5.0 Hz, 1H, 5-H); 5.30 (td, *J*= 8.0 Hz, *J*= 2.5 Hz, 1H, 3-H); 6.43 (s, 1H, 7-H); 6.91 (s, 1H, TAr-$CH_{arom}$).

**$^{13}$C-NMR** (100 MHz, $CDCl_3$): δ in ppm =
-4.9 ($Si(CH_3)_2$); -4.7 ($Si(CH_3)_2$); 14.2 (6-$CH_3$); 18.3 ($Si\mathbf{C}(CH_3)_3$); 19.2 (TAr-$CH_3$); 22.0 (2-$CH_3$); 25.8 ($Si\mathbf{C}(CH_3)_3$); 35.4 (C-4); 61.9 (C-1); 78.2 (C-5); 115.2 (C-9); 118.8 (C-7); 124.3 (C-3); 137.6 (C-2); 142.2 (C-6); 152.9 (C-8); 164.6 (C-10).

**IR** (Si-Film): ν in $cm^{-1}$ =
3333br; 2956s; 2927s; 2856s; 1657w; 1508w; 1472m; 1462m; 1441m; 1253s; 1185w; 1100vs; 1006s; 938m; 887m; 836s; 776s; 737m.

**MS** (FI, 7 kV, 3mA, 35°C): m/e =
368 ([M+1]); 367 ([M]); 365; 309; 285; 282; 252; 237; 235; 224; 167; 132; 115; 85; 75; 58; 57.

**Drehwert:**

$[\alpha]_D^{20}$= -6.8; ($c$= 2.20; $CHCl_3$)

**$C_{19}H_{33}NO_2SSi$:**
(M= 367.62 g·mol$^{-1}$)

**Beispiel K - L: Herstellung der Verbindung 34-I**

Beispiel K:

**(2S, 4R, 6*Z*, 9S, 10*E*)-1,9-Bis((1,1-dimethylethyl)dimethylsilyloxy]-11-(2-methylthiazol-4-yl)-4-phenylsulfonyl-2,6,10-trimethyl-undeca-6.10-dien**

In 100 mL abs. $CH_3CN$/Ether (3:2) werden 4.66 g (12.676 mmol) der nach Beispiel J hergestellten Verbindung nacheinander mit 4.324 g (16.479 mmol, 1.3 eq) $Ph_3P$ und 1.164 g (17.113 mmol, 1.35 eq) Imidazol vorgelegt. Zu dieser Lösung werden langsam 4.625 g (17.746 mmol, 1.4 eq) lod zugegeben und 60 min bei RT nachgerührt.
Zur Aufarbeitung werden 400 mL kalter Ether zugegeben (Niederschlagbildung), filtriert und das Filtrat dreimal mit ges. $Na_2S_2O_3$-Lsg. gewaschen. Die organische Phase wird anschließend über $MgSO_4$ getrocknet, über Kieselgel filtriert und eingeengt. Das Rohprodukt wird unter Lichtausschluß im Ölpumpenvakuum (ca. 0.1 mbar) getrocknet und so in der nachfolgenden Umsetzung verwendet.
In 350 mL abs. THF werden 5.645 g (12.676 mmol, 1.3 eq) der nach Beispiel C hergestellten Verbindung mit 6.7 g (25.35 mmol, 2 eq) 18-Krone-6 bei -78°C vorgelegt und mit einer Lösung von 3.59 g (17.113 mmol, 1.35 eq, 95%iges KHMDS) KHMDS, in etwas abs. THF, langsam versetzt. Nach 60 min wird das rohe Allyliodid, gelöst in 60 mL abs. THF, tropfenweise bei -78°C zugegeben. Nach 60 min wird das Kühlbad entfernt, mit 250 mL Ether verdünnt und durch Zugabe von 250 mL ges. $NH_4Cl$-Lsg. die Reaktion gequenscht. Die Phasen werden getrennt, die wässrige Phase noch einmal mit Ether extrahiert, die vereinigten org. Phasen noch zweimal mit ges. $Na_2S_2O_3$-Lsg. gewaschen, anschließend über $MgSO_4$ getrocknet, über 2 cm Kieselgel filtriert und eingeengt.
Zweimalige chromatographische Reinigung über eine 10:1-5:1-Hex/EE-Gradientenkieselgelsäule und eine 10:1-Hex/EE-Kieselgelsäule erbrachte 8.185 g der Titelverbindung, als Diastereoisomerengemisch, mit noch etwas Edukt (Beispiel C), welches sich über eine MC-Kieselgelsäule abtrennen läßt.

**$R_f$-Wert** (Hex/EE= 3:1) ≈ 0.54 F III (intensiv violett);

**IR** (Si-Film): ν in cm$^{-1}$ =
2957vs; 2885vs; 2855vs; 1506m; 1472s; 1462s; 1447s; 1388m; 1361m; 1305s; 1256s; 1184m; 1145vs; 1102vs; 1027m; 1006m; 939s; 911s; 837vs; 775vs; 737vs; 691 m; 668w.

**MS** (FI, 7 kV, 3mA, 150 °C): m/e =
694 ([M+2]); 693 ([M+1]); 692 ([M], 100); 636; 550; 431; 410 (<1); 282 (3); 267; 159 (2); 142; 132; 115; 114.

**$C_{36}H_{71}NO_4S_2Si_2$:**
(M= 692.17g·mol$^{-1}$)

Beispiel L:

**(2S, 6Z, 9S, 10*E*)-1,9-Bis[(1,1-dimethylethyl)dimethylsilyloxy]-11-(2-methylthiazol-4-yl)-2,6,10-trimethyl-undeca-6,10-dien** (Verbindung der Formel 34.I)

In 12 mL MeOH/THF (2:1) werden 580 mg (0.838 mmol) der nach Beispiel K hergestellten Verbindung bei -15°C mit 175 mg (1.232 mmol) $Na_2HPO_4$ vorgelegt. Nun werden 1.466 g (3.189 mmol) 5% Natriumamalgam zugegeben, nach 30 min das Kühlbad entfernt und die Temperatur der Reaktionslösung über einen Zeitraum von 90 min auf RT ansteigen gelassen. Zur Aufarbeitung wird filtriert, mit Ether und Wasser nachgewaschen. Die filtrierte Lösung wird mit ges. $NK_4Cl$-Lsg. gewaschen, über $MgSO_4$ getrocknet, über 1 cm Kieselgel filtriert und im Vakuum eingeengt.
Chromatographische Reinigung über eine 25:1-Hex/EE-Kieselgelsäule erbrachte 300 mg (64.9%) der Titelverbindung, als farbloses Öl.

**$R_f$-Wert** (Hex/EE= 5:1) ≈ 0.74

$^{1}$**H-NMR** (400 MHz, $CDCl_3$): δ in ppm =
0.008 (s, 3H, $-SiCH_3$); 0.02 (s, 6H, $-Si(CH_3)_2$); 0.04 (s, 3H, $-SiCH_3$); 0.85 (d, J= 6.5 Hz, 3H, 2-$CH_3$); 0.88 (s, 18H, $-SiC(CH_3)_3$'s); 1.29-1.39 (m, 3H); 1.65 (s, 3H, 6-$CH_3$); 1.97 (m, 2H); 1.99 (s, 3H, 10-$CH_3$); 2.24 (m, 2H, 8-H); 2.70 (s, 3H, TAr-$CH_3$); 3.33 (dd, *J*= 9.5 Hz, *J*= 6.5 Hz, 1H, 1-H); 3.43 (dd, *J*= 9.5 Hz, *J*= 5.5 Hz, 1H, 1-H); 4.07 (dd, $J_{9-H,8-H} \cong J_{9-H,8'-H}$= 6.5 Hz, 1H, 9-H); 5.12 (dd, $J_{7-H,8-H} \cong J_{7-H,8'-H}$= 6.5 Hz, 1H, 7-H); 6.44 (s, 1H, 11-H); 6.90 (s, 1H, TAr-$CH_{arom}$).

$^{13}$**C-NMR** (100 MHz, $CDCl_3$): δ in ppm =
-5.4 ($SiCH_3$); -4.7 ($SiCH_3$); 13.9 (10-$CH_3$); 16.7 (2-$CH_3$); 18.6 (2-Si**C**$(CH_3)_3$); 19.2(TAr-$CH_3$); 23.5 (6-$CH_3$); 25.8 (C-8); 25.9 (SiC(**C**$H_3)_3$); 26.0 (SiC(**C**$H_3)_3$); 32.3 (C-4); 33.2 (C-3); 35.3 (C-5); 35.8 (C-2); 68.4 (C-1); 79.1 (C-9); 114.9 (C-13); 118.7 (C-11); 121.4 (C-7); 136.9 (C-6); 142.6 (C-10); 153.3 (C-12); 164.3 (C-14).

**IR** (Si-Film): ν in $cm^{-1}$ =
2956s; 2928s; 2856s; 1472m; 1462m; 1388w; 1360w; 1255m; 1183w; 1097vs; 1006w; 940m; 836s; 775s.

**MS** (FI, 7 kV, 3mA, 110 °C): m/e =
553 ([M+1]); 552 ([M], 100); 495 ([M-tBu]); 463; 417; 365; 283 (8); 282 (33); 269 (2); 224; 167; 159; 115 (<1).

**Drehwert:**

$$[\alpha]_D^{20} = +8.4;\ (c = 4.35;\ CHCl_3)$$

| | | | | | |
|---|---|---|---|---|---|
| **$C_{30}H_{57}NO_2SSi_2$** | **EA** | ber. | C: 65.3 % | H: 10.4 % | N: 2.5 % |
| (M= 552.01 $g \cdot mol^{-1}$) | | gef. | C: 65.51 % | H: 10.23 % | N: 2.58 % |

**Beispiel M - T: Herstellung der Verbindung 2**

Beispiel M:

**N-Tosyl-D-Valin**

Einer Lsg. von D-Valin (11.7 g, 0.1 mol) in 200 mL 1N NaOH wurde bei RT eine Lösung von Tosylchlorid (20 g, 0.1 mol) in Diethylether (100 mL) zugetropft und das Zweiphasengemisch 4 h kräftig gerührt. Anschließend wurde die Diethyletherphase abgetrennt, die wässrige Phase noch zweimal mit Diethylether gewaschen und bei 0°C durch Zugabe konz. HCl angesäuert. Nach 30 min wurde abgesaugt, ausgiebig mit Eiswasser nachgewaschen und mehere Stunden bei 0.01 Torr getrocknet. Man erhielt 18.1 g, (67%), farblose Kristalle (Schmp.: 147°C).

Beispiel N:

**(3S)-5-[(1,1-Dimethylethyl)dimethylsilyloxy]-2,2-dimethyl-3-hydroxypentansäure-methylester**

In 50 mL abs. $CH_2Cl_2$ werden 1.355 g (5 mmol) der nach Beispiel M hergestellten Verbindung unter Argon bei RT langsam (30 min) mit 5 mL einer 1M $BH_3 \cdot$THF-Komplex-Lsg. (in THF) versetzt und 20 min nachgerührt. (Kat-Bildung)

Die Lösung wird auf -78°C abgekühlt, mit 940 mg (5 mmol) TBS geschützten 3-Hydroxypropanal und 960 mg (5.5 mmol, 1.1 eq) 1-Methoxy-2-methyl-1-(trimethylsilyloxy)-prop-1-en, jeweils in 5 mL abs. $CH_2Cl_2$ gelöst, nacheinander versetzt.

Nach 4 h wird mit 35 mL Phosphatpuffer (pH=6.9) gequenscht, die Phasen werden getrennt, die wässrige Phase noch dreimal mit $CH_2Cl_2$ (je 20 mL) ausgeschüttelt, die vereinigten org. Phasen anschließend über $MgSO_4$ getrocknet, filtriert und eingeengt. Der Rückstand wird zur Rückgewinnung von N-Tosyl-D-Valin mit kaltem Hexan aufgenommen, filtriert (ca. 96% Kat. rückgewonnen) und eingeengt.

Chromatographische Reinigung über eine 15:1-Hex/EE-Kieselgelsäule erbrachte
1.248 g (88%) der Titelverbindung, als farbloses Öl.

**$R_f$-Wert** (Hex/EE= 5:1) ≈ 0.39 F I (blau);

**$^1$H-NMR** (400 MHz, $CDCl_3$): δ in ppm = 0.06 (s, 6H, $-Si(CH_3)_2$); 0.88 (s, 9H, $-SiC(CH_3)_3$); 1.15 (s, 3H, 2-$CH_3$); 1.19 (s, 3H, 2-$CH_3$); 1.57 (ddd, *J*= 11.0 Hz, *J*= 5.5 Hz, *J*= 1.0 Hz, 2H, 4-H); 3.37 (d, *J*= 3.5 Hz, 1H, 3-OH); 3.68 (s, 3H, $-CO_2CH_3$); 3.80 (m, 1H, 5-Ha); 3.87 (dd, *J*= 10.0 Hz, *J*= 5.0 Hz, 1H, 5-Hb); 3.92 (m, 1H, 3-H).

**$^{13}$C-NMR** (100 MHz, $CDCl_3$): δ in ppm =
-5.5 ($Si(CH_3)_2$); 18.2 (Si**C**$(CH_3)_3$); 20.6 (2-$CH_3$); 21.3 (2-$CH_3$); 25.9 (SiC(**C**$H_3)_3$); 33.7 (C-4); 47.1 (C-2); 51.8 ($CO_2$**C**$H_3$); 62.6 (C-5); 76.0 (C-3); 177.8 (C-1).

**IR** (Si-Film): ν in $cm^{-1}$ =
3630br; 2955s; 2931s; 2884m; 2858s; 1733s; 1472m; 1435w; 1389w; 1257s; 1193m; 1138s; 1099vs; 1006w; 985w; 884w; 838vs.

**MS** (FI, 7 kV, 3mA, 20 °C): m/e =
291 ([M]); 261; 235 (4); 234 (15); 233 ([M-tBu], 100); 189; 131; 102.

**Drehwert:**

$$[\alpha]_D^{20} = +3.0; (c= 1.88; CHCl_3)$$

| | | | | |
|---|---|---|---|---|
| **$C_{14}H_{30}O_4Si$** (M= 290.47 g·$mol^{-1}$) | **EA** | ber. | C: 57.9 % | H: 10.4 % |
| | | gef. | C: 58.26 % | H: 10.31 % |

Beispiel O:

**(3S)-3,5-[(1,1-Dimethylethyl)dimethylsilyloxy]-2,2-dimethyl-pentansäuremethylester**

In 100 mL abs. $CH_2Cl_2$ werden 5.32 g (18.315 mmol) der nach Beispiel N hergestellten Verbindung bei 0°C mit 6.38 mL 2,6-Lutidin (3 eq) vorgelegt. Nun werden 5.05 mL (1.2 eq) TBSTriflat langsam zugetropft. Nach 3 h bei 0°C wird das Kühlbad entfernt und die Reaktion durch Zugabe von 25 mL ges. $NH_4Cl$-Lsg. gequenscht.

Die Phasen werden getrennt, die wässrige Phase noch zweimal mit $CH_2Cl_2$ extrahiert, die vereinigten org. Phasen anschließend über $MgSO_4$ getrocknet, über 5 cm Kieselgel filtriert und eingeengt.

Nach Trocknung im Öipumpenvakuum (ca. 0.1 mbar) erhielt man bereits NMR-sauberes Produkt, welches für die Analytik noch über eine 3% später 5% Ether in Hexan-Kieselgelsäule chromatographisch aufgereinigt wurde. Es wurden 7.123 g (96.1%) der Titelverbindung, als farbloses Öl erhalten.

**$R_f$-Wert** (Hex/EE= 10:1) ≈ 0.33 FI (blau);

**$^1$H-NMR** (400 MHz, $CDCl_3$): δ in ppm =
0.02 (s, 3H, $-SiCH_3$); 0.03 (s, 3H, $-SiCH_3$); 0.033 (s, 3H, $-SiCH_3$); 0.07 (s, 3H, $-SiCH_3$); 0.86 (s, 9H, $-SiC(CH_3)_3$); 0.89 (s, 9H, $-SiC(CH_3)_3$); 1.08 (s, 3H, 2-$CH_3$); 1.15 (s, 3H, 2-$CH_3$); 1.59 (m, 2H, 4-H); 3.62 (m, 2H, 5-H); 3.64 (s, 3H, $-CO_2CH_3$); 4.05 (dd, J= 7.5 Hz, J= 3.0 Hz, 1H, 3-H).

**$^{13}$C-NMR** (100 MHz, $CDCl_3$): δ in ppm =
-5.3 ($Si(CH_3)_2$); -4.3 ($SiCH_3$); -3.9 ($SiCH_3$); 18.3 (Si**C**$(CH_3)_3$); 20.4 (2-$CH_3$); 21.7 (2-$CH_3$); 25.9 (SiC(**C**$H_3)_3$); 26.0 (SiC(**C**$H_3)_3$); 36.9 (C-4); 48.3 (C-2); 51.6 ($CO_2$**C**$H_3$); 60.3 (C-5); 73.4 (C-3); 177.6 (C-1).

**IR** (Si-Film): ν in $cm^{-1}$ =
2955s; 2930s; 2886m; 2858s; 1736s; 1472m; 1464m; 1434w; 1388w; 1257s; 1135m; 1039w; 1006w; 939w; 837vs.

**MS** (FI, 7 kV, 3mA, 20 °C): m/e =
405 ([M], 1); 349 (9); 348 (23); 347 ([M-tBu], 100); 303; 267; 233 (1); 220; 199; 159; 132; 115 (2).

**Drehwert:**

$[\alpha]_D^{20}$= -6.0; ($c$= 3.91; $CHCl_3$)

| | | | | |
|---|---|---|---|---|
| **$C_{20}H_{44}O_4Si_2$** (M= 404.73 g·mol$^{-1}$) | **EA** | ber. | C: 59.4 % | H: 11.0 % |
| | | gef. | C: 59.50% | H: 10.74 % |

Beispiel P:

**(5S)-4,6-Bis-[(1,1-dimethylethyl)dimethylsilyloxy]-3,3-dimethyl-hexan-2-on**

Einer Lösung von (1.013 g, 2.5 mmol) der nach Beispiel O hergestellten Verbindung in abs. Pentan (12 mL) werden bei 0°C 5.5 mL einer 1M Trimethylsilylmethyllithium-Lsg. in Pentan (2.2 eq) in einer Portion zugefügt. Nach 4 h Rühren werden der farblosen Suspension 2.5 mL MeOH zugefügt und die resultierende blaßgelbe Emulsion 1 h bei RT kräftig gerührt.

Zur Aufarbeitung wird mit Diethylether/Wasser verdünnt, die Phasen anschließend getrennt und die wässrige Phase noch dreimal mit Diethylether extrahiert. Die vereinigten org. Phasen werden über $Na_2SO_4$ getrocknet, filtriert und eingeengt. Flashchromatographie an Kieselgel (75 g, Hex/$Et_2O$, 98:2) erbrachte 947 mg (97%) der Titelverbindung, als farbloses Öl.

**$R_f$-Wert** (Hex/EE= 96:4) ≈ 0.4 F III (gelbbraun);

**$^1$H-NMR** (400 MHz, $CDCl_3$): δ in ppm =
- 0.01 (s, 3H, -$SiCH_3$); 0.00 (s, 3H, -$SiCH_3$); 0.02 (s, 3H, -$SiCH_3$); 0.06 (s, 3H, -$SiCH_3$); 0.85 (s, 18H, -SiC$(CH_3)_3$); 1.01 (s, 3H, 3-$CH_3$); 1.06 (s, 3H, 3-$CH_3$); 1.39-1.59 (m, 2H, 5-H); 2.10 (s, 3H, 1-H); 3.54-3.64 (m, 2H, 6-H); 4.00 (dd, $J_{4\text{-H},5a\text{-H}}$= 3.0 HZ, $J_{4\text{-H}, 5b\text{-H}}$= 8.0 Hz, 1H, 3-H).

**$^{13}$C-NMR** (100 MHz, $CDCl_3$): δ in ppm =
-4.9 ($SiCH_3$); -3.7 ($SiCH_3$); -3.6 ($SiCH_3$); 18.6 (Si**C**$(CH_3)_3$); 18.7 (Si**C**$(CH_3)_3$); 20.4 (3-$CH_3$); 22.3 (3-$CH_3$); 26.3 (SiC(**C**$H_3)_3$); 26.4 (SiC(**C**$H_3)_3$); 27.1 (C-1); 37.6 (C-5); 53.8 (C-3); 60.4 (C-1); 73.7 (C-6); 213.7 (C-2).

**MS** (FI, 7 kV, 3mA, °C): m/e =
389 ([M+H], 1); 331 ([M-tBu], 100).

Beispiel Q:

**(3S)-1,3-[(1,1-Dimethylethyl)dimethylsilyloxy]-4,4-dimethyl-pentan-5-ol**

In 100 mL abs. Toluol werden 4.047 g (10 mmol) der nach Beispiel P hergestellten Verbindung bei -20°C tropfenweise mit 30 mL (3 eq) einer 1M-DIBAH-Lsg. (in Heptan) versetzt und 2 h nachgerührt.

Nun wird bei 0°C durch Zugabe von 5 mL MeOH gequenscht, mit 150 mL Ether verdünnt und langsam mit 250 mL ges. NaK-Tartrat-Lsg. versetzt und kräftig nachgerührt, bis sich zwei klare Phasen gebildet haben. Die Phasen werden getrennt, die wässrige Phase noch dreimal mit Ether extrahiert, die vereinigten org. Phasen anschließend über $MgSO_4$ getrocknet, über 1 cm Kieselgel filtriert und eingeengt.

Chromatographische Reinigung über eine 10:1-Hex/EE-Kieselgelsäule erbrachte 3.527 g (93.6%) der Titelverbindung, als farbloses Öl.

**$R_f$-Wert** (Hex/EE= 10:1) ≈ 0.32
**$R_f$-Wert** (Hex/EE= 5:1) ≈ 0.53 F I (intensiv blau);

**$^1$H-NMR** (400 MHz, $CDCl_3$): δ in ppm =
0.04 (s, 6H, -$Si(CH_3)_2$); 0.078 (s, 3H, -$SiCH_3$); 0.084 (s, 3H, -$SiCH_3$); 0.79 (s, 3H, 2-$CH_3$); 0.878 (s, 9H, -SiC$(CH_3)_3$); 0.883 (s, 9H, -SiC$(CH_3)_3$); 0.98 (s, 3H, 2-$CH_3$); 1.62 (m, 1H, 4-H); 1.90 (m, 1H, 4-H); 2.87 (dd, $J$= 7.0 Hz, $J$= 4.0 Hz, 1H, 1-OH); 3.28 (dd, $J$= 10.5 Hz, $J$= 7.0 Hz, 1H, 3-H); 3.66 (m, 4H, 1-H u. 5-H).

**$^{13}$C-NMR** (100 MHz, $CDCl_3$): δ in ppm =
-5.33 ($SiCH_3$); -5.30 ($SiCH_3$); -4.3 ($SiCH_3$); -4.0 ($SiCH_3$); 18.2 (Si**C**$(CH_3)_3$); 18.3 (Si**C**$(CH_3)_3$); 22.0 (2-$CH_3$);

22.8 (2-$CH_3$); 25.9 (SiC(**C**$H_3$)$_3$); 26.0 (SiC(**C**$H_3$)$_3$); 36.4 (C-2); 39.3 (C-4); 60.7 (C-5); 70.2 (C-1); 76.7 (C-3).

**IR** (Si-Film): ν in $cm^{-1}$ =
3450br; 2958s; 2931s; 2885m; 2858s; 1473m; 1464m; 1407w; 1389m; 1361m; 1256s; 1103vs; 1043s; 1006m; 939m; 836vs.

**MS** (EI, eV, °C): m/e =
377 ([M], 3); 350; 321 (10); 320 (25); 319 ([M-tBu],100); 261; 187; 173 (1); 131; 115 (4); 114.

| | | | | |
|---|---|---|---|---|
| **$C_{19}H_{44}O_3Si_2$** | **EA** | ber. | C: 60.6 % | H: 11.8 % |
| (M= 376.72 g·$mol^{-1}$) | | gef. | C: 60.82 % | H: 11.70 % |

Beispiel R:

**(3S)-3,5-[(1,1-Dimethylethyl)dimethylsilyloxy]-2,2-dimethyl-pentanal**

In 100 mL abs. $CH_2Cl_2$ und 2 mL abs. Pyridin werden 820 mg (2.177 mmol) der nach Beispiel Q hergestellten Verbindung bei 0°C mit 1.21 g (1.3 eq, 2.83 mmol) Dess-Martin-Periodinan portionsweise versetzt und 2 h nachgerührt. Nun wird mit 100 mL Ether verdünnt (Niederschlagbildung), filtriert und das Filtrat zweimal mit je 50 mL ges. $NaHCO_3$/ ges. $Na_2S_2O_3$-Lsg. (1:1) gewaschen. Die Phasen werden getrennt, die wässrige Phase wird noch zweimal mit $CH_2Cl_2$ extrahiert, die vereinigten org. Phasen werden über $MgSO_4$ getrocknet, über 1 cm Kieselgel filtriert und eingeengt. Der Rohaldehyd wird nach Trocknung an der Ölpumpe (ca. 0.1 mbar) direkt weiterverwendet.

**$R_f$-Wert** (Hex/EE= 10:1) ≈ 0.67 F I (blau);

Beispiel S:

**(3S,5RS)-1,3-Bis[(1,1-dimethylethyl)dimethylsilyloxy]-4,4-dimethyl-heptan-5-ol**

Die nach Beispiel R hergestellte Verbindung wird in 10 mL abs. Diethylether bei 0°C tropfenweise mit 762 µL (≥1.05 eq, 2.286 mmol) einer 3M EtMgBr-Lsg. (in Ether) versetzt. Nach 2 h wird mit Ether verdünnt und durch Zugabe von 50 mL ges. $NH_4Cl$-Lsg. gequenscht. Die Phasen werden getrennt, die wässrige Phase noch dreimal mit Ether extrahiert, die vereinigten org. Phasen anschließend über $MgSO_4$ getrocknet, filtriert und eingeengt.

Chromatographische Reinigung über eine 25:1-Hex/EE-Kieselgelsäule erbrachte 530 mg (60.2%) der Titelverbindung und 316 mg (38.5%), als Nebenprodukt (Beispiel Q), resultierend aus der Reduktion durch das EtMgBr.

**$R_f$-Wert** (Hex/EE= 10:1) unpol. Produkt ≈ 0.53
**$R_f$-Wert** (Hex/EE= 10:1) pol. Produkt ≈ 0.44
**$R_f$-Wert** (Hex/Ether= 9:1) unpol. Produkt ≈ 0.32
**$R_f$-Wert** (Hex/Ether= 9:1) pol. Produkt ≈ 0.25 F I (blau);

**$^1$H-NMR** (400 MHz, $CDCl_3$) unpol. Produkt: δ in ppm =
0.03 (s, 3H, -Si$CH_3$); 0.04 (s, 3H, -Si$CH_3$); 0.11 (s, 3H, -Si$CH_3$); 0.112 (s, 3H, -Si$CH_3$); 0.73 (s, 3H, 4-$CH_3$); 0.88 (s, 9H, -SiC($CH_3$)$_3$); 0.90 (s, 9H,-SiC($CH_3$)$_3$); 0.97 (s, 3H, 4-$CH_3$); 1.00 (t, *J*= 7.0 Hz, 3H, 7-H); 1.35 (m, 2H, 6-H); 1.70 (mc, 1H, 2-H); 1.92 (mc, 1H, 2-H); 3.63 (m, 2H, 1-H); 3.71 (m, 1H, 3-H); 4.29 (s, 1H, 5-OH).

**$^{13}$C-NMR** (100 MHz, $CDCl_3$) unpol. Produkt: δ in ppm =
-5.31 (Si$CH_3$); -5.27 (Si$CH_3$); -4.3 (Si$CH_3$); -3.9 (Si$CH_3$); 11.3 (C-7); 18.20 (Si**C**($CH_3$)$_3$); 18.24 (Si**C**($CH_3$)$_3$); 20.5 (4-$CH_3$); 23.5 (4-$CH_3$); 24.5 (C-6); 25.9 (SiC(**C**$H_3$)$_3$); 26.1 (SiC(**C**$H_3$)$_3$); 36.0 (C-2); 40.7 (C-4); 60.1 (C-1); 77.4 (C-3); 80.5 (C-5).

**IR** (Si-Film): ν in $cm^{-1}$ =
3500br; 2957vs; 2930s; 2884s; 2858s; 1472s; 1464m; 1409w; 1389m; 1362m; 1326w; 1256s; 1216w; 1101vs; 1048m; 1022m; 1004s; 976w; 955m; 940m; 925w; 836vs; 810m; 776s.

**MS** (FI, 7 kV, 3mA, 20°C): m/e =

405 ([M], 4); 349 (7); 348 (27); 347 ([M-tBu], 100); 159 (3); 115 (7).

| | | | | |
|---|---|---|---|---|
| **$C_{21}H_{48}O_3Si_2$** | **EA** | ber. | C: 62.3 % | H: 12.0 % |
| (M= 404.76 g·mol$^{-1}$) | | gef. | C: 62.57 % | H: 11.98 % |

**$^1$H-NMR** (400 MHz, $CDCl_3$) pol. Produkt: δ in ppm =
0.05 (s, 3H, -$SiCH_3$); 0.06 (s, 3H, -$SiCH_3$); 0.07 (s, 6H, -$Si(CH_3)_2$); 0.74 (s, 3H, 4-$CH_3$); 0.85 (s, 3H, 4-$CH_3$); 0.89 (s, 9H, -$SiC(CH_3)_3$); 0.90 (s, 9H,-$SiC(CH_3)_3$); 0.98 (t, *J*= 7.0 Hz, 3H, 7-H); 1.29 (mc, 1H, 6-H); 1.49 (m, 2H, 2-H); 2.00 (mc, 1H, 6-H); 2.74 (dd, *J*=4.5 Hz, *J*= 1.0 Hz, 1H, 5-OH); 3.33 (ddd, *J*= 10.0 Hz, *J*= 4.5 Hz, *J*= 1.5 Hz, 1H, 3-H); 3.68 (td, *J*= 9.5 Hz, *J*= 4.5 Hz, 1H, 5-H); 3.75 (m, 2H, 1-H).

**$^{13}$C-NMR** (100 MHz, $CDCl_3$) pol. Produkt: δ in ppm =
-5.4 (2·$SiCH_3$); -4.4 ($SiCH_3$); -3.6 ($SiCH_3$); 11.7 (C-7); 18.3 (Si**C**($CH_3)_3$); 18.4 (Si**C**($CH_3)_3$); 18.5 (4-$CH_3$); 18.7 (4-$CH_3$); 24.2 (C-6); 26.0 (SiC(**C**$H_3)_3$); 26.1 (SiC(**C**$H_3)_3$); 36.5 (C-2); 42.8 (C-4); 61.9 (C-1); 75.5 (C-3); 78.0 (C-5).

**IR** (Si-Film): ν in cm$^{-1}$ =
3484br; 2957vs; 2931s; 2884m; 2858s; 1472m; 1464m; 1389w; 1361w; 1256s; 1094vs; 1034w; 1005w; 938m; 836vs; 775s.

**MS** (FI, 7 kV, 3mA, 20°C): m/e =
405 ([M], 7); 365 (2); 364 (8); 349 (7); 348 (26); 347 ([M-tBu], 100); 307 (6); 306 (17); 305 (78); 267 (3); 115 (3).

**$C_{21}H_{48}O_3Si_2$:**
(M= 404.76 g·mol$^{-1}$)

Beispiel T:

**(5S)-5,7-Bis[(1,1-dimethylethyl)dimethylsilyloxy]-4,4-dimethyl-heptan-3-on** (Verbindung der Formel 2)

1.) Variante:

In 60 mL abs. $CH_2Cl_2$ und 1 mL abs. Pyridin werden 495 mg (1.223 mmol) der nach Beispiel S hergestellten Verbindung bei 0°C mit 778 mg (1.5 eq, 1.834 mmol) Dess-Martin-Periodinan portionsweise versetzt und 2 h nachgerührt. Nun wird mit 100 mL Ether verdünnt (Niederschlagsbildung), filtriert und das Filtrat zweimal mit je 60 mL ges. $NaHCO_3$/ges. $Na_2S_2O_3$-Lsg. (1:1) gewaschen. Die Phasen werden getrennt, die wässrige Phase wird noch zweimal mit $CH_2Cl_2$ extrahiert, die vereinigten org. Phasen werden über $MgSO_4$ getrocknet, über 1 cm Kieselgel filtriert und eingeengt. Nach Trocknung an der Ölpumpe (ca. 0.1 mbar) wurden 487 mg (99%) der Titelverbindung, als (NMR-sauber) farbloses Öl erhalten.

2.) Variante:

Einer Lösung von DIPA (110 µL, 0.78 mmol, 1.38 eq) in abs. THF (4 mL) werden bei-78°C *n*-BuLi (0.3 mL, 2.5M-Lsg. in Hexan, 0.75 mmol, 1.33 eq) zugetropft und langsam (40 min) auf RT erwärmen gelassen.

Der wieder auf -78°C abgekühlten LDA-Lsg. werden eine Lösung von (220 mg, 0.566 mmol) der nach Beispiel P hergestellten Verbindung in abs. THF zügig zugetropft. Nach 30 min Rühren wird der Enolat-Lsg. Methyliodid (0.5 mL, 9 mmol, 16 eq) zugefügt, das Kühlbad entfernt und 1.5 h nachgerührt. Zur Aufarbeitung wird bei 0°C mit 10 mL ges. $NH_4Cl$-Lsg. gequenscht, die Phasen getrennt, die wässrige Phase noch dreimal mit je 10 mL $CH_2Cl_2$ ausgeschüttelt. Die vereinigten org. Phasen werden über $Na_2SO_4$ getrocknet, filtriert und eingeengt. Flashchromatographie an Kieselgel (10 g, Hex/$Et_2O$, 97:3) erbrachte 195 mg (85%) der Titelverbindung, als farbloses Öl.

**$R_f$-Wert** (MC/Hex= 1:1) ≈ 0.44 F I (blau);
F III (intensiv violettblau);

**$^1$H-NMR** (400 MHz, $CDCl_3$): δ in ppm =
0.02 (s, 3H, -$SiCH_3$); 0.03 (s, 3H, -$SiCH_3$); 0.04 (s, 3H, -$SiCH_3$); 0.08 (s, 3H, -$SiCH_3$); 0.87 (s, 9H, -$SiC(CH_3)_3$); 0.88 (s, 9H, -$SiC(CH_3)_3$); 0.99 (t, *J*= 7.3 Hz, 3H, 7-$CH_3$); 1.04 (s, 3H, 4-$CH_3$); 1.10 (s, 3H, 4-$CH_3$); 1.50 (m, 2H, 2-H); 2.51 (je dq, 2H, 6-H); 3.62 (m, 2H, 1-H); 4.06 (dd, *J*= 7.5 Hz, *J*= 3.0 Hz, 1H, 3-H).

**$^{13}$C-NMR** (100 MHz, $CDCl_3$): δ in ppm =
-5.3 (2-$SiCH_3$); -4.1 (2.$SiCH_3$); 7.7 (C-7); 18.3 (2·Si**C**$(CH_3)_3$); 20.0 (4-$CH_3$); 22.2 (4-$CH_3$); 25.9 (SiC(**C**$H_3)_3$); 26.1 (SiC(**C**$H_3)_3$); 31.6 (C-6); 37.3 (C-2); 53.0 (C-4); 60.1 (C-1); 73.4 (C-3); 215.6 (C=O).

**IR** (Si-Film): ν in $cm^{-1}$ =
2956s; 2928s; 2895m; 2856s; 1472m; 1462m, 1388w; 1360w; 1255m; 1183w; 1097vs; 1006w; 940m; 836vs; 775s.

**MS** (FI, 7 kV, 3mA, 20°C): m/e =
403 ([M]); 347; 346 ([M-tBu]); 345 (100); 303; 57.

| | | | | |
|---|---|---|---|---|
| **$C_{21}H_{46}O_3Si_2$** (M= 402.76 g·mol$^{-1}$) | **EA** | ber. | C: 62.6 % | H: 11.5 % |
| | | gef. | C: 62.67 % | H: 11.29 % |

**Beispiel U - V: Herstellung der Verbindung 234**

Beispiel U:

**(2S, 6*Z*, 9S,10*E*)-9-[(1,1-dimethylethyl)dimethylsilyloxy]-11-(2-methylthiazol-4-yl)-2,6,10-trimethyl-undeca-6,10-dien-1-ol**

In 80 mL MeOH/$CH_2Cl_2$ (1:1) werden 1.385 g (2.509 mmol) der nach Beispiel L hergestellten Verbindung bei 0°C portionsweise mit 583 mg (1 eq, 2.509 mmol) CSA versetzt und 5 h nachgerührt, wobei die Temperatur langsam auf 10°C ansteigen darf.
Die Reaktion wird durch Zugabe von 150 mL ges. $NaHCO_3$-Lsg. gequenscht und mit 150 mL Ether verdünnt. Die Phasen werden getrennt, die wässrige Phase noch zweimal mit je 100 mL Ether extrahiert, die vereinigten organischen Phasen anschließend über $MgSO_4$ getrocknet, über 2 cm Kieselgel filtriert und imVakuum eingeengt.
Chromatographische Reinigung über eine 5:1-Hex/EE-Kieselgelsäule erbrachte 1.094 g (99.6%) der Titelverbindung, als farbloses, zähes Öl.

**$R_f$-Wert** (Hex/EE= 5:1) ≈ 0.11

**$^1$H-NMR** (400 MHz, $CDCl_3$): δ in ppm =
-0.01 (s, 3H, -$SiCH_3$); 0.03 (s, 3H, -$SiCH_3$); 0.88 (s, 9H, -$SiC(CH_3)_3$); 0.90 (d, ***J*=** 6.5 Hz, 3H, 2-$CH_3$); 1.32-1.42 (m, 3H); 1.60 (m, 1H, 2-H); 1.66 (d, $^4J$= 1.0 Hz, 3H, 6-$CH_3$); 1.96 (m, 1-2H); 1.98 (d, $^4J$= 1.0 Hz, 3H, 10-$CH_3$); 2.24 (m, 2H, 8-H); 2.69 (s, 3H, TAr-$CH_3$); 3.39 (m, 1H, 1-H); 3.43 (m, 1H, 1-H); 4.08 (dd, $J_{9-H,8-H}$≅$J_{9-H,8'-H}$= 6.5 Hz, 1H, 9-H); 5.14 (dd, $J_{7-H.8-H}$≅$J_{7-H,8'-H}$ = 7.0 Hz, 1H, 7-H); 6.44 (s, 1H, 11-H); 6.91 (s, 1H, TAr-$CH_{arom}$).

**$^{13}$C-NMR** (100 MHz, $CDCl_3$): δ in ppm =
-4.9 ($SiCH_3$); -4.7 ($SiCH_3$); 13.9 (10-$CH_3$); 16.2 (2-$CH_3$); 18.2 (Si**C**$(CH_3)_3$); 19.1 (TAr-$CH_3$); 23.4 (6-$CH_3$); 25.3 (C-5); 25.8 (SiC(**C**$H_3)_3$); 32.1 (C-4); 33.1 (C-3); 35.5 (C-8); 35.7 (C-2); 68.1 (C-1); 79.2 (C-9); 114.8 (C-13); 118.7 (C-11); 121.7 (C-7); 136.8 (C-6); 142.7 (C-10); 153.2 (C-12); 164.4 (C-14).

**IR** (Si-Film): ν in $cm^{-1}$ =
3385br; 2956s; 2927s; 2856s; 1508w; 1461m; 1449m; 1406w; 1388w; 1376w; 1360w; 1253m; 1184m; 1102vs; 1005m; 940s; 888s; 836vs; 776s; 737m.

**MS** (FI, 7 kV, 3mA, 150°C): m/e =
439 ([M+H]); 438 ([M]); 396; 380; 284; 283; 282 (100); 183; 143; 115; 57.

**Drehwert:**

$$[\alpha]_D^{20} = +9.5;\ (c= 1.37;\ CHCl_3)$$

**$C_{24}H_{43}NO_2SSi$:**

(M= 437.75 g·mol$^{-1}$)

Beispiel V:

**(3S, 6R, 7S, 8S, 12*Z*,15S,16*E*)-4,4,6,8,12,16-hexamethyl-7-hydroxy-16-(2-methylthiazol-4-yl)-1,3,15-tris[(1,1-dimethylethyl)dimethylsilyloxy]-heptadec-12,16-dien-5-on** (Verbindung der Formel 234, wobei R = Methyl)

In 80 mL abs. $CH_2Cl_2$ mit 2 mL abs. Pyridin werden 983 mg (2.246 mmol) der nach Beispiel U hergestellten Verbindung bei 0°C mit 1.24 g (1.3 eq, 2.92 mmol) Dess-Martin-Periodinan portionsweise versetzt und 4 h nachgerührt. Zur Aufarbeitung wird mit 200 mL Ether verdünnt (Niederschlagsbildung), über eine 1 cm Kieselgelfritte filtriert und anschließend das Filtrat dreimal mit je 100 mL ges. $NaHCO_3$/ges. $Na_2S_2O_3$-Lsg. (1:1) gewaschen. Die organische Phase wird über $MgSO_4$ getrocknet, über 5 cm Kieselgel filtriert und eingeengt. Der Rohaldehyd wird anschließend im Ölpumpenvakuum (ca. 0.1 mbar) getrocknet und so in der nachfolgenden Reaktion direkt weiterverwendet.

In 10 mL abs. THF werden 441 µL (1.4 eq, 3.14 mmol) DIPA bei -20°C mit 1.258 mL (1.4 eq, 3.14 mmol) einer 2.5M nBuLi-Lsg. (in Hexan) langsam versetzt, 20 min nachgerührt. Die fertige LDA-Lsg. wird auf -78°C abgekühlt, langsam mit 1.266 g (1.4 eq, 3.14 mmol) der nach Beispiel T hergestellten Verbindung, verdünnt in 10 mL abs. THF, versetzt, 15 min nachgerührt und über einen Zeitraum von 45 min auf -35°C erwärmen gelassen. Anschließend wird auf -95°C eingekühlt, der Rohaldehyd, gelöst in etwas abs. THF, tropfenweise zugegeben und 90 min nachgerührt, wobei die Temperatur langsam auf -80°C ansteigen darf. Zur Aufarbeitung wird das Kühlbad entfernt, mit 50 mL ges. $NH_4Cl$-Lsg. gequenscht und mit Ether verdünnt. Die Phasen werden getrennt, die wässrige Phase wird noch zweimal mit Ether extrahiert, die vereinigten org. Phasen werden über $MgSO_4$ getrocknet, filtriert und eingeengt.

Chromatographische Vorreinigung über 25:1-Hex/EE-Kieselgelsäulen erbrachte 1.305 g (69%, über zwei Stufen) der Titelverbindung, als Mischfraktion der beiden Diastereomere. Die Trennung der diastereomeren Aldolprodukte erfolgte über präparative HPLC in 9:1-Hex/EE.

**$R_f$-Wert** (MC) vom Aldehyd ≈ 0.26
**$R_f$-Wert** (MC) Titelverbindung ≈ 0.14

**$^1$H-NMR** (400 MHz, $CDCl_3$) vom Aldehyd: δ in ppm =
-0.01 (s, 3H, $-SiCH_3$); 0.03 (s, 3H, $-SiCH_3$); 0.88 (s, 9H, $-SiC(CH_3)_3$); 1.07 (d, *J*= 6.5 Hz, 3H, 2-$CH_3$); 1.30-1.43 (m, 3H); 1.65 (d, $^4J$= 1.5 Hz, 3H, 6-$CH_3$); 1.64-1.72 (m, 1H); 1.99 (d, $^4J$= 1.0 Hz, 3H, 10-$CH_3$); 2.00-2.06 (m, 2H); 2.18-2.33 (m, 3H); 2.69 (s, 3H, TAr-$CH_3$); 4.07 (t, $J_{9\text{-H},8\text{-H}} \cong J_{9\text{-H},8\text{-H}}$= 6.5 Hz, 1H, 9-H); 5.15 (, $J_{7\text{-H},8H} \cong J_{7\text{-H},8'\text{-H}}$= 6.5 Hz, 1H, 7-H); 6.44 (s, 1H, 11-H); 6.91 (s, 1H, TAr-$CH_{arom}$); 9.59 (d, J= 1.5 Hz, 1H, 1-H).

**$^{13}$C-NMR** (100 MHz, $CDCl_3$) vom Aldehyd: δ in ppm =
-5.0 ($SiCH_3$); -4.7 ($SiCH_3$); 13.3 (2-$CH_3$); 14.0 (10-$CH_3$); 18.2 (Si**C**$(CH_3)_3$); 19.2 (TAr-$CH_3$); 23.4 (6-$CH_3$); 25.2 (C-5); 25.8 (SiC(**C**$H_3)_3$); 30.4 (C-4); 31.8 (C-3); 35.4 (C-8); 46.3 (C-2); 78.9 (C-9); 115.0 (C-13); 118.7 (C-11); 122.1 (C-7); 136.0 (C-6); 142.4 (C-10); 153.2 (C-12); 164.3 (C-14); 205.1 (C-1).

**$^1$H-NMR** (400 MHz, $CDCl_3$) Titelverbindung: δ in ppm =
-0.01 (s, 3H, $-SiCH_3$); 0.02 (s, 6H, $-Si(CH_3)_2$); 0.03 (s, 3H, $-SiCH_3$); 0.07 (s, 3H, $-SiCH_3$); 0.09 (s, 3H, $-SiCH_3$); 0.81 (d, J= 7.0 Hz, 3H, 8-$CH_3$); 0.87 (s, 18H, $-SiC(CH_3)_3$); 0.89 (s, 9H, $-SiC(CH_3)_3$); 1.01 (d, *J*= 7.0 Hz, 3H, 6-$CH_3$); 1.08 (s, 3H, 4-$CH_3$); 1.20 (s, 3H, 4-$CH_3$); 1.24-1.35 (m, 2H); 1.43-1.53 (m, 3H); 1.57-1.67 (m, 1H); 1.65 (s, 3H, 12-$CH_3$); 1.69-1.77 (m, 1H); 1.90-2.02 (m, 2H, 11-H); 1.98 (s, 3H, 16-$CH_3$); 2.23 (m, 2H, 14-H); 2.70 (s, 3H, TAr-$CH_3$); 3.29 (m, 1H, 6-H); 3.59 (m, 1H); 3.66 (m, 1H, 7-H); 3.89 (dd, *J*= 7.5 Hz, *J*= 3.0 Hz, 1H, 15-H); 4.08 (t, *J*= 6.5 Hz, 1H, 3-H); 5.12 (t, *J*= 7.6 Hz, 1H, 13-H); 6.44 (s, 1H, 17-H); 6.90 (s, 1H, TAr-$CH_{arom}$).

**$^{13}$C-NMR** (100 MHz, $CDCl_3$) Titelverbindung: δ in ppm =
-5.3 ($SiCH_3$); -4.9 ($SiCH_3$); -4.7 ($SiCH_3$); -4.1 ($SiCH_3$); -3.8 ($SiCH_3$); 9.6 (6-$CH_3$); 13.9 (16-$CH_3$); 15.4 (8-$CH_3$); 18.3 (Si**C**$(CH_3)_3$); 19.2 (TAr-$CH_3$); 20.5 (4-$CH_3$); 22.9 (4-$CH_3$); 23.5 (12-$CH_3$); 25.2 (C-11); 25.9 (SiC(**C**$H_3)_3$); 26.1 (SiC(**C**$H_3)_3$); 32.4 (C-10); 33.0 (C-9); 35.3 (C-14); 35.5 (C-8); 37.9 (C-2); 41.4 (C-6); 54.0 (C-4); 60.5 (C-1); 74.2 + 74.9 (C-3 u. C-7); 79.1 (C-15); 114.9 (C-19); 118.7 (C-17); 121.5 (C-13); 136.9 (C-12); 142.6 (C-16); 153.3 (C-18); 164.3 (C-20); 222.2 (C-5).

**IR** (Si-Film): ν in cm$^{-1}$ =
2956s; 2932s; 2885s; 2858s; 1472s; 1463s; 1444m; 1389m; 1366m; 1257vs; 1198m; 1132m; 1075m; 1006m; 940w; 874m; 838s; 814m; 777vs.

**$^1$H-NMR** (400 MHz, $CDCl_3$) pol. Produkt: $\delta$ in ppm =
-0.01 (s, 3H, $-SiCH_3$); 0.02 (s, 3H, $-SiCH_3$); 0.03 (s, 3H, $-SiCH_3$); 0.04 (s, 3H, $-SiCH_3$); 0.05 (s, 3H, $-SiCH_3$); 0.10 (s, 3H, $-SiCH_3$); 0.87 (s, 9H, $-SiC(CH_3)_3$); 0.88 (s, 18H, $-SiC(CH_3)_3$); 0.97 (d, *J*= 6.5 Hz, 3H, 8-$CH_3$); 1.05 (d, *J*= 6.5 Hz, 3H, 6-$CH_3$); 1.10 (s, 3H, 4-$CH_3$); 1.14 (s, 3H, 4-$CH_3$); 1.29-1.41 (m, 3H); 1.51 (m, 3H); 1.58 (m, 1H); 1.67 (s, 3H, 12-$CH_3$); 1.90-2.00 (m, 2H, 11-H); 1.99 (d, $^4J$= 1.0 Hz, 3H, 16-$CH_3$); 2.18-2.30 (m, 2H, 14-H); 2.70 (s, 3H, TAr-$CH_3$); 3.22 (qd, *J*= 7.0 Hz, *J*= 2.0 Hz, 1H, 6-H); 3.40 (d, *J*= 8.0 Hz, 1H, 7-OH); 3.47 (s, 1H); 3.58-3.69 (m, 2H); 4.03 (dd, *J*= 7.0 Hz, J= 3.5 Hz, 1H, 15-H); 4.08 (t, *J*= 7.0 Hz, 1H, 3-H); 5.14 (t, *J*= 7.0 Hz, 1H, 13-H); 6.45 (s, 1H, 17-H); 6.90 (s, 1H, TAr-$CH_{arom}$).

**$^{13}$C-NMR** (100 MHz, $CDCl_3$) pol. Produkt: $\delta$ in ppm =
-5.3 ($SiCH_3$); -4.9 ($SiCH_3$); -4.7 ($SiCH_3$); -4.0 ($SiCH_3$); -3.7 ($SiCH_3$); 10.8 (C-6); 13.9 (16-$CH_3$); 15.5 (8-$CH_3$); 18.4 ($Si\mathbf{C}(CH_3)_3$); 19.2 (TAr-$CH_3$); 19.6 (4-$C\mathbf{H}_3$); 22.8 (4-$CH_3$); 23.6 (12-$CH_3$); 25.2 (C-11); 25.8 ($SiC(\mathbf{C}H_3)_3$); 25.9 ($Si\mathbf{C}(CH_3)_3$); 26.2 ($SiC(\mathbf{C}H_3)_3$); 32.3 (C-10); 32.9 (C-9); 35.4 (C-14); 35.5 (C-8); 37.8 (C-2); 41.4 (C-6); 54.3 (C-4); 60.2 (C-1); 72.7 + 75.1 (C-3 u. C-7); 79.0 (C-15); 114.9 (C-19); 118.7 (C-17); 121.7 (C-13); 136.6 (C-12); 142.5 (C-16); 153.2 (C-18); 164.3 (C-20); 221.8 (C-5).

**IR** (Si-Film): $\nu$ in $cm^{-1}$ =
2956m; 2928m; 2856m; 1686w; 1619w; 1508w; 1472m; 1462m; 1407w; 1388m; 1361m; 1322w; 1255m; 1183m; 1104vs; 1032m; 977m; 939m; 836s; 775s; 738m.

Beispiel W:

**(3S, 6R, 7S, 8S, 12Z, 15S, 16*E*)-4,4,6,8,12,16-hexamethyl-16-(2-methylthiazol-4-yl)-1,3,7,15-tetrakis[(1,1-dimethylethyl)-dimethylsilyloxy]-heptadec-12,16-dien-5-on**

In 15 mL abs. $CH_2Cl_2$ werden 493 mg (0.588 mmol) der nach Beispiel V hergestellten Verbindung bei 0°C mit 250 µL 2,6-Lutidin (3 eq, 1.764 mmol) vorgelegt. Nun werden 203 µL (1.5 eq, 0.882 mmol) TBSTriflat langsam zugetropft.

Nach 3 h bei 0°C wird das Kühlbad entfernt und die Reaktion durch Zugabe von ges. $NH_4Cl$-Lsg. gequenscht. Die Phasen werden getrennt, die wässrige Phase noch zweimal mit $CH_2Cl_2$ extrahiert, die vereinigten org. Phasen anschließend über $MgSO_4$ getrocknet, über 5 cm Kieselgel filtriert und eingeengt.

Chromatographische Reinigung über eine 25:1-Hex/EE-Kieselgelsäule erbrachte 560 mg (quant.) der Titelverbindung, als farbloses Öl.

**$R_f$-Wert** (Hex/EE= 10:1) ≈ 0.56 F IV (intensiv blau);

**$^1$H-NMR** (400 MHz, $CDCl_3$): $\delta$ in ppm =
-0.01 (s, 3H, $-SiCH_3$); 0.017 (s, 3H, $-SiCH_3$); 0.02 (s, 3H, $-SiCH_3$); 0.03 (s, 3H, $-SiCH_3$); 0.047 (s, 6H, $-SiCH_3$); 0.053 (s, 3H, $-SiCH_3$); 0.08 (s, 3H, $-SiCH_3$); 0.871 (s, 9H, $-SiC(CH_3)_3$); 0.876 (s, 9H, $-SiC(CH_3)_3$); 0.88 (s, 9H, $-SiC(CH_3)_3$); 0.875 (v, 3H, 8-$CH_3$); 0.89 (s, 9H, $-SiC(CH_3)_3$); 1.01 (s, 3H, 4-$CH_3$); 1.03 (d, J= 7.0 Hz, 3H, 6-$CH_3$); 1.21 (s, 3H, 4-$CH_3$); 1.24-1.28 (m, 2H); 1.30-1.40 (m, 4H); 1.64 (s, 3H, 12-$CH_3$); 1.90-1.97 (m, 2H, 11-H); 1.98 (d, $^4J$= 1.0 Hz, 3H, 16-$CH_3$); 2.22 (m, 2H, 14-H); 2.70 (s, 3H, TAr-$CH_3$); 3.13 (qd, *J*= ? Hz, *J*= 6.5 Hz, 1H, 6-H); 3.56 (ddd, *J*= ? Hz, *J*= 7.5 Hz, *J*= 2.0 Hz, 1H, 1-H); 3.66 (td, *J*= 9.6 Hz, J= 9.0 Hz, *J*= 5.0 Hz, 1H, 1-H); 3.75 (dd, *J*= 7.0 Hz, *J*= 1.5 Hz, 1H, 7-H); 3.87 (dd, *J*= 7.5 Hz, *J*= 2.5 Hz, 1H, 15-H); 4.07 (t, *J*= 6.5 Hz, 1H, 3-H); 5.12 (t, *J*= 7.0 Hz, 1H, 13-H); 6.44 (s, 1H, 17-H); 6.90 (s, 1H, TAr-$CH_{arom}$).

**$^{13}$C-NMR** (100 MHz, $CDCl_3$): $\delta$ in ppm =
-5.3 ($SiCH_3$); -4.9 ($SiCH_3$); -4.7 ($SiCH_3$); -4.0 ($SiCH_3$); -3.8 ($SiCH_3$); -3.7 ($SiCH_3$); 13.9 (16-$CH_3$); 15.4 (8-$CH_3$); 17.5 (6-$CH_3$); 18.2 ($Si\mathbf{C}(CH_3)_3$); 18.3 ($Si\mathbf{C}(CH_3)_3$); 18.5 ($Si\mathbf{C}(CH_3)_3$); 19.2 (TAr-$CH_3$); 19.4 (4-$CH_3$); 23.5 (12-$CH_3$); 24.5 (4-$CH_3$); 24.9 (C-11); 25.9 ($SiC(\mathbf{C}H_3)_3$); 26.0 ($SiC(\mathbf{C}H_3)_3$); 26.1 ($SiC(CH_3)_3$); 26.2 ($SiC(CH_3)_3$); 31.1 ($CH_2$); 32.6 ($CH_2$); 35.3 (C-14); 38.1 (C-2); 39.0 (CH); 45.0 (C-6); 53.7 (C-4); 61.0 (C-1); 74.1 (C-3); 77.5 (C-7); 79.0 (C-15); 114.9 (C-19); 118.7 (C-17); 121.6 (C-13); 136.8 (C-12); 142.5 (C-16); 153.3 (C-18); 218.2 (C-5).

Beispiel X:

**(3S, 6R, 7S, 8S, 12Z, 15S, 16*E*)-4,4,6,8,12,16-hexamethyl-16-(2-methylthiazol-4-yl)-3,7,15-tris[(1,1-dimethylethyl)-dimethylsilyloxy]-heptadec-12,16-dien-5-on-1-ol**

In 20 mL MeOH/$CH_2Cl_2$ (1:1) werden 540 mg (≈0.567 mmol) der nach Beispiel W hergestellten Verbindung bei 0°C mit 132 mg (1 eq, 0.567 mmol) CSA langsam versetzt und 4 h nachgerührt.

Zur Aufarbeitung wird mit $CH_2Cl_2$ verdünnt und die Reaktion durch Zugabe von ges. $NaHCO_3$-Lsg. gequenscht. Anschließend werden die Phasen getrennt, die wässrige Phase wird noch zweimal mit $CH_2Cl_2$ extrahiert, die vereinigten org. Phasen werden über $MgSO_4$ getrocknet, filtriert und einrotiert.

Flashchromatographie über eine 10:1-Hex/EE-Kieselgelsäule erbrachte 412 mg (87%) der Titelberbindung, als farbloses Öl.

**$R_f$-Wert** (Hex/EE= 10:1) ≈ 0.17 F III (intensiv blau);
**$R_f$-Wert** (Hex/EE= 5:1) ≈ 0.32 F IV (intensiv blau);

**$^1$H-NMR** (400 MHz, $CDCl_3$): δ in ppm =
-0.01 (s, 3H, -$SiCH_3$); 0.03 (s, 3H, -$SiCH_3$); 0.06 (s, 9H, 3mal -$SiCH_3$); 0.10 (s, 3H, -$SiCH_3$); 0.86-0.92 (m, 30H, 3mal -$SiC(CH_3)_3$ und 8-$CH_3$); 1.048 (d, J= 6.9 Hz, 3H, 6-$CH_3$); 1.051 (s, 3H, 4-$CH_3$); 1.09-1.20 (m, 2H); 1.21 (s, 3H, 4-$CH_3$); 1.26-1.50 (m, 5H); 1.65 (s, 3H, 12-$CH_3$); 1.90-2.05 (m, 2H, 11-H); 1.98 (d, $^4J$= 0.9 Hz, 3H, 16-$CH_3$); 2.22 (mc, 2H, 14-H); 2.70 (s, 3H, TAr-$CH_3$); 3.12 (qd, *J*= 7.1 Hz, *J*= 6.7 Hz, 1H, 6-H); 3.63 (dd, *J*= 6.0 Hz, *J*= 5.6 Hz, 2H, 1-H); 3.79 (dd, *J*= 7.1 Hz, *J*= 1.3 Hz, 1H, 7-H); 4.03-4.10 (m, 2H, 3-H und 15-H); 5.13 (t, *J*= 7.5 Hz, 1H, 13-H); 6.44 (s, 1H, 17-H); 6.90 (s, 1H, TAr-$CH_{arom}$).

**$^{13}$C-NMR** (100 MHz, $CDCl_3$): δ in ppm =
-4.9 ($SiCH_3$); -4.7 ($SiCH_3$); -3.93 ($SiCH_3$); -3.91 ($SiCH_3$); -3.8 ($SiCH_3$); -3.6 ($SiCH_3$); 13.9 (16-$CH_3$); 14.2 (6-$CH_3$); 15.6 (8-$CH_3$); 17.6 (4-$CH_3$); 17.8 (4-$CH_3$), 18.2 (Si**C**$(CH_3)_3$); 18.3 (Si**C**$(CH_3)_3$); 18.5 (Si**C**$(CH_3)_3$); 19.2 (TAr-$CH_3$); 23.5 (12-$CH_3$); 24.9 (C-11); 25.9 (SiC(**C**$H_3)_3$); 26.0 (SiC(**C**$H_3)_3$); 26.2 (SiC(**C**$H_3)_3$); 30.9 (C-10); 32.5 (C-9); 35.4 (C-14); 38.4 (C-2); 38.8 (CH); 45.1 (C-6); 53.8 (C-4); 60.2 (C-1); 73.1 (C-3); 77.5 (C-7); 79.0 (C-15); 114.9 (C-19); 118.7 (C-17); 121.6 (C-13); 136.7 (C-12); 142.5 (C-16); 153.3 (C-18); 164.3 (C-20); 218.2 (C-5).

**IR** (Si-Film): ν in $cm^{-1}$ =
3320br; 2956s; 2928s; 1856s; 1686w; 1472m; 1462m; 1407w; 1388m; 1361 w; 1255s; 1183w; 1104vs; 1032m; 977m; 939s; 836vs; 775vs; 738s.

**MS** (FI, 7 kV, 3mA, 195°C): m/e =
840 ([M+H]); 839 ([M]); 781; 706; 649; 550; 283; 282 (100); 268; 189; 132; 115; 57.

Beispiel Y:

**(3S, 6R, 7S. 88, 12*Z*, 15S, 16*E*)-4,4,6,8,12,16-hexamethyl-16-(2-methylthiazol-4-yl)-3,7,15-tris[(1,1-dimethylethyl)-dimethylsilyloxy]-heptadec-12,16-dien-5-onsäure**

In 50 mL abs. $CH_2Cl_2$ und 1 mL abs. Pyridin werden 389 mg (0.4639 mmol) der nach Beispiel X hergestellten Verbindung mit 258 mg (1.3 eq) Dess-Martin-Periodinan versetzt und 2 h nachgerührt. Nun wird mit 150 mL Ether verdünnt, filtriert und das Filtrat mit 100 mL ges. $NaHCO_3$/ges. $Na_2S_2O_3$-Lsg. (1:1) gewaschen. Die Phasen werden getrennt, die wässerige Phase wird noch zweimal mit $CH_2Cl_2$ extrahiert, die vereinigten org. Phasen werden über $MgSO_4$ getrocknet, über 1 cm Kieselgel filtriert, eingeengt und getrocknet. Der Rohaldehyd wird ohne Reinigung roh, nach Trocknung im Ölpumpenvakuum gleich weiter umgesetzt.

Der Rohaldehyd wird in 10.3 mL tert.-Butanol und 10.3 mL 2,3-Dimethylbut-2-en bei RT mit einer Lsg. von 211 mg (5 eq) $NaClO_2$ und 211 mg $NaH_2PO_4$ in 2.11 mL Wasser versetzt und 40 min nachgerührt. Zu Aufarbeitung wird mit 150 mL (2:1) $CH_2Cl_2$/Wasser verdünnt, mit 2 Tropfen TFA leicht angesäuert, die Phasen getrennt, die wässerige Phase wird noch zweimal mit $CH_2Cl_2$ extrahiert, die vereinigten org. Phasen werden über $MgSO_4$ getrocknet, über 1 cm Kieselgel filtriert und eingeengt. Flashchromatographie über eine 10:1-5:1-3:1-Hex/EE-Gradientenkieselgelsäule erbrachte 380 mg (96.1%) der Titelverbindung, als sehr zähes, farbloses Öl.

**$R_f$-Wert** (Hex/EE= 5:1) vom Aldehyd ≈ 0.56 F III oder F IV (intensiv blau);
**$R_f$-Wert**(Hex/EE=5:1)Titetverbindung≈0.30 F III oder F IV (intensiv violettblau);

**$^1$H-NMR** (400 MHz, $CDCl_3$): δ in ppm =
-0.02 (s, 3H, $-SiCH_3$); 0.02 (s, 3H, $-SiCH_3$); 0.03 (s, 3H, $-SiCH_3$); 0.07 (s, 3H, $-SiCH_3$); 0.08 (s, 3H, $-SiCH_3$); 0.12 (s, 3H, $-SiCH_3$); 0.88 (s, 18H. $-SiC(CH_3)_3$); 0.885 (v, 3H, 8-$CH_3$); 0.89 (s, 9H, $-SiC(CH_3)_3$); 1.06 (d, J= 7.0 Hz, 3H, 6-$CH_3$); 1.15 (s, 6H, 4-$CH_3$); 1.35-1.50 (m, 5H); 1.67 (s, 3H, 12-$CH_3$); 1.86-1.94 (m, 2H, 11-H); 1.94 (d, $^4J$= 1.0 Hz, 3H, 16-$CH_3$); 2.12-2.22 (m, 2 od. 3H); 2.33 (dd, *J*= 16.6 Hz, *J*= 6.5 Hz, 1H, 2-H); 2.43 (dd, *J*= 16.6 Hz, *J*= 3.5 Hz, 1H, 2-H); 2.70 (s, 3H, TAr-$CH_3$); 3.13 (qd, *J*= 7.0 Hz, *J*= 6.0 Hz, 1H, 6-H); 3.74 (dd, *J*= 5.5 Hz, *J*= 2.0 Hz, 1H, 7-H); 4.12 (m, 1H, 15-H); 4.40 (dd, *J*= 7.0 Hz, *J*= 3.5 Hz, 1H, 3-H); 5.17 (t, *J*= 7.0 Hz, 1H, 13-H); 6.63 (s, 1H, 17-H); 6.92 (s, 1H, TAr-$CH_{arom}$).

**$^{13}$C-NMR** (100 MHz, $CDCl_3$): δ in ppm =
-5.0 ($SiCH_3$); -4.7 ($SiCH_3$); -4.5 ($SiCH_3$); -4.1 ($SiCH_3$); -4.0 ($SiCH_3$); -3.9 ($SiCH_3$); 14.0 (16-$CH_3$); 15.8 (8-$CH_3$); 16.4 (6-$CH_3$); 18.3 (Si**C**$(CH_3)_3$); 18.5 (Si**C**$(CH_3)_3$); 18.6 (4-$CH_3$); 18.7 (4-$CH_3$); 23.4; 23.5 (12-$CH_3$); 24.9 (C-11); 25.6; 25.8 (SiC(**C**$H_3)_3$); 26.1 (SiC(**C**$H_3)_3$); 26.2 (SiC(**C**$H_3)_3$); 31.7 (C-10); 32.5 (C-9); 33.9 (C-9); 35.4 (C-14); 39.5 (C-8 ?); 39.9; 44.2 (C-6); 54.0 (C-4); 63.7; 73.1 (C-3); 76.7 (C-7); 79.2 (C-15); 114.4 (C-19); 118.1 (C-17); 121.7 (C-13); 137.1 (C-12); 143.7 (C-16); 152.7 (C-18); 165.3 (C-20); 174.0 (C-1); 218.2 (C-5).

**MS** (FI, 7 eV, 3mA, 195°C): m/e =
854 ([M+H]); 853 ([M]); 796; 795; 720; 684; 663; 649; 593; 541; 283; 282; 203; 168; 93; 57.

Beispiel Z:

**(3S, 6R, 7S, 8S, 12*Z*, 15S, 16*E*)-3,7-bis[(1,1-dimethylethyl)-dimethylsilyloxy]-4,4,6,8,12,16-hexamethyl-15-hydroxy-16-(2-methylthiazol-4-yl)-heptadec-12,16-dien-5-onsäure**

In 10 mL abs. THF werden 365 mg (0.44576 mmol) der nach Beispiel Y hergestellten Verbindung bei RT mit 2.23 mL (5 eq) einer 1M-TBAF-Lsg. (im THF) versetzt und 10 h nachgerührt.

Zur Aufarbeitung wird mit 100 mL Ether verdünnt und durch Zugabe von 70 mL ges. $NH_4Cl$-Lsg. gequenscht. Die Phasen werden getrennt, die wässerige Phase wird noch zweimal mit Ether extrahiert, die vereinigten org. Phasen werden über $MgSO_4$ getrocknet, filtriert und eingeengt.

Flashchromatographie über eine 6:5:1-4:3:1-2:1:1-MC/Hex/EE-Gradientenkieselgel-säule erbrachte 180 mg der Titelverbindung, als farbloses, zähes Öl.

**$^1$H-NMR** (400 MHz, $CDCl_3$): δ in ppm =
0.04 (s, 3H, $-SiCH_3$); 0.055 (s, 3H, $-SiCH_3$); 0.07 (s, 3H, $-SiCH_3$); 0.10 (s, 3H, $-SiCH_3$); 0.865 (s, 9H, $-SiC(CH_3)_3$); 0.89 (s, 9H, $-SiC(CH_3)_3$); 0.90 (d (hv), *J*≈ 7.0 Hz, 3H, 8-$CH_3$); 1.05 (d, *J*= 6.5 Hz, 3H, 6-$CH_3$); 1.10 (s, 3H, 4-$CH_3$); 1.19 (s, 3H, 4-$CH_3$); 1.34-1.50 (m, 2H); 1.48-1.56 (mc, 3H); 1.70 (s, 3H, 12-$CH_3$); 1.92-2.01 (m, 1H, 14-H); 2.01 (d, $^4J$= 1.0 Hz, 3H, 16-$CH_3$); 2.04-2.14 (m, 1H, 14-H); 2.26 (dd, *J*= 16.6 Hz, *J*= 6.0 Hz, 1H, 2-H); 2.33 (mc, 2H, 11-H); 2.435 (dd, *J*= 16.6 Hz, *J*= 4.0 Hz, 1H, 2-H); 2.70 (s, 3H, TAr-$CH_3$); 3.14 (qd, *J*= 7.0 Hz, *J*= 6.5 Hz, 1H, 6-H); 3.76 (dd, *J*= 7.0 Hz, *J*= 2.0 Hz, 1H, 7-H); 4.14 (t, *J*≈ 6.5 Hz, 1H, 15-H); 4.40 (dd, *J*= 6.0 Hz, *J*= 4.0 Hz, 1H, 3-H); 5.17 (t, *J*= 7.0 Hz, 1H, 13-H); 6.63 (s, 1H, 17-H); 6.94 (s, 1H, TAr-$CH_{arom}$).

**$^{13}$C-NMR** (100 MHz, $CDCl_3$): δ in ppm =
-4.7 ($SiCH_3$); -4.1 ($SiCH_3$); -3.9 ($SiCH_3$); -3.8 ($SiCH_3$); 13.8 (16-$CH_3$); 14.6 (8-$CH_3$); 16.0 (6-$CH_3$); 17.1; 18.2 (Si**C**$(CH_3)_3$); 18.5 (Si**C**$(CH_3)_3$); 18.9 (TAr-$CH_3$); 19.9 (4-$CH_3$); 23.1 (4-$CH_3$); 23.6 (12-$CH_3$); 26.1 (SiC(**C**$H_3)_3$); 26.2 (SiC(**C**$H_3)_3$); 26.3 ($CH_2$); 26.4 ($CH_2$); 31.3 ($CH_2$); 32.5 ($CH_2$); 34.2 ($CH_2$); 39.2 (CH); 40.9 ($CH_2$); 44.8 (CH); 51.9; 53.8 (C-4); 74.2 (C-3); 77.3 (C-7); 77.4 (C-15); 115.1 (C-19); 118.7 (C-17); 120.3 (C-13); 139.4 (C-12); 142.2 (C-16); 152.7 (C-18); 165.0 (C-20); 174.9 (C-1); 217.9 (C-5).

**IR** (51-Film): ν in $cm^{-1}$ =
3357br; 2958s; 2932s; 2856m; 1696m; 1508w; 1472m; 1387m; 1293m; 1255m; 1186m; 1106vs; 988s; 952m; 875s; 837vs; 815m; 776vs; 737s.

**MS** (FI, 7 eV, 3mA, 190°C): m/e =
739 ([M+H]); 738 ([M]); 722; 721; 720; 682; 662; 606; 427; 329; 295; 203; 129; 57.

Beispiel AA:

**Silyliertes Epothilon D**

In einem 250 mL Dreihalsrundkolben mit Argonanschluss, Druckausgleich und Intensivkühler werden 65 mg (0.338 mmol, 2 eq) N'-(3-Dimethylaminopropyl)-N-ethylcarbodiimid·HCl (EDCl), 54 mg (0.507 mmol, 3 eq) DMAP und 54 mg (0.338 mmol, 2 eq) DMAP·HCl in 100 mL ethanolfreiem abs. $CHCl_3$ [über bas. Alox B filtriert und über $CaH_2$ destilliert] vorgelegt. Die Lösung wird auf Rückflußtemperatur erwärmt und anschließend eine Lösung von 125 mg (0.169 mmol) der nach Beispiel Z hergestellten Verbindung in 8 mL abs. $CHCl_3$, per Injektionsapparatur über einen Zeitraum von 17 h tropfenweise zugegeben und 30 min nach der letzten Zugabe nachgerührt.

Die Reaktionslösung wird abkühlen gelassen und durch Zugabe von 100 mL ges. $NH_4Cl$-Lsg. gequenscht und 30 min kräftig nachgerührt. Anschließend werden die Phasen getrennt, die wässrige Phase zweimal mit $CH_2Cl_2$ extrahiert, die vereinigten org. Phasen über $MgSO_4$ getrocknet, über 0.5 cm Kieselgel filtriert und eingeengt.

Chromatographie über eine 25:1-Hex/EE-Gradientenkieselgelsäule erbrachte 84 mg (69%) der Titelverbindung, als fahl gelbliches Öl.

**$R_f$-Wert** (Hex/EE= 10:1) ≈ 0.37 F III u. F IV (violettblau);

**$^1$H-NMR** (400 MHz, $CDCl_3$): δ in ppm =
- 0.12 (s, 3H, -$SiCH_3$); 0.07 (s, 3H, -$SiCH_3$); 0.095 (s, 3H, -$SiCH_3$); 0.10 (s, 3H, -$SiCH_3$); 0.83 (s, 9H, -SiC$(CH_3)_3$); 0.93 (s, 9H, -SiC$(CH_3)_3$); 0.97 (d, J= 7.0 Hz, 3H, 8-$CH_3$); 1.09 (d, J= 6.5 Hz, 3H, 6-$CH_3$); 1.13 (s, 3H, 4-$CH_3$); 1.18 (s, 3H, 4-$CH_3$); 1.47-1.61 (m, 3H); 1.66 (s, 3H, 12-$CH_3$); 1.66-1.77 (m, 2 H); 2.02-2.08 (m, 1H od. 2H); 2.09 (d, $^4J$= 1.0 Hz, 3H, 16-$CH_3$); 2.41-2.49 (m, 1H); 2.62-2.71 (m, 2H); 2.69 (s, 3H, TAr-$CH_3$); 2.79 (dd, *J*= 16.6 Hz, *J*= 1.5 Hz, 1H, 2-H); 3.01 (qd, *J*= 7.0 Hz, *J*= 6.5 Hz, 1H, 6-H); 3.88 (d, *J*= 9.0 Hz, 1H, 7-H); 4.02 (d, *J*= 8.5 Hz, 1H, 3-H); 4.96 (d, *J*= 9.6 Hz, 1H, 15-H); 5.15 (t, *J* ≈ 8.0 Hz, 1H, 13-H); 6.55 (s, 1H, 17-H); 6.95 (s, 1H, TAr-$CH_{arom}$).

**$^{13}$C-NMR** (100 MHz, $CDCl_3$): δ in ppm =
-5.6 ($SiCH_3$); -3.70 ($SiCH_3$); -3.69 ($SiCH_3$); -3.3 ($SiCH_3$); 15.3 + 17.8 (C-16 u. C-6); 18.6 (Si**C**$(CH_3)_3$); 18.7 (Si**C**$(CH_3)_3$); 19.2 (TAr-$CH_3$); 23.1 (4-$CH_3$'s); 24.3; 24.5; 25.7; 26.2 (SiC(**C**$H_3)_3$); 26.4 (SiC(**C**$H_3)_3$); 27.4; 29.7; 31.4; 31.9; 32.5; 39.2 (C-2); 53.4 (C-4); 76.3 (C-3 u./od. C-7); 79.9 (C-15); 115.9 (C-19); 119.2 (C-17); 119.4 (C-13); 138.8 (C-12); 140.6 (C-16); 152.5 (C-18); 164.5 (C-20); 171.2 (C-1); 215.1 (C-5).

**$C_{39}H_{69}NO_5SSi_2$:**
(M= 720.21 g·mol$^{-1}$)

Beispiel AB:

**Epothilon D**

In 5 mL abs. THF werden 75 mg (0.104 mmol) der nach Beispiel AA hergestellten Verbindung bei 0°C mit 1.5 mL abs. Pyridin vorgelegt. Nun werden 1.5 mL HF·Pyridin tropfenweise zugegeben und langsam auf RT erwärmen gelassen. Nach 18 h (fast nur Edukt) werden weitere 1.5 mL HF·Pyridin tropfenweise, wieder bei 0°C, nachgegeben und 18 h nachgerührt.

Zur Aufarbeitung wird bei 0°C tropfenweise mit ges. $NaHCO_3$-Lsg. versetzt, anschließend mit Diethylether und ges. $NaHCO_3$-Lsg. verdünnt. Anschließend werden die Phasen getrennt, die wässrige Phase dreimal mit Diethylether extrahiert, die vereinigten org. Phasen über $MgSO_4$ getrocknet, filtriert und eingeengt.

Chromatographie über eine 3:1-Hex/EE-Kieselgelsäule erbrachte 44 mg (69.8%) (monodesilyliertes Produkt) und 10 mg (19.6%) der Titelverbindung (Epothilon D). Das monodesilylierte Produkt wird nochmals obigen Bedingungen unterworfen. Nach Chromatographie über eine 3:1-Hex/EE-Kieselgelsäule wurden nochmals 39 mg (76.3%) der Titelverbindung erhalten, insgesamt also 49 mg (95.8%) Epothilon D.

**$R_f$-Wert** (Hex/EE= 1:1) monosilyliert ≈ 0.67 F IV (blau);
**$R_f$-Wert** (Hex/EE= 3:1) monosilyliert ≈ 0.19 F IV (blau);
**$R_f$-Wert** (Hex/EE= 1:1) Titelverbindung ≈ 0.52 F IV (blau);
**$R_f$-Wert** (Hex/EE= 3:1) Titelverbindung ≈ 0.11 F IV (blau);

**$^1$H-NMR** (400 MHz, $CDCl_3$): δ in ppm =

1.01 (d, J= 7.0 Hz, 3H, 8-$CH_3$); 1.06 (s, 3H, 4-$CH_3$); 1.18 (d, $J$= 7.0 Hz, 3H, 6-$CH_3$); 1.24-1.31 (m, 4H); 1.33 (s, 3H, 4-$CH_3$); 1.65 (s, 3H, 12-$CH_3$); 1.68-1.78 (m, 1 od. 2 H, ? ); 1.83-1.89 (m, 1H); 2.06 (d, $^4J$= 1.0 Hz, 3H, 16-$CH_3$); 2.22 (ddd, $J$= 15.6 Hz, $J_{11\text{-Ha},10\text{-Ha}}$= 3.5 Hz, $J_{11\text{-Ha},10\text{-Hb}}$= 2.0 Hz, 1H, 11-Ha); 2.28 (dd, $J$= 14.6 Hz, $J_{2\text{-Ha},3\text{-H}}$= 3.0 Hz, 1H, 2-Ha); 2.29-2.35 (m, 1H); 2.45 (dd, $J_{2\text{-Hb},3\text{-H}}$= 14.6 Hz, $J_{2\text{-Hb},3\text{-H}}$= 11.0 Hz, 1H, 2-Hb); 2.63 (dt, $J$= 15.1 Hz, $J_{14\text{-Ha},15\text{ u. }13\text{-H}}$≈ 10.0 Hz, 1H, 14-Ha); 2.68 (s, 3H, TAr-$CH_3$); 3.02 (br, 1H, OH); 3.15 (qd, $J_{6\text{-H},6\text{-CH}3}$= 6.9 Hz, $J_{6\text{-H},7\text{-H}}$= 2.5 Hz, 1H, 6-H); 3.45 (br, 1H, OH); 3.71 (dd, $J_{7\text{-H},8\text{-H}}$= 4.0 Hz, $J_{7\text{-H},6\text{-H}}$= 2.5 Hz, 1H, 7-H); 4.28 (dd. $J_{3\text{-H},2\text{-Hb}}$= 11.0 Hz, $J_{3\text{-H},2\text{-Ha}}$= 2.5 Hz, 1H, 3-H); 5.13 (dd, $J_{13\text{-H},14\text{-Ha}}$ = 10.0 Hz, $J_{13\text{-H},14\text{-Hb}}$= 5.0 HZ, 1H, 13-H); 5.21 (dd, $J_{15\text{-H},14\text{-Ha}}$= 10.0 Hz, $J_{15\text{-H},14\text{-Hb}}$= 1.5 Hz, 1H, 15-H); 6.57 (s, 1H, 17-H); 6.94 (s, 1H, TAr-$CH_{arom}$).

**$^{13}$C-NMR** (100 MHz, $CDCl_3$): δ in ppm =

13.4 (16-$CH_3$); 15.7 (6-$CH_3$); 18.0 (8-$CH_3$); 19.0 (TAr-$CH_3$); 22.9 (4-$CH_3$'s); 25.4 (C-11); 31.6 + 31.7 (C-9 u. C-10); 32.5 (C-14); 38.4 (C-8); 39.6 (C-2); 41.7 (C-6); 53.5 (C-4); 72.3 (C-3); 74.1 (C-7); 78.9 (C-15); 115.6 (C-19); 119.2 (C-17); 120.9 (C-13); 138.4 (C-12); 139.2 (C-16); 152.0 (C-18); 165.0 (C-20); 170.4 (C-1); 220.7 (C-5).

**$C_{39}H_{69}NO_5SSi_2$:**
(M= 720.21 g·mol$^{-1}$)

Beispiel AC:

**Epothilon B**

In 3.5 mL abs. $CHCl_3$ (über Alox B gesäult und von $CaH_2$ destilliert) werden 43 mg (87.4 μmol) der nach Beispiel AB hergestellten Verbindung bei -18°C mit 32.5 mg (≈1.5 eq, 131.1μmol, ca. 70%ig) mCPBA versetzt und 5 h, bei konstant gehaltener Temperatur, gerührt.

Zur Aufarbeitung wird mit 14 mL $CH_2Cl_2$ verdünnt und durch Zugabe von 15 mL ges. $NaHCO_3$-Lsg. die reaktion gequenscht. Die Phasen werden getrennt, die wässrige Phase noch viermal mit $CH_2Cl_2$ extrahiert, die vereinigten organischen Phasen anschließend über $MgSO_4$ getrocknet, filtriert und eingeengt.

Chromatographische Vorreinigung über eine 1:1-Hex/EE-Kieselgelsäule erbrachte 36 mg (81.1%) der Titelverbindung, als 4:1-Gemisch von Epothilon B und seinem α-Epoxidisomer. Eine HPLC-Trennung einer kleinen Menge wurde über 20% iPrOH in Hexan durchgeführt.

**$^1$H-NMR** (400 MHz, $CDCl_3$): δ in ppm =

0.99 (d, $J$= 7.0 Hz, 3H, 8-$CH_3$); 1.07 (s, 3H, 4-$CH_3$); 1.16 (d, $J$= 7.0 Hz, 3H, 6-$CH_3$); 1.27 (s, 3H, 4-$CH_3$); 1.36 (s, 3H, 12-$CH_3$); 1.35-1.45 (m, 3H); 1.46-1.54 (m, 2H); 1.65-1.77 (m, 3H); 1.91 (dd, $J$= 15.6 Hz, $J$= 8.0 Hz, 1H, 14-H); 2.08 (s, 3H, 16-$CH_3$); 2.06-2.13 (m (v), 1H, 14-H); 2.35 (dd, $J$= 13.5 Hz, $J$= 2.5 Hz, 1H, 2-H); 2.53 (dd, $J$= 14.1 Hz, $J$= 10.0 Hz, 1H, 2-H); 2.65 (br, 1H, OH); 2.69 (s, 3H, TAr-$CH_3$); 2.80 (dd, $J$= 7.5 Hz, $J$= 4.5 Hz, 1H, 13-H); 3.29 (qd, $J$= 7.0 Hz, $J$= 4.0 Hz, 1H, 6-H); 3.71 (t, $J$= 4.0 Hz, 1H, 7-H); 4.22 (br, 2H, 3-H u. OH); 5.41 (dd, $J$= 8.0 Hz, $J$= 3.0 Hz, 1H, 15-H); 6.58 (s, 1H, 17-H); 6.96 (s, 1H, TAr-$CH_{arom}$).

Präparative Methoden:

Alle Umsetzungen metallorganischer Reagenzien und alle Reaktionen in absoluten Lösemitteln werden unter Luft- und Feuchtigkeitsausschluß durchgeführt. Die verwendeten Glasapparaturen werden vor Versuchsbeginn mehrmals im Vakuum (ca. 0.01 mbar) ausgeheizt und mit getrocknetem Argon der Firma Linde belüftet. Wenn nicht anders angegeben, werden sämtliche Reaktionsansätze magnetisch gerührt.

Methylenchlorid wird über eine basische Aluminiumoxidsäule der Aktivitätsstufe I (Woelm) vorgetrocknet und über Calciumhydrid absolutiert. Diethylether wird nach Vortrocknung auf einer basischen Aluminiumoxidsäule über eine 8:1 Natrium/Kalium-Legierung refluxiert bis zur stabilen Blaufärbung des Benzophenon-Indikators und vor der Verwendung frisch abdestilliert. Das Tetrahydrofuran (THF) wird über KOH vorgetrocknet, über eine mit basischem Aluminiumoxid beschickte Säule zur filtriert und anschließend über Kalium mit Triphenylmethan als Indikator destilliert.

Der Essigsäureethylester (EE) wird nach Vortrocknung über Calciumchlorid ebenso wie Hexan (Hex) vor der Verwendung zur Säulenchromatographie am Rotationsver-dampfer abdestilliert.

Chromatographische Verfahren:

Sämtliche Reaktionen werden durch Dünnschichtchromatographie (DC) auf Kieselgel-60-Alufolien mit UV-Indikator $F_{254}$ der Firma Merck verfolgt. Als Laufmittel werden zumeist Lösemittelgemische aus Hexan (Hex) und Essigsäureethylester (EE) verwendet. Zum Sichtbarmachen nicht UV-aktiver Substanzen bewährt sich meist Anisaldehyd/Eisessig/Schwefelsäure (1:100:1) als Standard-Tauchreagenz.

Die präperative Säulenchromatographie wird an Kieselgel-60 der Firma Merck (0,04-0,063 mm, 230-400 mesh) durchgeführt, wobei als Eluens Lösemittelgemische aus Hexan (Hex) und Essigsäureethylester (EE) bzw. Diisopropylether dienen.

Im analytischen, wie auch im präperativen Maßstab werden die hochdruckflüssig-keitschromatographischen Trennungen (HPLC) auf Modulsystemen der Firmen Knauer (Pumpe 64, UV- und RI-Detektoren, Säulen und Schreiber), Waters/Millipore (Injek-tionssystem U6K9) und Milton-Roy (Integrator C1-10) durchgeführt. Für die analytische HPLC wird zumeist eine Knauer-Säule (4.250 mm) mit 5 µm Nucleosil und für die präperative HPLC eine Säule (16.250 mm, 32·250 mm bzw. 64·300 mm) mit 7 µm oder 5 µm Nucleosil 50 verwendet.

Färbereagenzien

Färbereagenz I (F I): 1 g Cer(IV)sulfat in 10 ml konz. Schwefelsäure und 90 ml Wasser liefert mit den meisten reduzierbaren Verbindungen intensiv blaue Farbreaktion beim Trocknen.

Färbereagenz II (F II): Eine 10%ige ethanolische Lösung von Molybdatophosphorsäure stellt ein weiteres Tauchreagenz zum Nachweis ungesättigter und reduzierbarer Verbindungen dar. Im Unterschied zum Färbereagenz 1 zeigt das Molydat-Färbereagenz, speziell auf einige Funktionalitäten ansprechend, ein breiteres Farbspektrum bei praktisch gleicher Zuverlässigkeit.

Färbereagenz III (F III): 1 ml Anisaldehyd in 100 ml Ethanol und 2 ml konz. Schwefelsäure stellt ein äußerst empfindliches Färbereagenz dar, daß zudem auch das wohl breiteste Farbspektrum zeigt.

Färbereagenz IV (F IV): 1 g Vanillin in 100 Ethanol und 2 mL konz. Schwefelsäure ist ähnlich, wie das Anisaldehyd-Reagenz ein sehr empfindliches Färbereagenz dar mit breitem Farbspektrum.

Färbereagenz V (F V): 1 g 2,4-Dinitrophenylhydrazin in 25 ml Ethanol, 8 ml Wasser und 5 ml konz. Schwefelsäure stellt ein hervorragendes, seletiv schon ohne Erwärmung auf Aldehyde und etwas langsamer auf Ketone ansprechendes. Tauchreagenz dar.

Färbereagenz VI (F VI): Eine 0.5%ige wässerige Lösung von Kaliumpermanganat zeigt durch Entfärbung oxidierbare Gruppen an, wobei ungesättigte, nicht aromatische Struktureinheiten spontan ohne Erwärmung reagieren.

Spektroskopische Verfahren und allgemeine Analytik:

NMR-Spektroskopie

Die $^1$H-NMR-Spektren werden mit einem DRX 250 DRX 400 Spektrometer der Firma Bruker mit den Substanzen als Lösung in deuterierten Lösemitteln und Tetramethylsilan als internem Standard aufgenommen. Die Auswertung der Spektren erfolgt nach den Regeln erster Ordnung. Ist eine auftretende Signalmultiplizität damit nicht zu erklären, erfolgt die Angabe des beobachteten Liniensatzes. Zur Bestimmung der Stereochemie wird die NOE-Spektroskopie (Nudear Overhauser Effect) verwendet.

Zur Charakterisierung der Signale werden folgende Abkürzungen verwendet: s (Singulett), d (Dublett), dd (Doppeldublett), ddd (6-Liniensystem bei zwei gleichen Kopplungskonstanten bzw. ein 8-Liniensystem bei drei verschiedenen Kopplungs-konstanten), t (Triplett), q (Quartett), quint (Quintett), sext (Sextett), sept (Septett), m (Multiplett), mc (zentriertes Multiplett), br (breit), hv (halbverdecktes Signal) und v (verdecktes Signal).

Die $^{13}$C-NMR-Spektren werden mit einem AC 250 der Firma Bruker mit $CDCl_3$-Signal bei 77,0 ppm als internem Standard vermessen, wobei die Protonenresonanzen breitbandentkoppelt werden.

IR-Spektroskopie

Die Infrarot-Spektren werden mit Geräten der Firma Perkin-Elmer (Modell 257 bzw. 580 B) und Nicolet (FTIR-Interferometer System 55XC) aufgenommen. Die Öle werden als Filme zwischen Kaliumbromidscheiben vermessen. Die Angaben der Banden erfolgt nach fallender Wellenzahl ($cm^{-1}$). Zur Charakterisierung werden folgende Bezeichnungen gewählt: vs (very strong), s (strong), m (medium), w (weak).

Abkürzungen:

**abs.:** absolut, **Ar:** Aryl/Aromat, **ber.:** berechnet, **Brine:** kalt gesättigte Kochsalzlösung, **nBuLi:** nButyllithium, **c:** Konzentration, **COSY:** korrelierte Spektroskopie (correlated spectroscopy), **CSA:** Camphersulfonsäure, **DC:** Dünnschichtchromatographie, **DCM:** Dichlormethan; **DDQ:** Dichloro-dicyano-quinon, **d.e.:** diastereomeric excess, **DIBAH:** Diisobutyl-aluminiumhydrid, **DIPA:** Diisopropylamin, **DMAP:** Dimethylaminopyridin, **DMF:** N,N'-Dimethylformamid, **DMS:** Dimethylsulfid, **DMSO:** Dimethylsulfoxid, **ds:** Diastereoselektion, **EA:** Elementar-analyse, **e.e.:** enantiomeric excess, **EE:** Essigsäureethylester, **EI:** Elektronenstoßionisation, **eq:** Äquivalent(e), **eV:** Elektronenvolt, **FG:** functional group, **FI:** Feldionisation, **gef.:** gefunden, **ges.:** gesättigt(e), **h:** Stunde(n), **Hex:** n-Hexan, **HMDS:** Hexamethyldisilazid, **HPLC:** Hochdruckflüssigkeitschromatographie (high pressure liquid chromatographie, **Hünig Base:** N-Ethyl-diisopropylamin, **HRMS:** High Resolution Massenspektrometrie, **HV:** Hochvakuum, **iPrOH:** 2-Propanol, **IR:** Infrarotspektrometrie/Infrarotspektrum, **J:** Kopplungskonstante, **LDA:** Lithiumdiisopropylamid, **Lsg.:** Lösung, **Lsm.:** Lösemittel, **MC:** Methylenchlorid; **Me: Methyl, MeLi:** Methyllithium, **min:** Minute(n), **MS:** Massenspektrometrie/Massenspektren, **NMR:** Kernmagnetische Resonanz (Nuclear Magnetic Resonanz), **NOE:** Kern-Overhauser-Effekt (Nuclear Overhauser Effect), **PCC:** Pyridiniumchlorochromat, **PG:** Schutzgruppe (protection group), **Ph:** Phenyl, **ppm:** parts per million, **Rkt.:** Reaktion, **rt:** Retentionszeit, **RT:** Raumtemperatur (20-30 °C), **Std.:** Stunde(n), **TBAF:** Tetra-n-Butylammoniumfluorid, **TBDPS:** tert.-Butyldiphenyl-silyl-, **TBDPSCl:** tert.-Butyldiphenyl-silylchlorid, **TBS:** tert.-Butyldimethyl-silyl-, **TBSCl:** tert.-Butyldimethyl-silylchlorid, **TBSTriflat:** tert.-Butyldimethyl-silyl-triflat, **TEA:** Triethylamin, tert./t: tertiär, **TFA:** Trifluorethansäure,

## Patentansprüche

1. Verfahren zur Herstellung von Epothilon B und Derivaten der allgemeinen Formel

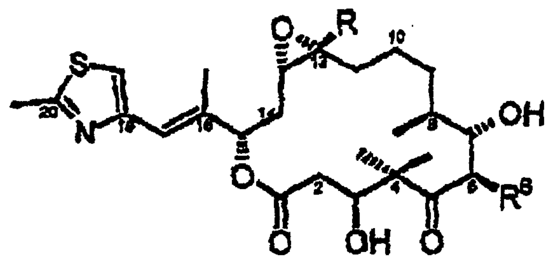

(I)

worin
R eine Methylgruppe und
$R^6$ eine gerad- oder verzweigtkettige Alkylgruppe mit bis zu 6 Kohlenstoffatomen, eine Cycloalkylalkylgruppe mit bis zu 10 Kohlenstoffatomen, eine Phenyl-, 1- oder 2-Naphthyl-, Heteroaryl-, Benzyl- oder Methylheteroarylgruppe bedeuten,
**dadurch gekennzeichnet, daß** eine Verbindung der allgemeinen Formel 3

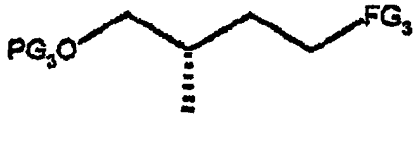

(3)

worin
$PG_3$ eine Hydroxyschutzgruppe und
$FG_3$ eine Phenylsulfonylgruppe

bedeuten,
mit einer Verbindung der allgemeinen Formel 4

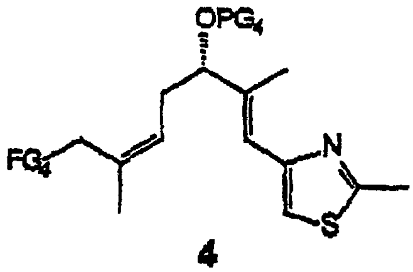

**4**

worin
$FG_4$ ein Todatom oder eine andere Fluchtgruppe und
$PG_4$ eins Hydroxyschutzgruppe
bedeuten,
zu einer Verbindung der allgemeinen Formet 34-I

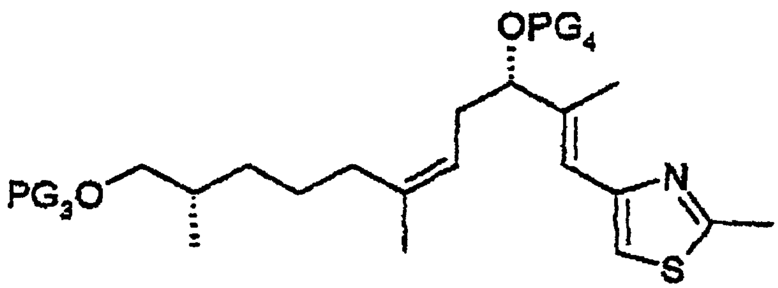

**(34-I)**

worin
$PG_3$ und $PG_4$ die vorstehend angegebenen Bedeutungen haben, umgesetzt,
die Verbindung der allgemeinen Formel 34-I mit einer Verbindung der allgemeinen Formel 2

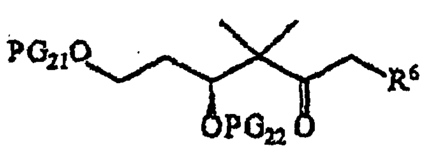

**2**

worin
$PG_{21}$ und $PG_{22}$ unabhängig voneinander jewells eine Hydroxyschutzgruppe und
$R^6$ eine gerad- oder verzweigtkettige Alkylgruppe mit bis zu 6 Kohlenstoffatomen, eine Cycloalkylalkylgruppe mit bis zu 10 Kohlenstoffatomen, eine Phenyl-, 1-oder 2-Naphtyl-, Heteroaryl-, Benzyl- oder Methylheteroarylgruppe bedeuten,
zu einer Verbindung der allgemeinen Formel 234

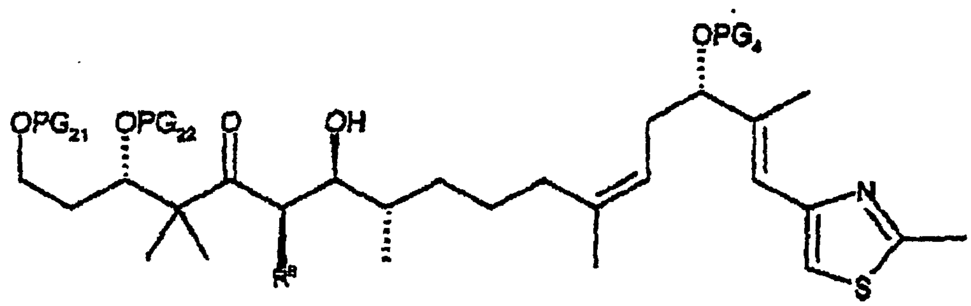

(234)

worin

$PG_{21}$, $PG_{22}$, $PG_4$ und $R^6$ die bereits angegebenen Bedeutungen haben,
und diese Verbindung der allgemeinen Formel 234 über die Stufen

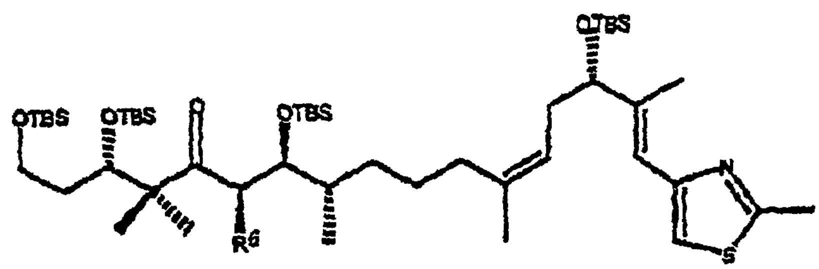

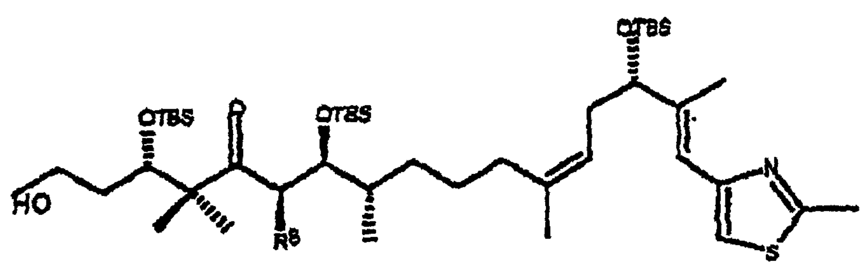

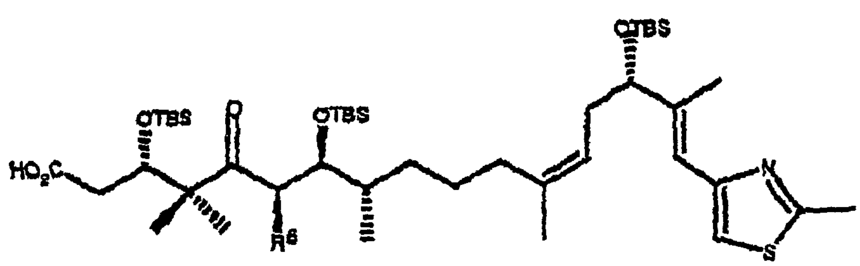

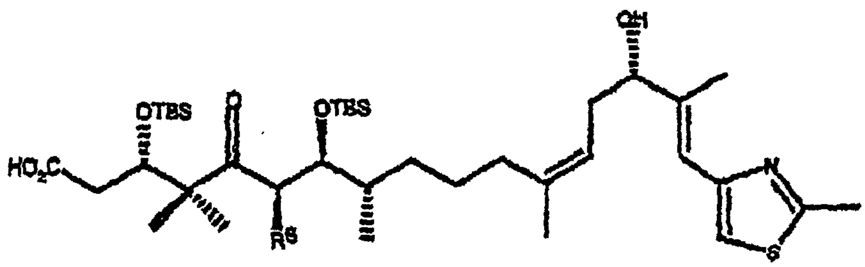

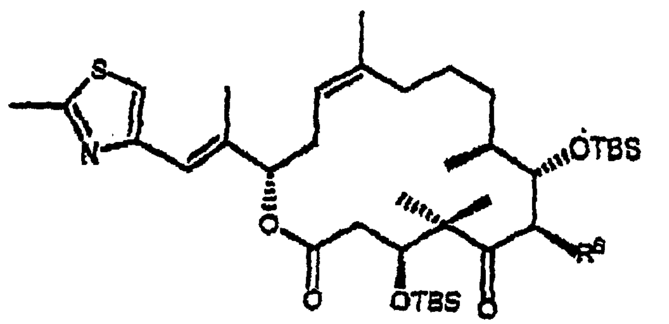

zum Epothilon D oder einem Derivat von Epothilon D umgesetzt

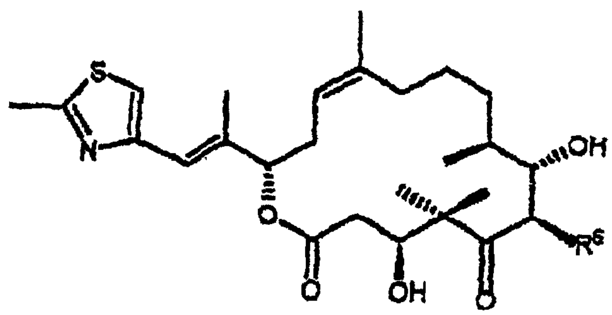

und dieses dann gegebenenfalls durch Epoxidierung in Epothilon B (R = $CH_3$) oder ein Derivat von Epothilon **B** überführt wird.

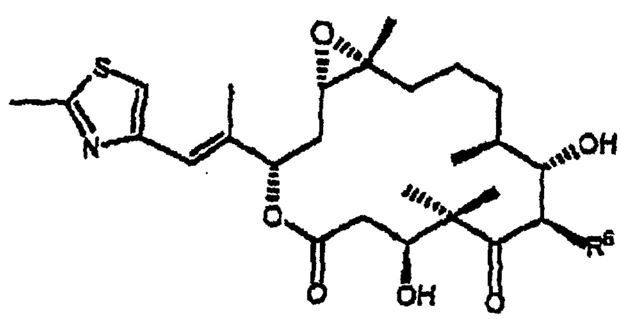

**2.** Verfahren zur Herstellung der Verbindungen der allgemeinen Formel 2 gemäß Anspruch 1

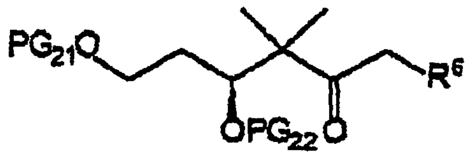

**2**

worin
$PG_{21}$ und $PG_{22}$ unabhängig voneinander jeweils eins Hydroxyschutzgruppe und
$R^6$ eine gerad- oder verzweigtkettige Alkylgruppe mit bis zu 6 Kohienstoffatomen, eine Cycloalkylalkylgruppe mit bis zu 10 Kohlenstoffatomen, eine Phenyl-, 1- oder 2-Naphthyl-, Heteroaryl-, Benzyl- oder Methylheteroarylgruppe bedeuten,
**dadurch gekennzeichnet, daß** eine Verbindung der allgemeinen Formel 2-I

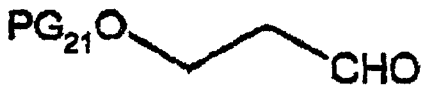

**(2-I)**

worin
$PG_{21}$ eine Hydroxyschutzgruppe
bedeutet,
unter chiraler Katalyse mit einem Silylketenacetal der allgemeinen Formel

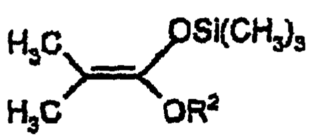

**($R^2$ = Methyl, Ethyl)**..

unter Vermittlung durch N-Tosylvalin/Diboran
zu einer Verbindung der allgemeinen Formel 2-II

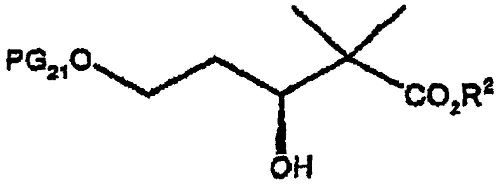

**(2-II)**

worin
$PG_{21}$ und $R_2$ die vorstehend angegebenen Bedeutungen haben, umgesetzt,
anschließend die 3-Hydroxygruppe unter Erhalt einer Verbindung der allgemeinen Formel 2-III

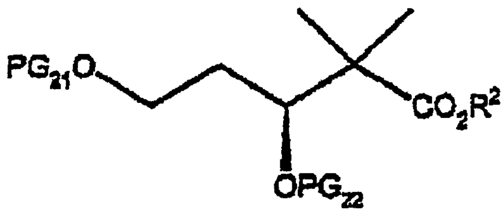

(2-III)

worin
$PG_{21}$ und $PG_{22}$ unabhängig voneinander jeweils eine Hydroxyschutzgruppe bedeuten und $R^2$ die vorstehend angegebene Bedeutung hat,
geschützt,
dann an die Carbonylgruppe der Verbindung 2-III eine Methylgruppe unter Ethalt einer Verbindung der allgemeinen Formel 2-IV

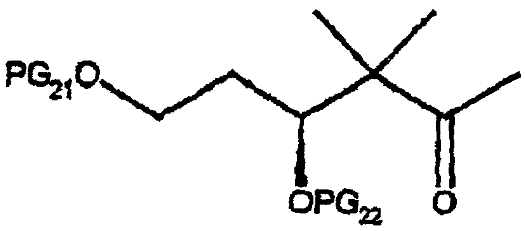

(2-IV)

worin
$PG_{21}$ und $PG_{22}$ unabhängig voneinander jeweils eine Hydroxyschutzgruppe bedeuten,
addiert,
und anschließend mit einem Alkyl-, Cycloalkylalkyl-, Aryl-, Heteroaryl-, Methylaryl- oder Methylheteroarylhalogenid der allgemeinen Formel 2-X

$$R^6Hal \qquad (2\text{-}X)$$

worin
$R^6$ die bereits angegenene Bedeutung hat und
Hal für ein Halogenatom Fluor, Chlor oder Brom steht,
in eine Verbindung der allgemeinen Formel 2

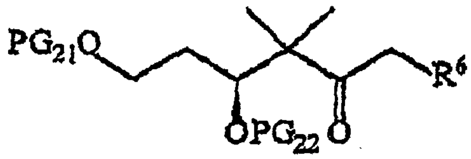

(2)

worin
$PG_{21}$, $PG_{22}$ und $R^6$ die vorstehend angegebenen Bedeutungen haben,
überführt
oder die Verbindung 2-III zum Alkohol reduziert, dieser selektiv zum Aldehyd oxidiert, an diesen mit einem Metallorganyl ein Rest der Formel $-CH_2-R^6$ addiert und der entstandene Alkohol zum Keton der allgemeinen Formel 2 oxidiert wird.

**3.** Verfahren zur Herstellung der Verbindungen der allgemeinen Formel **3** gemäß Anspruch 1

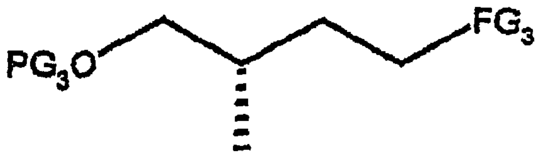

**(3)**

worin
$PG_3$ eine Hydroxyschutzgruppe und
$FG_3$ eine Phenylsulfonylgruppe
bedeuten,
**dadurch gekennzeichnet, daß** die freie Hydroxylgruppe der Verbindung der allgemeinen Formel 3-I

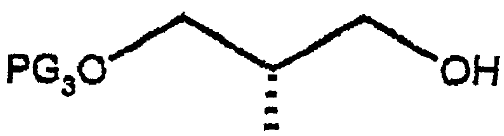

(3-I)

worin
$PG_3$ eine Hydroxyschutzgruppe
bedeutet,
in eine bessere Abgangsgruppe $FG_{31}$ (Verbindung 3-II)

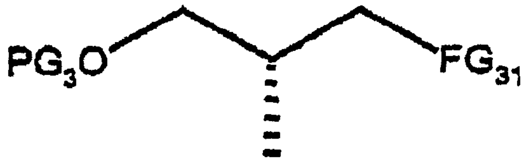

(3-II)

überführt
und danach unter Verdrängung von $FG_{31}$ die Gruppe $-CH_2-SO_2$-Phenyl ($PG_3$ =$-SO_2$-Phenyl) unter Erhalt einer Verbindung der allgemeinen Formel 3

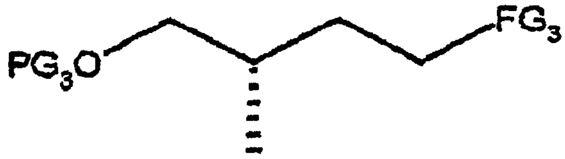

(3)

addiert wird.

**Claims**

**1.** Process for preparing epothilone B and derivatives of the general formula

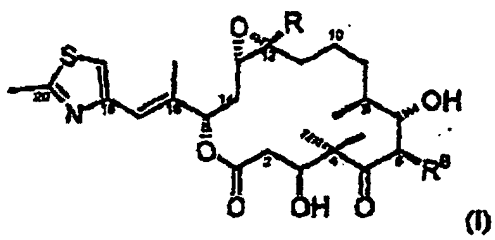

(I)

in which
R is a methyl group and
$R^6$ is a straight-chain or branched-chain alkyl group having up to 6 carbon atoms, a cycloalkylalkyl group having up to 10 carbon atoms, a phenyl, 1- or 2-naphthyl, heteroaryl, benzyl or methylheteroaryl group,
**characterized in that** a compound of the general formula 3

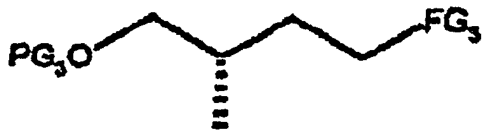

(3)

in which
$PG_3$ is a hydroxyl protective group and
$FG_3$ is a phenylsulphonyl group,
is reacted with a compound of the general formula

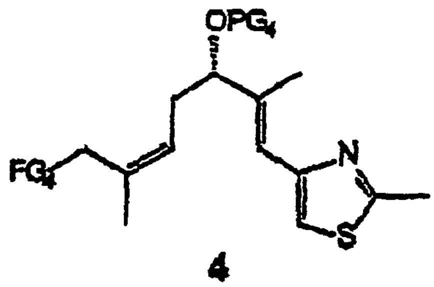

4

in which
$FG_4$ is an iodine atom or another leaving group and
$PG_4$ is a hydroxyl protective group,
to give a compound of the general formula 34-I

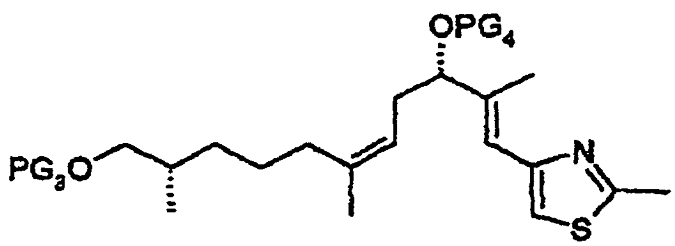

(34-I)

in which
$PG_3$ and $PG_4$ have the abovementioned meanings,
the compound of the general formula 34-I is reacted with a compound of the general formula 2

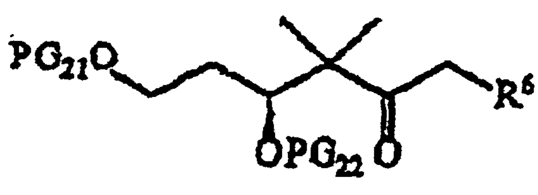

2

in which
$PG_{21}$ and $PG_{22}$ are, independently of one another, each a hydroxyl protective group and

$R^6$ is a straight-chain or branched-chain alkyl group having up to 6 carbon atoms, a cycloalkylalkyl group having up to 10 carbon atoms, a phenyl, 1- or 2-naphthyl, heteroaryl, benzyl or methylheteroaryl group, to give a compound of the general formula 234

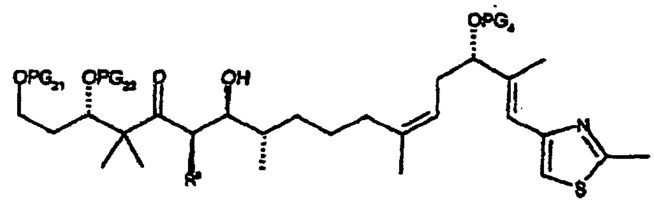

(234)

in which
$PG_{21}$, $PG_{22}$, $PG_4$ and $R^6$ have the meanings already mentioned,
and this compound of the general formula 234 is converted via the stages

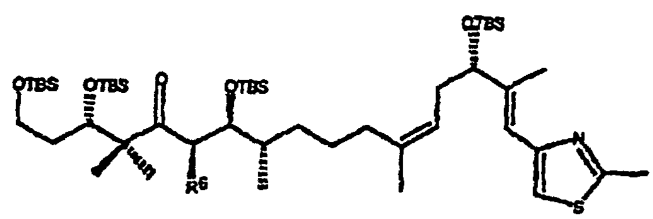

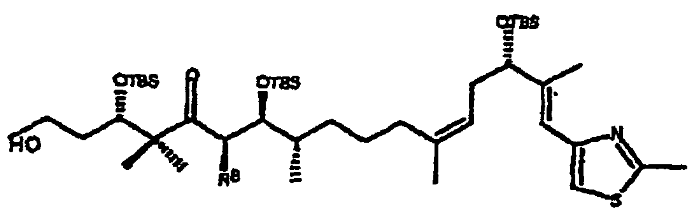

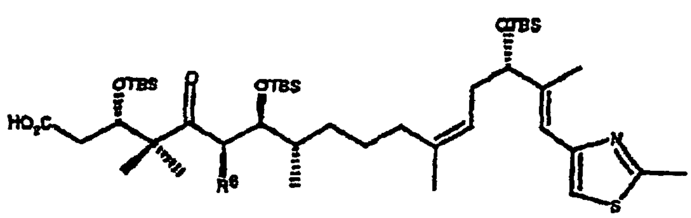

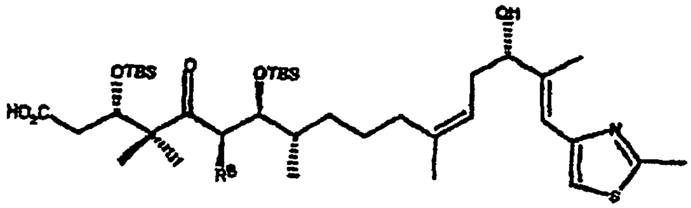

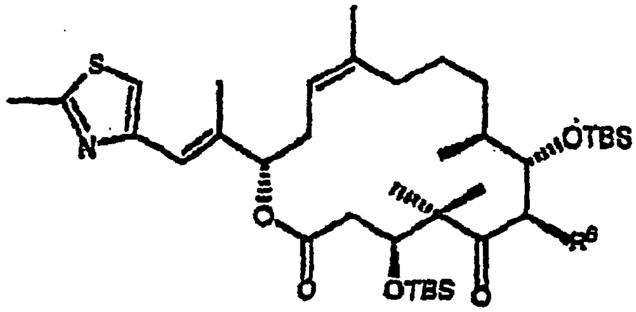

into epothilone D or a derivative of epothilone D

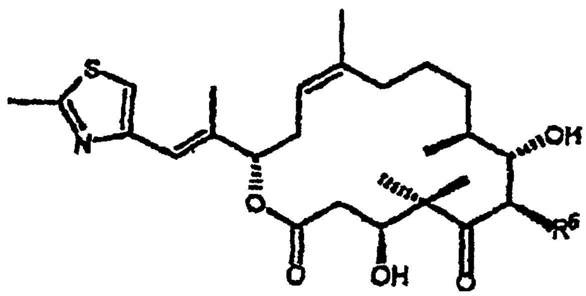

and the latter is then converted where appropriate by epoxidation into epothilone B (R = = $CH_3$) or a derivative of epothilone B.

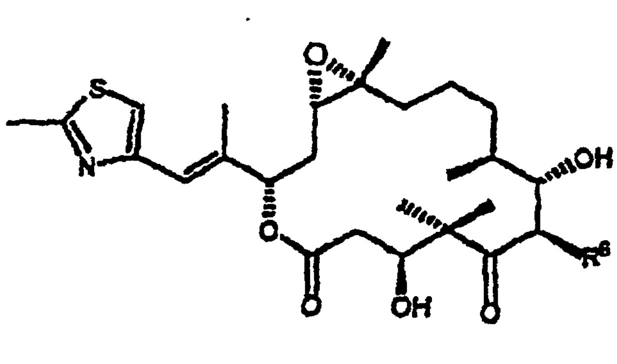

**2.** Process for preparing compounds of the general formula 2 according to Claim 1

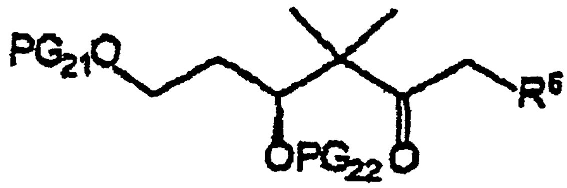

**2**

in which
$PG_{21}$ and $PG_{22}$ are, independently of one another, each a hydroxyl protective group and
$R^6$ is a straight-chain or branched-chain alkyl group having up to 6 carbon atoms, a cycloalkylalkyl group having up to 10 carbon atoms, a phenyl, 1- or 2-naphthyl, heteroaryl, benzyl or methylheteroaryl group,
**characterized in that** a compound of the general formula 2-I

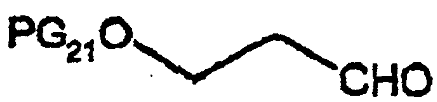

(2-I)

in which
$PG_{21}$ is a hydroxyl protective group,
is reacted with chiral catalysis using a silyl ketene acetal of the general formula

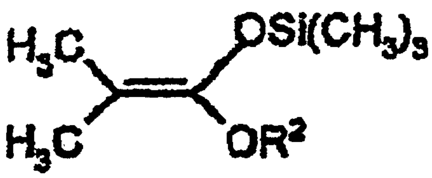

($R^2$ = methyl, ethyl)

with mediation by N-tosylvaline/diborane to give a compound of the general formula 2-II

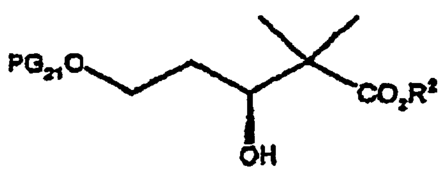

(2-II)

in which
$PG_{21}$ and $R_2$ have the abovementioned meanings,
subsequently the 3-hydroxyl group is protected to result in a compound of the general formula 2-III

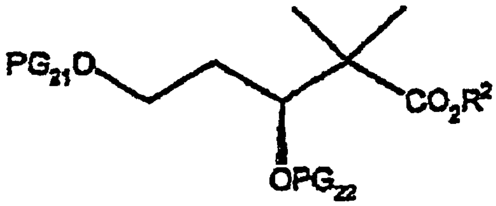

(2-III)

in which

$PG_{21}$ and $PG_{22}$ are, independently of one another, each a hydroxyl protective group, and $R^2$ has the abovementioned meaning,

then a methyl group is added to the carbonyl group of the compound 2-III to result in a compound of the general formula 2-IV

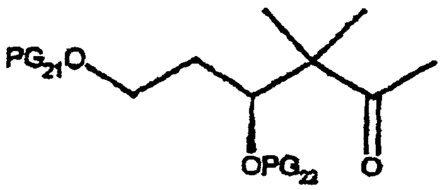

(2-IV)

in which

$PG_{21}$ and $PG_{22}$ are, independently of one another, each a hydroxyl protective group,

and subsequently converted with an alkyl, cycloalkyl, aryl, heteroaryl, methylaryl or methylheteroaryl halide of the general formula 2-X

$$R^6Hal \qquad (2\text{-}X)$$

in which

$R^6$ has the meaning indicated above, and

Hal is a halogen atom fluorine, chlorine or bromine,

into a compound of the general formula 2

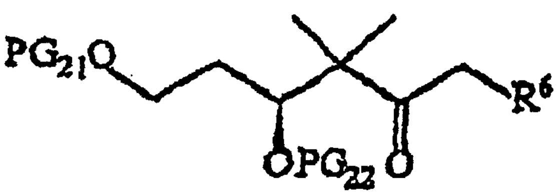

(2)

in which $PG_{21}$, $PG_{22}$ and $R^6$ have the abovementioned meanings,

or the compound 2-III is reduced to the alcohol, the latter is oxidized selectively to the aldehyde, a radical of the formula $-CH_2R^6$ is added onto the latter using an organometallic compound, and the resulting alcohol is oxidized to the ketone of the general formula 2.

**3.** Process for preparing compounds of the general formula 3 according to Claim 1

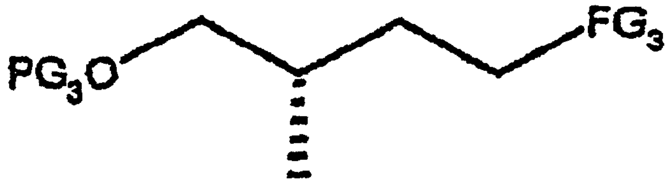

**(3)**

in which
$PG_3$ is a hydroxyl protective group and
$FG_3$ is a phenylsulphonyl group,
**characterized in that** the free hydroxyl group of the compound of the general formula 3-I

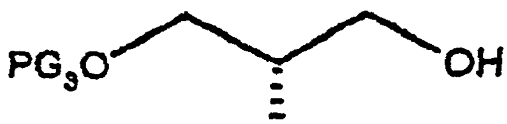

(3-I)

in which
$PG_3$ is a hydroxyl protective group,
is converted into a better leaving group $FG_{31}$ (compound 3-II)

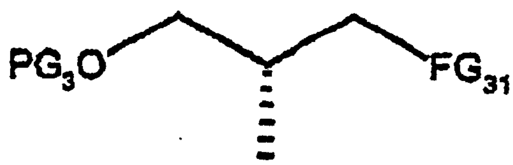

**(3-II)**

and that the group $-CH_2-SO_2$-phenyl ($FG_3 = -SO_2$-phenyl) is added, with displacement of $FG_{31}$, to result in a compound of the general formula 3

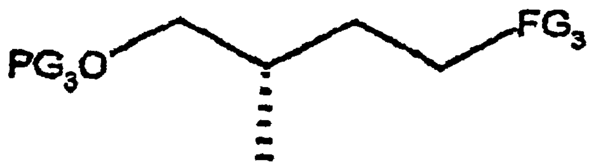

**(3)**.

**Revendications**

**1.** Procédé de préparation d'épothilone B et de dérivés de formule générale

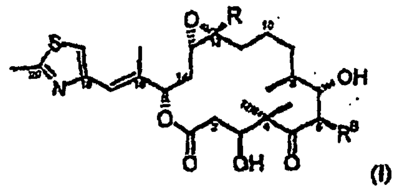

(I)

dans laquelle
R représente un groupe méthyle et
$R^6$ représente un groupe alkyle linéaire ou ramifié ayant jusqu'à 6 atomes de carbone, un groupe cycloalkylalkyle ayant jusqu'à 10 atomes de carbone, un groupe phényle, 1- ou 2-naphtyle, hétéroaryle, benzyle ou méthylhétéroaryle,
**caractérisé en ce qu'**un composé de formule générale 3

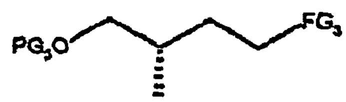

(3)

dans laquelle
$PG_3$ représente un groupe protecteur d'hydroxy et
$FG_3$ représente un groupe phénylsulfonyle,
est mis à réagir avec un composé de formule générale 4

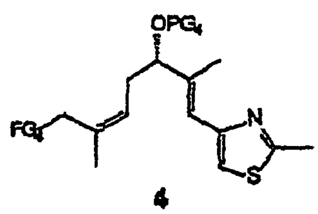

4

dans laquelle
$FG_4$ représente un atome d'iode ou un autre groupe partant et
$PG_4$ représente un groupe protecteur d'hydroxy,
pour donner lieu à un composé de formule générale 34-I

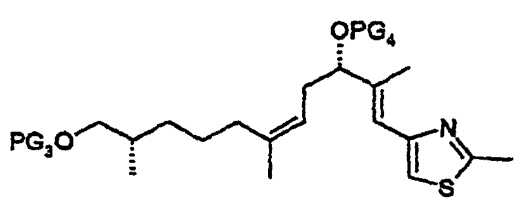

(34-I)

dans laquelle
$PG_3$ et $PG_4$ présentent les significations indiquées ci-dessus,
le composé de formule générale 34-I est mis à réagir avec un composé de formule générale 2

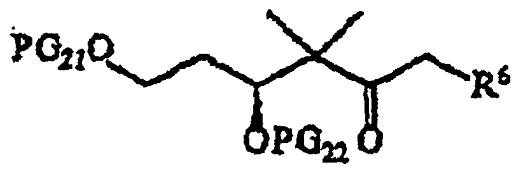

2

dans laquelle
$PG_{21}$ et $PG_{22}$ représentent chacun, indépendamment l'un de l'autre, un groupe protecteur d'hydroxy et
$R^6$ représente un groupe alkyle linéaire ou ramifié ayant jusqu'à 6 atomes de carbone, un groupe cycloalkylalkyle ayant jusqu'à 10 atomes de carbone, un groupe phényle, 1- ou 2-naphtyle, hétéroaryle, benzyle ou méthylhétéroaryle,
pour donner lieu à un composé de formule générale 234

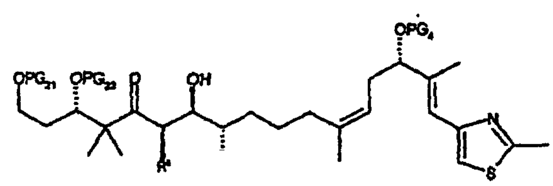

(234)

dans laquelle
$PG_{21}$, $PG_{22}$, $PG_4$ et $R^6$ présentent les significations déjà indiquées,
et ce composé de formule générale 234 est mis à réagir par l'intermédiaire des stades

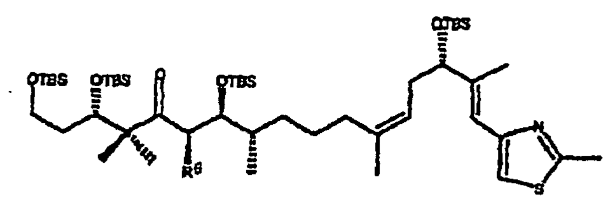

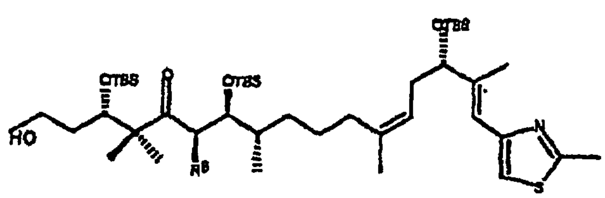

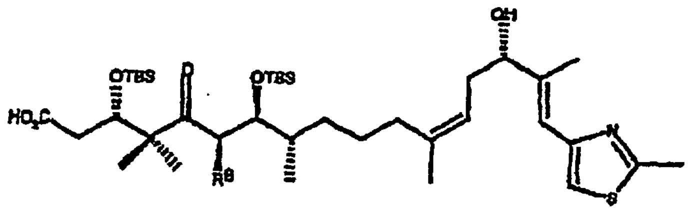

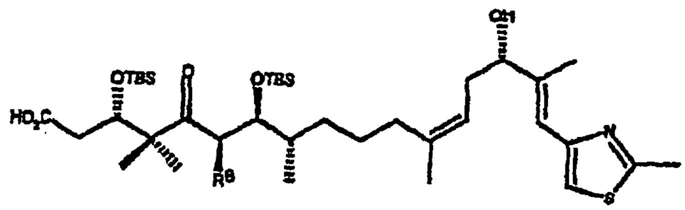

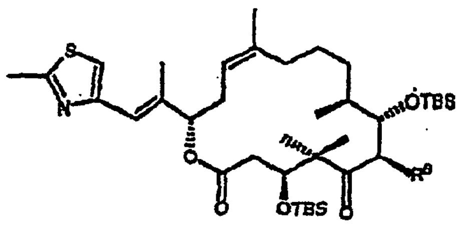

pour donner lieu à l'épothilone D ou un dérivé de l'épothilone D

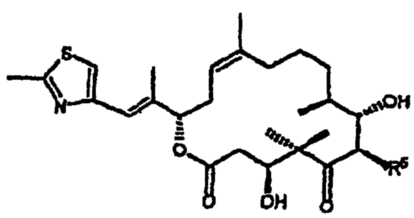

et celui-ci est ensuite éventuellement transformé par époxydation en épothilone B (R = $CH_3$) ou un dérivé de l'épothilone B.

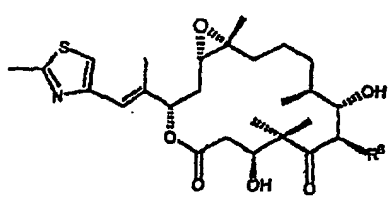

**2.** Procédé de préparation des composés de formule générale 2 selon la revendication 1

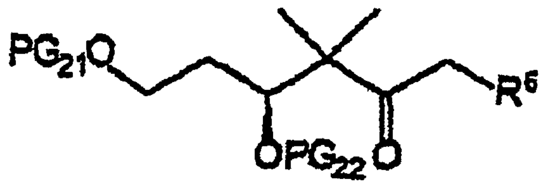

2

dans laquelle
$PG_{21}$ et $PG_{22}$ représentent chacun, indépendamment l'un de l'autre, un groupe protecteur d'hydroxy et
$R^6$ représente un groupe alkyle linéaire ou ramifié ayant jusqu'à 6 atomes de carbone, un groupe cycloalkylalkyle ayant jusqu'à 10 atomes de carbone, un groupe phényle, 1- ou 2-naphtyle, hétéroaryle, benzyle ou méthylhétéroaryle,
**caractérisé en ce qu'**un composé de formule générale 2-I

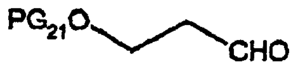

(2-I)

dans laquelle
$PG_{21}$ représente un groupe protecteur d'hydroxy,
est mis à réagir, sous catalyse chirale, avec un silylcétène-acétal de formule générale

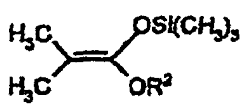

($R^2$ = méthyle, éthyle)

en faisant intervenir de la N-tosylvaline/diborane pour donner lieu à un composé de formule générale 2-II

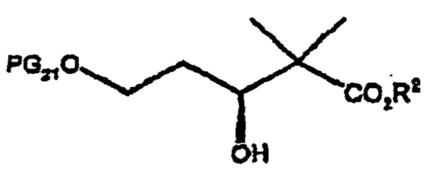

(2-II)

dans laquelle
$PG_{21}$ et $R_2$ présentent les significations indiquées ci-dessus,
le groupe 3-hydroxy est ensuite protégé en produisant un composé de formule générale 2-III

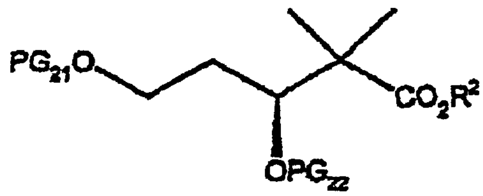

(2-III)

dans laquelle
$PG_{21}$ et $PG_{22}$ représentent chacun, indépendamment l'un de l'autre, un groupe protecteur d'hydroxy et $R^2$ présente la signification indiquée ci-dessus,
puis un groupe méthyle est additionné sur le groupe carbonyle du composé 2-III en produisant un composé de formule générale 2-IV

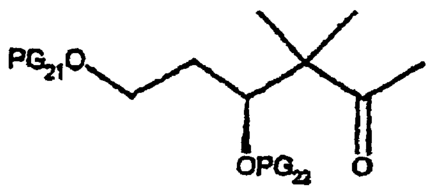

(2-IV)

dans laquelle
$PG_{21}$ et $PG_{22}$ représentent chacun, indépendamment l'un de l'autre, un groupe protecteur d'hydroxy,
et on procède ensuite, à l'aide d'un halogénure d'alkyle, de cycloalkylalkyle, d'aryle, d'hétéroaryle, de méthylaryle ou de méthylhétéroaryle de formule générale 2-X

$$R^6Hal \qquad (2\text{-}X)$$

dans laquelle
$R^6$ présente la signification déjà indiquée et
Hal représente un atome d'halogène fluor, chlore ou brome,
à la transformation en un composé de formule générale 2

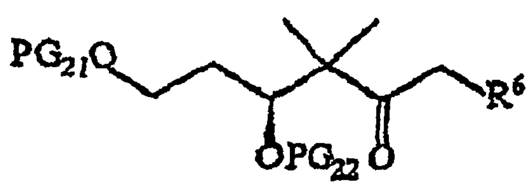

(2)

dans laquelle
$PG_{21}$, $PG_{22}$ et $R^6$ présentent les significations indiquées ci-dessus,
ou le composé 2-III est réduit en alcool, celui-ci est oxydé sélectivement en aldéhyde, un radical de formule $-CH_2-R^6$ est additionné sur celui-ci à l'aide d'un composé organométallique, et l'alcool formé est oxydé en cétone de

formule générale 2.

**3.** Procédé de préparation des composés de formule générale 3 selon la revendication 1

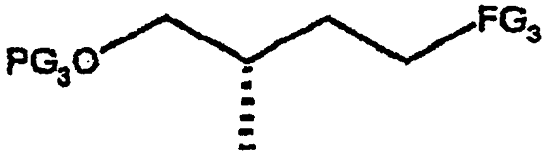

(3)

dans laquelle
$PG_3$ représente un groupe protecteur d'hydroxy et
$FG_3$ représente un groupe phénylsulfonyle,
**caractérisé en ce que** le groupe hydroxy libre du composé de formule générale 3-I

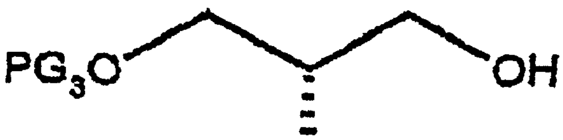

(3-I)

dans laquelle
$PG_3$ représente un groupe protecteur d'hydroxy,
est transformé en un meilleur groupe partant $FG_{31}$ (composé 3-II)

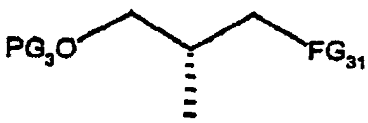

(3-II)

et ensuite, le groupe $-CH_2-SO_2$-phényle ($FG_3 = -SO_2$-phényle) est additionné en déplaçant $FG_{31}$, en produisant un composé de formule générale 3,

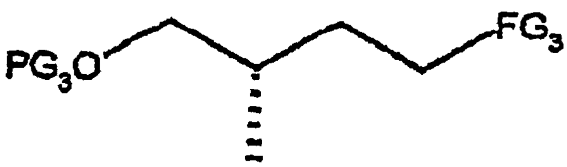

(3)